# EUROPEAN PATENT SPECIFICATION

(11) **EP 1 898 917 B1**
(45) Date of publication and mention of the grant of the patent: **14.01.2009**
(21) Application number: 06772650.5
(22) Date of filing: 07.06.2006
(51) Int. Cl.: A61K 31/506, C07D 239/48, C07D 401/12, C07D 403/04, A61P 35/00

(54) **AMINOPYRIMIDINES AS KINASE MODULATORS**
AMINOPYRIMIDINE ALS KINASE-MODULATOREN
AMINOPYRIMIDINES COMME MODULATEURS DE KINASE

(30) Priority: 10.06.2005 US 689715 P; 16.12.2005 US 751083 P
(43) Date of publication of application: 19.03.2008
(73) Proprietor: Janssen Pharmaceutica, N.V., 2340 Beerse (BE)
(72) Inventor: GAUL, Michael David, Yardley, Pennsylvania 19067 (US); XU, Guozhang, Bensalem, PA 19020 (US); BAUMANN, Christian, Andrew, Exton, PA 19341 (US)
(74) Representative: Warner, James Alexander
(86) International application number: PCT/US2006/022411
(87) International publication number: WO 2006/135719

(56) References cited:
- WO-A-01/00213
- WO-A-03/026664

## Description

### CROSS REFERENCE TO RELATED APPLICATIONS

This application claims priority to U.S. Provisional Application for Patent No. 60/689,715, filed June 10, 2005, and U.S. Provisional Application for Patent No. 60/751,083, filed December 16, 2005.

### FIELD OF THE INVENTION

The invention relates to novel compounds that function as protein tyrosine kinase modulators. More particularly, the invention relates to novel compounds that function as inhibitors of FLT3 and/or c-kit and/or TrkB.

### BACKGROUND OF THE INVENTION

The present invention relates to aminopyrimidines as inhibitors of tyrosine kinases, including FLT3, c-kit and/or TrkB. Pyrimidines have been reported with useful therapeutic properties: US 5104877 and WO 9214468 (preparation of [(tetrazolylbiphenyl)methylamino]pyrimidinecarboxylates and related compounds for treatment of psoriasis ); DE 10108480 and WO 2002068413 (preparation of pyrazolylpyrimidines as insecticides); WO 2002050066, WO 2002066461, WO 2002068415, US 6653300, US 2003036543, US 6664247, US 2003055068, US 2003078275, US 6653301, US 2003105090, US 2003004164, US 6656939, US 2003022885, US 6727251, US 2004116454, US 2004157893, US 2004132781 and US 2004167141; (pyrazole compounds useful as protein kinase inhibitors, and therapeutic use thereof) US 6107301 and US 6342503 (preparation of 1-N-alkyl-N-arylpyrimidinamines as CRF inhibitors ); WO 2001085700, WO 2001085700 and US 2003171374 (preparation of substituted amino pyrimidines and triazines as HIV replication inhibitors ); WO 2001085699, WO 2001085699 and US 2003186990 (preparation of prodrugs of HIV replication inhibiting pyrimidines); WO 2001022938 (preparation of azinylaminobenzonitriles and related compounds as virucides); WO 2000027825, US 2003114472 and US 2004039005 (preparation of arylaminopyrimidines as inhibitors of HIV replication); WO 2004058762, WO 2004058762 and US 2004152739 (preparation of pyrrolopyridinones as mitogen activated protein kinase-activated protein kinase-2 inhibiting compounds); WO 2003094920 (microbicidal pyrimidine or triazine compounds for preventing sexual HIV transmission); WO 2004005283 and US 2004097531 (preparation of imidazolpyrimidines and related compounds as JNK protein kinase inhibitors); see also: Wardakhan, Wagnat W.; Fleita, Daisy H.; Mohareb, Rafat M. Reaction of 4-aryl-3-thiosemicarbazides with phenyl isothiocyanate: a facile synthesis of thiazole, pyrazole and pyrimidine derivatives. Journal of the Chinese Chemical Society (Taipei) (1999), 46(1), 97-104; and Taylor, Edward C.; Ehrhart, Wendell A.; Tomlin, Clive O. S.; Rampal, Jang B. A novel ring-switching amination: conversion of 4-amino-5-cyanopyrimidine to 4,6-diamino-5-cyanopyrimidine. Heterocycles (1987), 25(1), 343-5. Of note also: JP 9274290 (developer and method for processing of silver halide photographic material); DE 10060412, WO 2002046151, and US 2004082586 (3,4-dihydro-2H-pyrroles as pesticides); WO 2004039785 and US 2004152896 (Process for the preparation of pyrrolidinyl ethylamine compounds via a copper-mediated aryl amination).

Protein kinases are enzymatic components of the signal transduction pathways which catalyze the transfer of the terminal phosphate from ATP to the hydroxy group of tyrosine, serine and/or threonine residues of proteins. Thus, compounds which inhibit protein kinase functions are valuable tools for assessing the physiological consequences of protein kinase activation. The overexpression or inappropriate expression of normal or mutant protein kinases in mammals has been a topic of extensive study and has been demonstrated to play a significant role in the development of many diseases, including diabetes, angiogenesis, psoriasis, restenosis, ocular diseases, schizophrenia, rheumatoid arthritis, atherosclerosis, cardiovascular disease and cancer. The cardiotonic benefits of kinase inhibition has also been studied. In sum, inhibitors of protein kinases have particular utility in the treatment of human and animal disease.

The Trk family receptor tyrosine kinases, TrkA, TrkB, and TrkC, are the signaling receptors that mediate the biological actions of the peptide hormones of the neurotrophin family. This family of growth factors includes nerve growth factor (NGF), brain-derived neurotrophic factor (BDNF), and two neurotrophins (NT), NT-3, and NT-4. TrkB serves as a receptor for both BDNF and NT-4. BDNF promotes the proliferation, differentiation and survival of normal neural components such as retinal cells and glial cells.

It has recently been reported (see, Nature 2004 Aug 26; 430(7003):973-4; 1034-40) that TrkB activation is a potent and specific suppressor of anchorage independent cell death (anoikis). Anchorage independent cell survival allows tumor cells to migrate through the systemic circulation and grow at distant organs. This metastatic process is often responsible for the failure of cancer treatment and the cause of mortality in cancer. Other studies (see, Cancer Lett. 2003 Apr 10;193(1):109-14) have also suggested that BDNF agonism of TrkB is capable of blocking cisplatin induced cell death. Taken together, these results suggest that TrkB modulation is an attractive target for treatment of benign and malignant proliferative diseases, especially tumor diseases.

The receptor tyrosine kinase c-kit and its ligand Stem Cell Factor (SCF) are essential for hemoatpoiesis, melanogenesis and fertility. SCF acts at multiple levels of the hemoatpoietic hierarchy to promote cell survival, proliferation, differentiation, adhesion and functional activation. It is of particular importance in the mast cell and erythroid lineages, but also acts on multipotential stem and progenitor cells, megakaryocytes, and a subset of lymphoid progenitors (see, Int J Biochem Cell Biol. 1999 Oct;31(10):1037-51). Sporadic mutations of c-kit as well as autocrine/paracrine activation mechanisms of the SCF/c-kit pathway have been implicated in a variety of malignancies. Activation of c-kit contributes to metastases by enhancing tumor growth and reducing apoptosis. Additionally, c-kit is frequently mutated and activated in gastrointestinal stromal tumors (GISTs), and ligand-mediated activation of c-kit is present in some lung cancers (see, Leuk Res. 2004 May;28 Suppl 1:S11-20). The c-kit receptor also is expressed on more than 10% of blasts in 64% of de novo acute myelogenous leukemias (AMLs) and 95% of relapsed AMLs. C-kit mediates proliferation and anti-apoptotic effects in AML (see, Curr Hematol Rep. 2005 Jan;4(1):51-8).

C-Kit expression has been documented in a wide variety of human malignancies, including mastocytosis, mast cell leukemia, gastrointestinal stromal tumour, sinonasal natural killer/T-cell lymphoma, seminoma, dysgerminoma, thyroid carcinoma; small-cell lung carcinoma, malignant melanoma, adenoid cystic carcinoma, ovarian carcinoma, acute myelogenous leukemia, anaplastic large cell lymphoma, angiosarcoma, endometrial carcinoma, pediatric T-cell ALL, lymphoma, breast carcinoma and prostate carcinoma. See, Heinrich, Michael C. et al. Review Article: Inhibition of KIT Tyrosine Kinase Activity: A Novel Molecular Approach to the Treatment of KIT-Positive Malignancies.

The fms-like tyrosine kinase 3 (FLT3) ligand (FLT3L) is one of the cytokines that affects the development of multiple hematopoietic lineages. These effects occur through the binding of FLT3L to the FLT3 receptor, also referred to as fetal liver kinase-2 (flk-2) and STK-1, a receptor tyrosine kinase (RTK) expressed on hematopoietic stem and progenitor cells. The FLT3 gene encodes a membrane-bound RTK that plays an important role in proliferation, differentiation and apoptosis of cells during normal hematopoiesis. The FLT3 gene is mainly expressed by early meyloid and lymphoid progenitor cells. See McKenna, Hilary J. et al. Mice lacking flt3 ligand have deficient hematopoiesis affecting hematopoietic progenitor cells, dendritic cells, and natural killer cells. Blood. Jun 2000; 95: 3489-3497; Drexler, H. G. and H. Quentmeier (2004). "FLT3: receptor and ligand." Growth Factors 22(2): 71-3.

The ligand for FLT3 is expressed by the marrow stromal cells and other cells and synergizes with other growth factors to stimulate proliferation of stem cells, progenitor cells, dendritic cells, and natural killer cells.

Hematopoietic disorders are pre-malignant disorders of these systems and include, for instance, the myeloproliferative disorders, such as thrombocythemia, essential thrombocytosis (ET), angiogenic myeloid metaplasia, myelofibrosis (MF), myelofibrosis with myeloid metaplasia (MMM), chronic idiopathic myelofibrosis (IMF), and polycythemia vera (PV), the cytopenias, and pre-malignant myelodysplastic syndromes. See Stirewalt, D. L. and J. P. Radich (2003). "The role of FLT3 in haematopoietic malignancies." Nat Rev Cancer 3(9): 650-65; Scheijen, B. and J. D. Griffin (2002). "Tyrosine kinase oncogenes in normal hematopoiesis and hematological disease." Oncogene 21(21): 3314-33.

Hematological malignancies are cancers of the body's blood forming and immune systems, the bone marrow and lymphatic tissues. Whereas in normal bone marrow, FLT3 expression is restricted to early progenitor cells, in hematological malignancies, FLT3 is expressed at high levels or FLT3 mutations cause an uncontrolled induction of the FLT3 receptor and downstream molecular pathway, possibly Ras activation. Hematological malignancies include leukemias, lymphomas (non-Hodgkin's lymphoma), Hodgkin's disease (also called Hodgkin's lymphoma), and myeloma-- for instance, acute lymphocytic leukemia (ALL), acute myeloid leukemia (AML), acute promyelocytic leukemia (APL), chronic lymphocytic leukemia (CLL), chronic myeloid leukemia (CML), chronic neutrophilic leukemia (CNL), acute undifferentiated leukemia (AUL), anaplastic large-cell lymphoma (ALCL), prolymphocytic leukemia (PML), juvenile myelomonocyctic leukemia (JMML), adult T-cell ALL, AML with trilineage myelodysplasia (AML/TMDS), mixed lineage leukemia (MLL), myelodysplastic syndromes (MDSs), myeloproliferative disorders (MPD), multiple myeloma, (MM) and myeloid sarcoma. See Kottaridis, P. D., R. E. Gale, et al. (2003). "Flt3 mutations and leukaemia." Br J Haematol 122(4): 523-38. Myeloid sarcoma is also associated with FLT3 mutations. See Ansari-Lari, Ali et al. FLT3 mutations in myeloid sarcoma. British Journal of Haematology. 2004 Sep. 126(6):785-91.

Mutations of FLT3 have been detected in about 30% of patients with acute myelogenous leukemia and a small number of patients with acute lymphomatic leukemia or myelodysplastic syndrome. Patients with FLT3 mutations tend to have a poor prognosis, with decreased remission times and disease free survival. There are two known types of activating mutations of FLT3. One is a duplication of 4-40 amino acids in the juxtamembrane region (ITD mutation) of the receptor (25-30% of patients) and the other is a point mutation in the kinase domain (5-7% of patients). The mutations most often involve small tandem duplications of amino acids within the juxtamembrane domain of the receptor and result in tyrosine kinase activity. Expression of a mutant FLT3 receptor in murine marrow cells results in a lethal myeloproliferative syndrome, and preliminary studies (Blood. 2002; 100: 1532-42) suggest that mutant FLT3 cooperates with other leukemia oncogenes to confer a more aggressive phenotype.

Taken together, these results suggest that specific inhibitors of the individual kinases FLT3 and c-kit, and especially of the group of kinases comprising FLT3 and c-kit, present an attractive target for the treatment of hematopoietic disorders and hematological malignancies.

FLT3 kinase inhibitors known in the art include AG1295 and AG1296; Lestaurtinib (also known as CEP 701, formerly KT-5555, Kyowa Hakko, licensed to Cephalon); CEP-5214 and CEP-7055 (Cephalon); CHIR-258 (Chiron Corp.); EB-10 and IMC-EB 10 (ImClone Systems Inc.); GTP 14564 (Merk Biosciences UK). Midostaurin (also known as PKC 412 Novartis AG); MLN 608 (Millennium USA); MLN-518 (formerly CT53518, COR Therapeutics Inc., licensed to Millennium Pharmaceuticals Inc.); MLN-608 (Millennium Pharmaceuticals Inc.); SU-11248 (Pfizer USA); SU-11657 (Pfizer USA); SU-5416 and SU 5614; THRX-165724 (Theravance Inc.); AMI-10706 (Theravance Inc.); VX-528 and VX-680 (Vertex Pharmaceuticals USA, licensed to Novartis (Switzerland), Merck & Co USA); and XL 999 (Exelixis USA). The following PCT International Applications and US Patent Applications disclose additional kinase modulators, including modulators of FLT3: WO 2002032861, WO 2002092599, WO 2003035009, WO 2003024931, WO 2003037347, WO 2003057690, WO 2003099771, WO 2004005281, WO 2004016597, WO 2004018419, WO 2004039782, WO 2004043389, WO 2004046120, WO 2004058749, WO 2004058749, WO 2003024969 and US Patent Application No. 20040049032.

See also Levis, M., K. P. Tse, et al. 2001 "A FLT3 tyrosine kinase inhibitor is selectively cytotoxic to acute myeloid leukemin blasts harboring PLT3 internal tandem duplication mutations." Blood 98(3): 885-7; Tse KF, et al. Inhibition of FLT3-mediated transformation by use of a tyrosine kinase inhibitor. Leukemia. 2001 Jul; 15(7):1001-10; Smith, B. Douglas et al. Single-agent CEP-701, a novel FLT3 inhibitor, shows biologic and clinical activity in patients with relapsed or refractory acute myeloid leukemia Blood, May 2004; 103: 3669 - 3676; Griswold, Ian J. et al. Effects of MLN518, A Dual FLT3 and KIT Inhibitor, on Normal and Malignant Hematopoiesis. Blood, Jul 2004; [Epub ahead of print]; Yee, Kevin W. H, et al. SU5416 and SU5614 inhibit kinase activity of wild-type and mutant FLT3 receptor tyrosine kinase. Blood, Sep 2002; 100: 2941 - 294; O'Farrell, Ann-Marie et al. SU11248 is a novel FLT3 tyrosine kinase inhibitor with potent activity in vitro and in vivo. Blood, May 2003; 101: 3597 - 3605; Stone, R.M. ct al. PKC 412 FLT3 inhibitor therapy in AML: results of a phase II trial. Ann Hematol. 2004; 83 Suppl US89-90; and Murata, K. et al. Selective cytotoxic mechanism of GTP-14564 a novel tyrosine kinase inhibitor in leukemia cells expressing a constitutively active Fms-like tyrosine kinase 3 (FLT3). J Biol Chem. 2003 Aug 29; 278(35):32892-8; Levis, Mark et al. Novel FLT3 tyrosine kinase inhibitors. Expert Opin. Investing. Drugs (2003) 12(12) 1951-1962; Levis, Mark et al. Small Molecule FLT3 Tyrosine Kinase Inhibitors. Current Pharmaceutical Design, 2004, 10, 1183-1193.

### SUMMARY OF THE INVENTION

The present invention provides novel aminopyrimidines (the compounds of Formula I) as protein tyrosine kinase modulators, particularly inhibitors of FLT3 and/or c-kit and/or TrkB, and the use of such compounds for reducing or inhibiting kinase activity of FLT3 and/or c-kit and/or TrkB in a cell or a subject, and the use of such compounds for preventing or treating in a subject disorders related to FLT3 and/or c-kit and/or TrkB, or treating a cell proliferative disorder.

Illustrative of the invention is a pharmaceutical composition comprising a compound of Formula I and a pharmaceutically acceptable carrier. Another illustration of the present invention is a pharmaceutical composition prepared by mixing any of the compounds of Formula I and a pharmaceutically acceptable carrier.

Other features and advantages of the invention will be apparent from the following detailed description of the invention and from the claims.

### DETAILED DESCRIPTION OF THE INVENTION

### DEFINITIONS

As used herein, the following terms are intended to have the following meanings (additional definitions are provided where needed throughout the Specification):

The term **"alkenyl,"** whether used alone or as part of a substituent group, for example, "C₁₋₄alkenyl(aryl)," refers to a partially unsaturated branched or straight chain monovalent hydrocarbon radical having at least one carbon-carbon double bond, whereby the double bond is derived by the removal of one hydrogen atom from each of two adjacent carbon atoms of a parent alkyl molecule and the radical is derived by the removal of one hydrogen atom from a single carbon atom. Atoms may be oriented about the double bond in either the *cis* (Z) or *trans* (E) conformation. Typical alkenyl radicals include, but are not limited to, ethenyl, propenyl, allyl (2-propenyl), butenyl and the like. Examples include C₂₋₈alkenyl or C₂₋₄alkenyl groups.

The term "C*_{a-b}*" (where *a* and *b* are integers referring to a designated number of carbon atoms) refers to an alkyl, alkenyl, alkynyl, alkoxy or cycloalkyl radical or to the alkyl portion of a radical in which alkyl appears as the prefix root containing from *a* to *b* carbon atoms inclusive. For example, C₁₋₄ denotes a radical containing 1, 2, 3 or 4 carbon atoms.

The term **"alkyl,"** whether used alone or as part of a substituent group, refers to a saturated branched or straight chain moriovalent hydrocarbon radical, wherein the radical is derived by the removal of one hydrogen atom from a single carbon atom. Unless specifically indicated (e.g. by the use of a limiting term such as "terminal carbon atom"), substituent variables may be placed on any carbon chain atom. Typical alkyl radicals include, but are not limited to, methyl, ethyl, propyl, isopropyl and the like. Examples include C₁₋₈alkyl, C₁₋₆alkyl and C₁₋₄alkyl groups.

The term **"alkylamino"** refers to a radical formed by the removal of one hydrogen atom from the nitrogen of an alkylamine, such as butylamine, and the term "dialkylamino" refers to a radical formed by the removal of one hydrogen atom from the nitrogen of a secondary amine, such as dibutylamine. In both cases it is expected that the point of attachment to the rest of the molecule is the nitrogen atom.

The term **"alkynyl,"** whether used alone or as part of a substituent group, refers to a partially unsaturated branched or straight chain monovalent hydrocarbon radical having at least one carbon-carbon triple bond, whereby the triple bond is derived by the removal of two hydrogen atoms from each of two adjacent carbon atoms of a parent alkyl molecule and the radical is derived by the removal of one hydrogen atom from a single carbon atom. Typical alkynyl radicals include ethynyl, propynyl, butynyl and the like. Examples include C₂₋₈alkynyl or C₂₋₄alkynyl groups.

The term **"alkoxy"** refers to a saturated or partially unsaturated branched or straight chain monovalent hydrocarbon alcohol radical derived by the removal of the hydrogen atom from the hydroxide oxygen substituent on a parent alkane, alkene or alkyne. Where specific levels of saturation are intended, the nomenclature "alkoxy", "alkenyloxy" and "alkynyloxy" are used consistent with the definitions of alkyl, alkenyl and alkynyl. Examples include C₁₋₈alkoxy or C₁₋₄alkoxy groups.

The term **"alkoxyether"** refers to a saturated branched or straight chain monovalent hydrocarbon alcohol radical derived by the removal of the hydrogen atom from the hydroxide oxygen substituent on a hydroxyether. Examples include 1-hydroxyl-2-methoxy-ethane or 1-(2-hydroxyl-ethoxy)-2-methoxy-ethane groups.

The term **"aralkyl"** refers to a C₁₋₆ alkyl group containing an aryl substituent. Examples include benzyl, phenylethyl or 2-naphthylmethyl. It is intended that the point of attachment to the rest of the molecule be the alkyl group.

The term **"aromatic"** refers to a cyclic hydrocarbon ring system having an unsaturated, conjugated π electron system.

The term **"aryl"** refers to an aromatic cyclic hydrocarbon ring radical derived by the removal of one hydrogen atom from a single carbon atom of the ring system. Typical aryl radicals include phenyl, naphthalenyl, fluorenyl, indenyl, azulenyl, anthracenyl and the like.

The term **"arylamino"** refers to an amino group, such as ammonia, substituted with an aryl group, such as phenyl. It is expected that the point of attachment to the rest of the molecule is through the nitrogen atom.

The term "**aryloxy"** refers to an oxygen atom radical substituted with an aryl group, such as phenyl. It is expected that the point of attachment to the rest of the molecule is through the oxygen atom.

The term **"benzo-fused cycloalkyl"** refers to a bicyclic fused ring system radical wherein one of the rings is phenyl and the other is a cycloalkyl or cycloalkenyl ring. Typical benzo-fused cycloalkyl radicals include indanyl, 1,2,3,4-tetrahydronaphthalenyl, 6,7,8,9-tetrahydro-5*H*-benzocycloheptenyl, 5,6,7,8,9,10-hexahydrobenzocyclooctenyl and the like. A benzo-fused cycloalkyl ring system is a subset of the aryl group.

The term **"benzo-fused heteroaryl"** refers to a bicyclic fused ring system radical wherein one of the rings is phenyl and the other is a heteroaryl ring. Typical benzo-fused heteroaryl radicals include indolyl, indolinyl, isoindolyl, benzo[*b*]furyl, benzo[*b*]thienyl, indazolyl, benzthiazolyl, quinolinyl, isoquinolinyl, cinnolinyl, phthalazinyl, quinazolinyl, and the like. A benzo-fused heteroaryl ring is a subset of the heteroaryl group.

The term **"benzo-fused heterocyclyl"** refers to a bicyclic fused ring system radical wherein one of the rings is phenyl and the other is a heterocyclyl ring. Typical benzo-fused heterocyclyl radicals include 1,3-benzodioxolyl (also known as 1,3-methylenedioxyphenyl), 2,3-dihydro-1,4-benzodioxinyl (also known as 1,4-ethylenedioxyphenyl), benzo-dihydro-furyl, benzo-tetrahydro-pyranyl, benzo-dihydro-thienyl and the like.

The term **"carboxyalkyl"** refers to an alkylated carboxy group such as tert-butoxycarbonyl, in which the point of attachment to the rest of the molecule is the carbonyl group.

The term **"cyclic heterodionyl"** refers to a heterocyclic compound bearing two oxo substituents. Examples include thiazolidinedionyl, oxazolidinedionyl and pyrrolidinedionyl.

The term **"cycloalkenyl"** refers to a partially unsaturated cycloalkyl radical derived by the removal of one hydrogen atom from a hydrocarbon ring system that contains at least one carbon-carbon double bond. Examples include oyclohexenyl, cyclopentenyl and 1,2,5,6-cyclooctadienyl.

The term **"cycloalkyl"** refers to a saturated or partially unsaturated monocyclic or bicyclic hydrocarbon ring radical derived by the removal of one hydrogen atom from a single ring carbon atom. Typical cycloalkyl radicals include cyclopropyl, cyclobutyl, cyclopentyl, cyclopentenyl, cyclohexyl, cyclohexenyl, cycloheptyl and cyclooctyl. Additional examples include C₃₋₈cyloalkyl, C₅₋₈cycloalkyl, C₃₋₁₂cycloalkyl, C₃₋₂₀cycloalkyl, decahydronaphthalenyl, and 2,3,4,5,6,7-hexahydro-1H-indenyl.

The term "fused ring system" refers to a bicyclic molecule in which two adjacent atoms are present in each of the two cyclic moieties. Heteroatoms may optionally be present. Examples include benzothiazole, 1,3-benzodioxole and decahydronaphthalene.

The term **"hetero"** used as a prefix for a ring system refers to the replacement of at least one ring carbon atom with one or more atoms independently selected from N, S, O or P. Examples include rings wherein 1, 2, 3 or 4 ring members are a nitrogen atom; or, 0, 1, 2 or 3 ring members are nitrogen atoms and 1 member is an oxygen or sulfur atom.

The term **"heteroaralkyl"** refers to a C₁₋₆ alkyl group containing a heteroaryl substituent. Examples include furylmethyl and pyridylpropyl. It is intended that the point of attachment to the rest of the molecule be the alkyl group.

The term **"heteroaryl"** refers to a radical derived by the removal of one hydrogen atom from a ring carbon atom of a heteroaromatic ring system. Typical heteroaryl radicals include furyl, thienyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl, pyrazinyl, indolizinyl, indolyl, isoindolyl, benzo[*b*]furyl, benzo[*b*]thienyl, indazolyl, benzimidazolyl, benzthiazolyl, purinyl, 4*H*-quinolizinyl, quinolinyl, isoquinolinyl, cinnolinyl, phthalzinyl, quinazolinyl, quinoxalinyl, 1,8-naphthyridinyl, pteridinyl and the like.

The term **"heteroaryl-fused cycloalkyl"** refers to a bicyclic fused ring system radical wherein one of the rings is cycloalkyl and the other is heteroaryl. Typical heteroaryl-fused cycloalkyl radicals include 5,6,7,8-tetrahydro-4*H*-cyclohepta(*b*)thienyl, 5,6,7-trihydro-4*H*-cyclohexa(*b*)thienyl, 5,6-dihydro-4*H*-cyclopenta(*b*)thienyl and the like.

The term **"heteroaryloxy"** refers to an oxygen atom radical substituted with a heteroaryl group, such as pyridyl. It is expected that the point of attachment to the rest of the molecule is through the oxygen atom.

The term **"heterocyclyl"** refers to a saturated or partially unsaturated monocyclic ring radical derived by the removal of one hydrogen atom from a single carbon or nitrogen ring atom. Typical heterocyclyl radicals include 2*H*-pyrrole, 2-pyrrolinyl, 3-pyrrolinyl, pyrrolidinyl, 1,3-dioxolanyl, 2-imidazolinyl (also referred to as 4,5-dihydro-1*H*-imidazolyl), imidazolidinyl, 2-pyrazolinyl, pyrazolidinyl, tetrazolyl, piperidinyl, 1,4-dioxanyl, morpholinyl, 1,4-dithianyl, thiomorpholinyl, thiomorpholinyl 1,1 dioxide, piperazinyl, azepanyl, hexahydro-1,4-diazepinyl and the like.

The term **"oxo"** refers to an oxygen atom radical; said oxygen atom has two open valencies which are bonded to the same atom, most preferably a carbon atom. The oxo group is an appropriate substituent for an alkyl group. For example, propane with an oxo substituent is either acetone or propionaldehyde. Heterocycles can also be substituted with an oxo group. For example, oxazolidine with an oxo substituent is oxazolidinone.

The term **"substituted,"** refers to a core molecule on which one or more hydrogen atoms have been replaced with one or more functional radical moieties. Substitution is not limited to a core molecule, but may also occur on a substituent radical, whereby the substituent radical becomes a linking group.

The term **"independently selected"** refers to one or more substituents selected from a group of substituents, wherein the substituents may be the same or different.

The substituent nomenclature used in the disclosure of the present invention was derived by first indicating the atom having the point of attachment, followed by the linking group atoms toward the terminal chain atom from left to right, substantially as in:

(C₁₋₆)alkylC(O)NH(C₁₋₆)alkyl(Ph)

or by first indicating the terminal chain atom, followed by the linking group atoms toward the atom having the point of attachment, substantially as in:

Ph(C₁₋₆)alkylamido(C₁₋₆)alkyl

either of which refers to a radical of the formula:

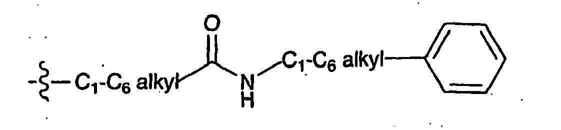

Lines drawn into ring systems from substituents indicate that the bond may be attached to any of the suitable ring atoms.

When any variable (e.g. R₄) occurs more than one time in any embodiment of Formula I, each definition is intended to be independent.

The terms "comprising", "including", and "containing" are used herein in their open, non-limited sense.

### NOMENCLATURE

Except where indicated, compound names were derived using nomenclature rules well known to those skilled in the art, by either standard IUPAC nomenclature references, such as Nomenclature of Organic Chemistry, Sections A, B, C, D, E, F and H, (Pergamon Press, Oxford, 1979, Copyright 1979 IUPAC) and A Guide to IUPAC Nomenclature of Organic Compounds (Recommendations 1993), (Blackwell Scientific Publications, 1993, Copyright 1993 IUPAC); or commercially available software packages such as Autonom (brand of nomenclature software provided in the ChemDraw Ultra^{®} office suite marketed by CambridgeSoft.com); and ACD/Index Name^{™} (brand of commercial nomenclature software marketed by Advanced Chemistry Development, Inc., Toronto, Ontario).

### ABBREVIATIONS

As used herein, the following abbreviations are intended to have the following meanings (additional abbreviations are provided where needed throughout the Specification):
- ATP: adenosine triphosphate
- Boc: *tert*-butoxycarbonyl
- DCM: dichloromethane
- DMF: dimethylformamide
- DMSO: dimethylsulfoxide
- DIEA: diisopropylethylamine
- DTT: dithiothreitol
- EDC: 1-(3-dimethylaminopropyl)-3-ethylcarbodiimide hydrochloride
- EDTA: ethylenediaminetetraaceticacid
- EtOAc: ethyl acetate
- FBS: fetal bovine serum
- FP: fluorescence polarization
- GM-CSF: granulocyte and macrophage colony stimulating factor
- HBTU: *O*-benzotriazol-1-yl-N,N,N',N'-tetramethyluronium Hexafluorophosphate
- HOBT: 1-hydroxybenzotriazole hydrate
- HPβCD: hydroxypropyl β-cyclodextrin
- HRP: horseradish peroxidase
- *i*-PrOH: isopropyl alcohol
- LC/MS (ESI): Liquid chromatography/mass spectrum (electrospray ionization)
- MeOH: Methyl alcohol
- NMM: *N*-methylmorpholine
- NMR: nuclear magnetic resonance
- PS: polystyrene
- PBS: phosphate buffered saline
- RPMI: Rosewell Park Memorial Institute
- RT: room temperature
- RTK: receptor tyrosine kinase
- NaHMDS: sodium hexamethyldisilazane
- SDS-PAGE: sodium dodecyl sulfate polyacrylamide gel electrophoreisis
- TEA: triethylamine
- TFA: trifluoroacetic acid
- THF: tetrahydrofuran
- TLC: thin layer chromatography

### FORMULA I

The present invention comprises compounds of Formula I: 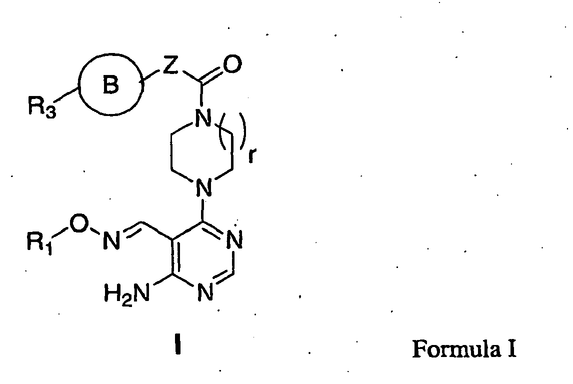 and N-oxides, pharmaceutically acceptable salts, solvates, geometric isomers and stereochemical isomers thereof, wherein:
**r** is 1 or 2;
**Z** is NH, N(alkyl), or CH₂;
**B** is phenyl, heteroaryl (wherein said heteroaryl is preferably pyrrolyl, furanyl, thiophenyl, imidazolyl, thiazolyl, oxazolyl, pyranyl, thiopyranyl, pyridinyl, pyrimidinyl, pyrazinyl, pyridinyl-N-oxide, or pyrrolyl-N-oxide, and most preferably pyrrolyl, furanyl, thiophenyl, imidazolyl, thiazolyl, oxazolyl, pyridinyl, pyrimidinyl, or pyrazinyl), or a nine to ten membered benzo-fused heteroaryl (wherein said nine to ten membered benzo-fused heteroaryl is preferably benzothiazolyl, benzooxazolyl, benzoimidazolyl, benzofuranyl, indolyl, quinolinyl, isoquinolinyl, or benzo[b]thiophenyl);
**R₁** is: 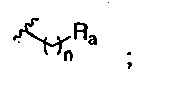
   wherein **n** is 1, 2, 3 or 4;
   **Rₐ** is hydrogen, alkoxy, phenoxy, phenyl, heteroaryl optionally substituted with R₅ (wherein said heteroaryl is preferably pyrrolyl, furanyl, thiophenyl, imidazolyl, thiazolyl, oxazolyl, pyranyl, thiopyranyl, pyridinyl, pyrimidinyl, triazolyl, pyrazinyl, pyridinyl-N-oxide, or pyrrolyl-N-oxide, and most preferably pyrrolyl, furanyl, thiophenyl, imidazolyl, thiazolyl, oxazolyl, pyridinyl, pyrimidinyl, triazolyl, or pyrazinyl), hydroxyl, amino, alkylamino, dialkylamino, oxazolidinonyl optionally substituted with R₅, pyrrolidinonyl optionally substituted with R₅, piperidinonyl optionally substituted with R₅, cyclic heterodionyl optionally substituted with R₅, heterocyclyl optionally substituted with R₅ (wherein said heterocyclyl is preferably pyrrolidinyl, tetrahydrofuranyl, tetrahydrothiophenyl, imidazolidinyl, thiazolidinyl, oxazolidinyl, tetrahydropyranyl, tetrahydrothiopyranyl, thiomorphlinyl, thiomorpholinyl-1,1-dioxide, piperidinyl, morpholinyl, or piperazinyl), -COOR_{y}, -CONR_{w}Rₓ, -N(R_{w})CON(R_{y})(Rₓ), -N(R_{y})CON(R_{w})(Rₓ), -N(R_{w})C(O)ORₓ, -N(R_{w})COR_{y}, -SR_{y}, -SOR_{y}, -SO₂R_{y}, -NR_{w}SO₂R_{y}, -NR_{w}SO₂Rₓ, -SO₃R_{y}, -OSO₂NR_{w}Rₓ, or -SO₂NR_{w}Rₓ;
   **R_{w}** and **Rₓ** are independently selected from: hydrogen, alkyl, alkenyl, aralkyl (wherein the aryl portion of said aralkyl is preferrably phenyl), or heteroaralkyl (wherein the heteroaryl portion of said heteroaralkyl is preferably pyrrolyl, furanyl, thiophenyl, imidazolyl, thiazolyl, oxazolyl, pyranyl, thiopyranyl, pyridinyl, pyrimidinyl, pyrazinyl, pyridinyl-N-oxide, or pyrrolyl-N-oxide, and most preferably pyrrolyl, furanyl, thiophenyl, imidazolyl, thiazolyl, oxazolyl, pyridinyl, pyrimidinyl, or pyrazinyl), or **R_{w}** and **Rₓ** may optionally be taken together to form a 5 to 7 membered ring, optionally containing a heteromoiety selected from O, NH, N(alkyl), SO₂, SO, or S, preferably selected from the group consisting of: 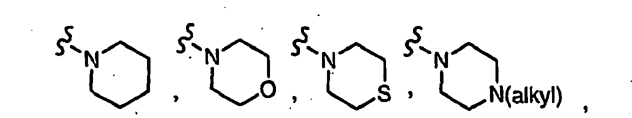 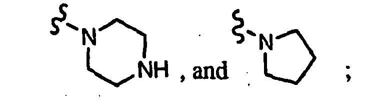
   **R_{y}** is selected from: hydrogen, alkyl, alkenyl, cycloalkyl (wherein said cycloalkyl is preferably cyclopentanyl or cyclohexanyl), phenyl, aralkyl (wherein the aryl portion of said aralkyl is preferably phenyl), heteroaralkyl (wherein the heteroaryl portion of said heteroaralkyl is preferably pyrrolyl, furanyl, thiophenyl, imidazolyl, thiazolyl, oxazolyl, pyranyl, thiopyranyl, pyridinyl, pyrimidinyl, pyrazinyl, pyridinyl-N-oxide, or pyrrolyl-N-oxide, and most preferably pyrrolyl, furanyl, thiophenyl, imidazolyl, thiazolyl, oxazolyl, pyridinyl, pyrimidinyl, or pyrazinyl), or heteroaryl (wherein said heteroaryl is preferably pyrrolyl, furanyl, thiophenyl, imidazolyl, thiazolyl, oxazolyl, pyranyl, thiopyranyl, pyridinyl, pyrimidinyl, pyrazinyl, pyridinyl-N-oxide, or pyrrolyl-N-oxide, and most preferably pyrrolyl, furanyl, thiophenyl, imidazolyl, thiazolyl, oxazolyl, pyridinyl, pyrimidinyl, or pyrazinyl);
   **R₅** is one, two, or three substituents independently selected from: halogen, cyano, trifluoromethyl, amino, hydroxyl, alkoxy, -C(O)alkyl, -SO₂alkyl, -C(O)N(alkyl)₂, alkyl, C(₁₋₄)alkyl-OH, or alkylamino; and
**R₃** is one or more substituents independently selected from; hydrogen, alkyl, alkoxy, halogen, alkoxyether, hydroxyl, thio, nitro, cycloalkyl optionally substituted with R₄ (wherein said cycloalkyl is preferably cyclopentanyl or cyclohexanyl), heteroaryl optionally substituted with R₄ (wherein said heteroaryl is preferably pyrrolyl, furanyl, thiophenyl, imidazolyl, thiazolyl, oxazolyl, pyranyl, thiopyranyl, pyridinyl, pyrimidinyl, triazolyl, pyrazinyl, pyridinyl-N-oxide, or pyrrolyl-N-oxide; and most preferably pyrrolyl, furanyl, thiophenyl, imidazolyl, thiazolyl, oxazolyl, pyridinyl, pyrimidinyl, triazolyl, or pyrazinyl), alkylamino, heterocyclyl optionally substituted with R₄ (wherein said heterocyclyl is preferably tetrahydropyridinyl. tetrahydropyrazinyl, dihydrofuranyl, dihydrooxazinyl, dihydropyrrolyl, dihydroimidazolyl, azepenyl, pyrrolidinyl, tetrahydrofuranyl, tetrahydrothiophenyl, imidazolidinyl, thiazolidinyl, oxazolidinyl, tetrahydropyranyl, tetrahydrothiopyranyl, piperidinyl, morpholinyl, or piperazinyl), -O(cycloalkyl), pyrrolidinonyl optionally substituted with R₄, phenoxy optionally substituted with R₄, -CN, -OCHF₂, -OCF₃, -CF₃, halogenated alkyl, heteroaryloxy optionally substituted with R₄, dialkylamino, -NHSO₂alkyl, thioalkyl, or -SO₂alkyl; wherein **R₄** is independently selected from halogen, cyano, trifluoromethyl, amino, hydroxyl, alkoxy, -C(O)alkyl, -CO₂alkyl, -SO₂alkyl, -C(O)N(alkyl)₂, alkyl, or alkylamino.

As used hereafter, the term "compounds of Formula I" is meant to include also the N-oxides, pharmaceutically acceptable salts, solvates, geometric isomers and stereochemical isomers thereof.

### EMBODIMENTS OF FORMULA I

In an embodiment of the present invention: N-oxides are optionally present on one or more of: N-1 or N-3 (see Figure 1a below for ring numbers).

### Figure 1a

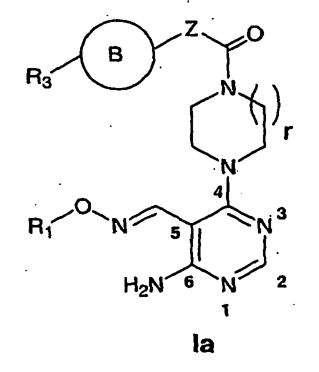

*Figure 1a illustrates ring atoms numbered 1 through 8, as used in the present specification*.

In an embodiment of the present invention, the oximine group (-O-N=C-) at postion 5 can be of either the E or the Z configuration.

Preferred embodiments of the invention are compounds of Formula I wherein one or more of the following limitations are present:
**r** is 1 or 2;
**Z** is NH, N(alkyl), or CH₂;
**B** is phenyl or heteroaryl;
**R₁** is: 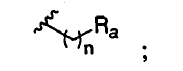
   wherein **n** is 1, 2, 3 or 4;
   **Rₐ** is hydrogen, alkoxy, phenoxy, phenyl, heteroaryl optionally substituted with R₅, hydroxyl, amino, alkylamino, dialkylamino, oxazolidinonyl optionally substituted with R₅, pyrrolidinonyl optionally substituted with R₅, piperidinonyl optionally substituted with R₅, cyclic heterodionyl optionally substituted with R₅, heterocyclyl optionally substituted with R₅, -COOR_{y}, -CONR_{w}Rₓ, -N(R_{w})CON(R_{y})(Rₓ), -N(R_{y})CON(R_{w})(Rₓ), -N(R_{w})C(O)ORₓ, -N(R_{w})COR_{y}, -SR_{y}, -SOR_{y}, -SO₂R_{y}, -NR_{w}SO₂R_{y}, -NR_{w}SO₂Rₓ, -SO₃R_{y} -OSO₂NR_{w}Rₓ, or -SO₂NR_{w}Rₓ;
   **R_{w}** and **Rₓ** are independently selected from: hydrogen, alkyl, alkenyl, aralkyl, or heteroaralkyl, or **R_{w}** and **Rₓ** may optionally be taken together to form a 5 to 7 membered ring, optionally containing a heteromoiety selected from O, NH, N(alkyl), SO₂, SO, or S;
   **R_{y}** is selected from: hydrogen, alkyl, alkenyl, cycloalkyl, phenyl, aralkyl, heteroaralkyl, or heteroaryl;
   **R₅** is one, two, or three substituents independently selected from: halogen, cyano, trifluoromethyl, amino, hydroxyl, alkoxy, -C(O)alkyl, -SO₂alkyl, -C(O)N(alkyl)₂, alkyl, -C(₁₋₄)alkyl-OH, or alkylamino; and
**R₃** is one or more substituents independently selected from: hydrogen, alkyl, alkoxy, halogen, alkoxyether, hydroxyl, thio, nitro, cycloalkyl optionally substituted with R₄, heteroaryl optionally substituted with R₄, alkylamino, heterocyclyl optionally substituted with R₄, -O(cycloalkyl), pyrrolidinonyl optionally substituted with R₄, phenoxy optionally substituted with R₄, -CN, -OCHF₂, -OCF₃, -CF₃, halogenated alkyl, heteroaryloxy optionally substituted with R₄, dialkylamino, -NHSO₂alkyl, thioalkyl, or -SO₂alkyl; wherein **R₄** is independently selected from: halogen, cyano, trifluoromethyl, amino, hydroxyl, alkoxy, -C(O)alkyl, -CO₂alkyl, -SO₂alkyl, -C(O)N(alkyl)₂, alkyl, or alkylamino.

Other preferred embodiments of the invention are compounds of Formula I wherein one or more of the following limitations are present:
**r** is 1 or 2;
**Z** is NH or CH₂;
**B** is phenyl or heteroaryl;
**R₁** is: 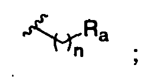
   wherein **n** is 1, 2, 3 or 4;
   **Rₐ** is hydrogen, alkoxy, phenoxy, phenyl, heteroaryl optionally substituted with R₅, hydroxyl, amino, alkylamino, dialkylamino, oxazolidinonyl optionally substituted with R₅, pyrrolidinonyl optionally substituted with R₅, piperidinonyl optionally substituted with R₅, cyclic heterodionyl optionally substituted with R₅, heterocyclyl optionally substituted with R₅, -COOR_{y}, -CONR_{w}Rₓ, -N(R_{w})CON(R_{y})(Rₓ), -N(R_{y})CON(R_{w})(Rₓ), -N(R_{w})C(O)ORₓ, -N(R_{w})COR_{y}, -SR_{y}, -SOR_{y}, -SO₂R_{y}, -NR_{w}SO₂R_{y}, -NR_{w}SO₂Rₓ, -SO₃R_{y}, -OSO₂NR_{w}Rₓ, or -SO₂NR_{w}Rₓ;
   **R_{w}** and **Rₓ** are independently selected from: hydrogen, alkyl, alkenyl, aralkyl, or heteroaralkyl, or **R_{w}** and **Rₓ** may optionally be taken together to form a 5 to' 7 membered ring, optionally containing a heteromoiety selected from O, NH, N(alkyl), SO₂, SO, or S;
   **R_{y}** is selected from: hydrogen, alkyl, alkenyl, cycloalkyl, phenyl, aralkyl, heteroaralkyl, or heteroaryl;
   **R₅** is one, two, or three substituents independently selected from: halogen, cyano, trifluoromethyl, amino, hydroxyl, alkoxy, -C(O)alkyl, -SO₂alkyl, -C(O)N(alkyl)₂, alkyl, -C(₁₋₄)alkyl-OH, or alkylamino; and
**R₃** is one or more substituents independently selected from: hydrogen, alkyl, alkoxy, halogen, alkoxyether, hydroxyl, cycloalkyl optionally substituted with R₄, heteroaryl optionally substituted with R₄, heterocyclyl optionally substituted with R₄, -O(cycloalkyl), phenoxy optionally substituted with R₄, heteroaryloxy optionally substituted with R₄, dialkylamino, or -SO₂alkyl; wherein **R₄** is independently selected from halogen, cyano, trifluoromethyl, amino, hydroxyl, alkoxy, -C(O)alkyl, -CO₂alkyl, -SO₂alkyl, -C(O)N(alkyl)₂, alkyl, or alkylamino.

Still other preferred embodiments of the invention are compounds of Formula I wherein one or more of the following limitations are present:
**r** is 1 or 2;
**Z** is NH or CH₂;
**B** is phenyl or heteroaryl;
**R₁** is: 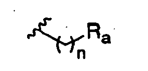
   wherein n is 1, 2, 3 or 4;
   **Rₐ** is hydrogen, alkoxy, heteroaryl optionally substituted with R₅, hydroxyl, amino, alkylamino, dialkylamino, oxazolidinonyl optionally substituted with R₅, pyrrolidinonyl optionally substituted with R₅, heterocyclyl optionally substituted with R₅, -CONR_{w}Rₓ, -N(R_{w})CON(R_{y})(Rₓ), -N(R_{y})CON(R_{w})(Rₓ), -N(R_{w})C(O)ORₓ, -N(R_{w})COR_{y}, -SO₂R_{y}, -NR_{w}SO₂R_{y}, or -SO₂NR_{w}Rₓ;
   **R_{w}** and **Rₓ** are independently selected from: hydrogen, alkyl, alkenyl, aralkyl, or heteroaralkyl, or **R_{w}** and **Rₓ** may optionally be taken together to form a 5 to 7 membered ring, optionally containing a heteromoiety selected from O, NH, N(alkyl), SO₂, SO, or S;
   **R_{y}** is selected from: hydrogen, alkyl, alkenyl, cycloalkyl, phenyl, aralkyl, heteroaralkyl, or heteroaryl;
   **R₅** is one, two, or three substituents independently selected from: halogen, cyano, trifluoromethyl, amino, hydroxyl, alkoxy, -C(O)alkyl, -SO₂alkyl, -C(O)N(alkyl)₂, alkyl, -C(₁₋₄)alkyl-OH, or alkylamino; and
**R₃** is one or more substituents independently selected from: hydrogen, alkyl, alkoxy, halogen, alkoxyether, hydroxyl, cycloalkyl optionally substituted with R₄, heteroaryl optionally substituted with R₄, heterocyclyl optionally substituted with R₄, -O(cycloalkyl), phenoxy optionally substituted with R₄, heteroaryloxy optionally substituted with R₄, dialkylamino, or -SO₂alkyl; wherein **R₄** is independently selected from halogen, cyano, trifluoromethyl, amino, hydroxyl, alkoxy, -C(O)alkyl, -CO₂alkyl, -SO₂alkyl, -C(O)N(alkyl)₂, alkyl, or alkylamino.

Particularly preferred embodiments of the invention are compounds of Formula I wherein one or more of the following limitations are present:
**r** is 1;
**Z** is NH or CH₂;
**B** is phenyl or heteroaryl;
**R₁** is 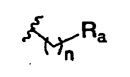
   wherein **n** is 1, 2, 3 or 4;
   **Rₐ** is hydrogen, hydroxyl, amino, alkylamino, dialkylamino, heteroaryl, heterocyclyl optionally substituted with R₅, -CONR_{w}Rₓ, -SO₂R_{y}, -NR_{w}SO₂R_{y}, -N(R_{y})CON(R_{w})(Rₓ), or -N(R_{w})C(O)ORₓ;
   **R_{w}** and **Rₓ** are independently selected from: hydrogen, alkyl, alkenyl, aralkyl, or heteroaralkyl; or **R_{w}** and **Rₓ** may optionally be taken to together to form a 5 to 7 membered ring, optionally containing a heteromoiety selected from O, NH, N(alkyl), SO, SO₂, or S;
   **R_{y}** is selected from: hydrogen, alkyl, alkenyl, cycloalkyl, phenyl, aralkyl, heteroaralkyl, or heteroaryl;
   **R₅** is one substituent independently selected from: -C(O)alkyl, -SO₂alkyl, -C(O)N(alkyl)₂, alkyl, or -C(₁₋₄)alkyl-OH; and
**R₃** is one substituent independently selected from: alkyl, alkoxy, halogen, cycloalkyl, heterocyclyl, -O(cycloalkyl), phenoxy, or dialkylamino.

Most particularly preferred embodiments of the invention are compounds of Formula I wherein one or more of the following limitations are present:
**r** is 1;
**Z** is NH or CH₂;
**B** is phenyl or pyridinyl;
**R₁** is: 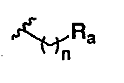
   wherein **n** is 1, 2, 3 or 4;
   **Rₐ** is hydrogen, dialkylamino, heterocyclyl optionally substituted with R₅, -CONR_{w}Rₓ, -N(R_{y})CON(R_{w})(Rₓ), or -NR_{w}SO₂R_{y};
   **R_{w}** and **Rₓ** are independently selected from: hydrogen, alkyl, alkenyl, aralkyl, heteroaralkyl, or **R_{w}** and **Rₓ** may optionally be taken together to form a 5 to 7 membered ring, optionally containing a heteromoiety selected from O, NH, N(alkyl), SO₂, SO, or S;
   **R_{y}** is selected from: hydrogen, alkyl, alkenyl, cycloalkyl, aralkyl, heteroaralkyl, or heteroaryl;
   **R₅** is one substituent independently selected from: -C(O)alkyl, -SO₂alkyl, -C(O)N(alkyl)₂, alkyl, or C(₁₋₄)alkyl-OH; and
**R₃** is one substituent independently selected from: alkyl, alkoxy, heterocyclyl, cycloalkyl, or -O(cycloalkyl).

### PHARMACEUTICALLY ACCEPTABLY SALTS

The compounds of the present invention may also be present in the form of pharmaceutically acceptable salts.

For use in medicines, the salts of the compounds of this invention refer to non-toxic "pharmaceutically acceptable salts." FDA approved pharmaceutically acceptable salt forms (Ref. International J. Pharm. 1986, 33, 201-217; J. Pharm. Sci., 1977, Jan, 66(1), p1) include pharmaceutically acceptable acidic/anionic or basic/cationic salts.

Pharmaceutically acceptable acidic/anionic salts include, and are not limited to acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate, pantothenate, phosphate/diphosphate, polygalacturonate, salicylate, stearate, subacetate, succinate, sulfate, tannate, tartrate, teoclate, tosylate and triethiodide. Organic or inorganic acids also include, and are not limited to, hydriodic, perchloric, sulfuric, phosphoric, propionic, glycolic, methanesulfonic, hydroxyethanesulfonic, oxalic, 2-naphthalenesulfonic, p-toluenesulfonic, cyclohexanesulfamic, saccharinic or trifluoroacetic acid.

Pharmaceutically acceptable basic/cationic salts include, and are not limited to aluminum, 2-amino-2-hydroxymethyl-propane-1,3-diol (also known as tris(hydroxymethyl)aminomethane, tromethane or "TRIS"), ammonia, benzathine, *t*-butylamine, calcium, calcium gluconate, calcium hydroxide, chloroprocaine, choline, choline bicarbonate, choline chloride, cyclohexylamine, diethanolamine, ethylenediamine, lithium, LiOMe, L-lysine, magnesium, meglumine, NH₃, NH₄OH, N-methyl-D-glucamine, piperidine, potassium, potassium-*t*-butoxide, potassium hydroxide (aqueous), procaine, quinine, sodium, sodium carbonate, sodium-2-ethylhexanoate (SEH), sodium hydroxide, triethanolamine (TEA) or zinc.

### STEREOCHEMICAL ISOMERS

One skilled in the art will recognize that the compounds of Formula I may have one or more asymmetric carbon atoms in their structure. It is intended that the present invention include within its scope single enantiomer forms of the compounds, racemic mixtures, and mixtures of enantiomers in which an enantiomeric excess is present.

The term "single enantiomer" as used herein defines all the possible homochiral forms which the compounds of Formula I and their N-oxides, addition salts, quaternary amines or physiologically functional derivatives may possess.

Stereochemically pure isomeric forms may be obtained by the application of art known principles. Diastereoisomers may be separated by physical separation methods such as fractional crystallization and chromatographic techniques, and enantiomers may be separated from each other by the selective crystallization of the diastereomeric salts with optically active acids or bases or by chiral chromatography. Pure stereoisomers may also be prepared synthetically from appropriate stereochemically pure starting materials, or by using stereoselective reactions.

The term "isomer" refers to compounds that have the same composition and molecular weight but differ in physical and/or chemical properties. Such substances have the same number and kind of atoms but differ in structure. The structural difference may be in constitution (geometric isomers) or in an ability to rotate the plane of polarized light (enantiomers).

The term "stereoisomer" refers to isomers of identical constitution that differ in the arrangement of their atoms in space. Enantiomers and diastereomers are examples of stereoisomers.
The term "chiral" refers to the structural characteristic of a molecule that makes it impossible to superimpose it on its mirror image.

The term "enantiomer" refers to one of a pair of molecular species that are mirror images of each other and are not superimposable.

The term "diastereomer" refers to stereoisomers that are not mirror images.

The symbols "*R*" and "*S*" represent the configuration of substituents around a chiral carbon atom(s).

The term "racemate" or "racemic mixture" refers to a composition composed of equimolar quantities of two enantiomeric species, wherein the composition is devoid of optical activity.

The term "homochiral" refers to a state of enantiomeric purity.

The term "optical activity" refers to the degree to which a homochiral molecule or nonracemic mixture of chiral molecules rotates a plane of polarized light.

The term "geometric isomer" refers to isomers that differ in the orientation of substituent atoms in relationship to a carbon-carbon double bond, to a cycloalkyl ring or to a bridged bicyclic system. Substituent atoms (other than H) on each side of a carbon-carbon double bond may be in an E or Z configuration. In the "E" (opposite sided) configuration, the substituents are on opposite sides in relationship to the carbon- carbon double bond; in the "Z" (same sided) configuration, the substituents are oriented on the same side in relationship to the carbon-carbon double bond. Substituent atoms (other than hydrogen) attached to a carbocyclic ring may be in a cis or trans configuration. In the "cis" configuration, the substituents are on the same side in relationship to the plane of the ring; in the "trans" configuration, the substituents are on opposite sides in relationship to the plane of the ring. Compounds having a mixture of "cis" and "trans" species are designated "cis/trans".

It is to be understood that the various substituent stereoisomers, geometric isomers and mixtures thereof used to prepare compounds of the present invention are either commercially available, can be prepared synthetically from commercially available starting materials or can be prepared as isomeric mixtures and then obtained as resolved isomers using techniques well-known to those of ordinary skill in the art.

The isomeric descriptors "R," "S," "E," "Z," "cis," and "trans" are used as described herein for indicating atom configuration(s) relative to a core molecule and are intended to be used as defined in the literature (IUPAC Recommendations for Fundamental Stereochemistry (Section E), Pure Appl. Chem., 1976, 45:13-30).

The compounds of the present invention may be prepared as individual isomers by either isomer-specific synthesis or resolved from an isomeric mixture. Conventional resolution techniques include forming the free base of each isomer of an isomeric pair using an optically active salt (followed by fractional crystallization and regeneration of the free base), forming an ester or amide of each of the isomers of an isomeric pair (followed by chromatographic separation and removal of the chiral auxiliary) or resolving an isomeric mixture of either a starting material or a final product using preparative TLC (thin layer chromatography) or a chiral HPLC column.

### POLYMORPHS AND SOLVATES

Furthermore, compounds of the present invention may have one or more polymorph or amorphous crystalline forms and as such are intended to be included in the scope of the invention. In addition, some of the compounds may form solvates, for example, with water (i.e., hydrates) or common organic solvents, and such are also intended to be encompassed within the scope of this invention. As used herein, the term "solvate" means a physical association of one or more compounds of the present invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. The term "solvate" is intended to encompass both solution-phase and isolatable solvates. Non-limiting examples of suitable solvates include ethanolates, methanolates, and the like. It is intended that the present invention include within its scope solvates of the compounds of the present invention. Thus, in the methods of treatment of the present invention, the term "administering" shall encompass the means for treating, ameliorating or preventing a syndrome, disorder or disease described herein with a compound specifically disclosed or a compound, or solvate thereof, which would obviously be included within the scope of the invention albeit not specifically disclosed for certain of the instant compounds.

### N-OXIDES

The compounds of Formula I may be converted to the corresponding N-oxide forms following art-known procedures for converting a trivalent nitrogen into its N-oxide form. Said N-oxidation reaction may generally be carried out by reacting the starting material of Formula I with an appropriate organic or inorganic peroxide. Appropriate inorganic peroxides comprise, for example, hydrogen peroxide, alkali metal or earth alkaline metal peroxides, e.g, sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. tbutyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

### TAUTOMERIC FORMS

Some of the compounds of Formula I may also exist in their tautomeric forms. Such forms although not explicitly indicated in the present application are intended to be included within the scope of the present invention.

### PREPARATION OF COMPOUNDS OF THE PRESENT INVENTION

During any of the processes for preparation of the compounds of the present invention, it may be necessary and/or desirable to protect sensitive or reactive groups on any of the molecules concerned. This may be achieved by means of conventional protecting groups, such as those described in Protecting Groups, P. Kocienski, Thieme Medical Publishers, 2000; and T.W. Greene & P.G.M. Wuts, Protective Groups in Organic Synthesis, 3rd ed. Wiley Interscience, 1999. The protecting groups may be removed at a convenient subsequent stage using methods known in the art.

### General Reaction Scheme

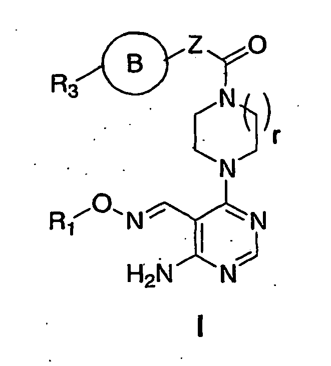

Compounds of formula **I** can be prepared by methods known to those who are skilled in the art. The following reaction schemes are only meant to represent examples of the invention and are in no way meant to be a limit of the invention.

The compounds of formula **I**, wherein B, Z, r, R₁, and R₃ are defined as in Formula I, may be synthesized as outlined by the general synthetic route illustrated in Scheme 1. Treatment of pyrimidine-4,6-diol **II** under Vilsmeier reaction conditions (DMF/POCl₃) can provide 4,6-dichloro-pyrimidine-5-carbaldehyde **III,** which upon treatment with ammonia can provide the key intermediate 4-amino-6-chloropyrimidine-5-carbaldehyde **IV.** Treatment of **IV** with a cyclic amine **V** in a solvent such as DMSO at a temperature of 25 °C to 150 °C in the presence of a base such as diisopropylethylamine can provide the pyrimidine **VI.** Treatment of **VI** with the appropriate R₁ONH₂ in a solvent such as MeOH can provide the final product **I.** Although only the anti form of Formula **I** is pictured, it is expected that both the anti and syn geometric isomers may be formed in the final reaction. The isomers may be separable by column chromatography and are spectrascopically distinct via ¹H NMR chemical shifts of the corresponding methine hydrogen Hₐ of the oxime (Figure 1b).

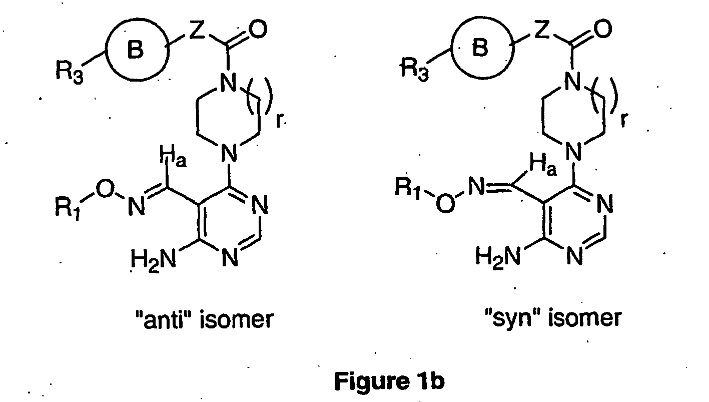

The observed ¹H NMR spectra of the major anti isomer show a characteristic further downfield chemical shift of the Hₐ methine hydrogen as compared to the Hₐ methine hydrogen chemical shift of the syn isomer. The observed difference in ¹H chemical shifts of the Hₐ hydrogen of the anti and syn oxime isomers correlates with literature known in the art (Biorg. Med. Chem. Lett. 2004, 14, 5827-5830).

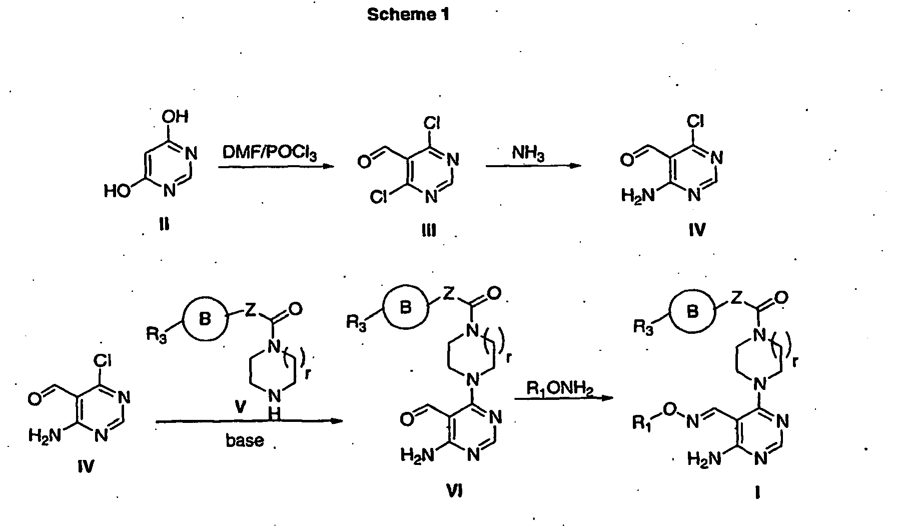

The R₁ONH₂ reagents, wherein R₁ is defined as in Formula **I**, are either commercially available or can be prepared by the reaction sequence illustrated in Scheme 2a. Alkylation of benzylidene **VII** with an appropriate electrophile R₁LG, where LG may be a leaving group such as bromide or iodide, and a base such as KOH in a solvent such as DMSO can provide the benzylidene intermediate **VIII,** which upon treatment under acidic conditions such as 4N HCl can provide the desired R₁ONH₂ reagent. A related method to prepare the R₁ONH₂ reagents, wherein n, R₁, and Rₐ are defined as in Formula I, is illustrated in Scheme 2b. Alkylation of benzylidene **VII** with an appropriate electrophile PGO(CH₂)ₙLG, where PG is a known alcohol protecting group and LG may be a leaving group such as bromide or iodide, with a base such as KOH in a solvent such as DMSO can provide the O-alkylated benzylidene. Deprotection of the alcohol protecting group known to those skilled in the art under standard conditions, conversion of the alcohol to an appropriate leaving group known by those skilled in the art such as a mesylate, and a subsequent SN₂ displacement reaction with an appropriate nucleophilic heterocycle, heteroaryl, amine, alcohol, sulfonamide, or thiol followed by acid mediated benzylidene removal can provide the R₁ONH₂ reagent. If Rₐ nucleophile is a thiol, further oxidation of the thiol can provide the corresponding sulfoxides and sulfones. If Rₐ nucleophile is an amino, acylation of the nitrogen with an appropriate acylating or sulfonylating agent can provide the corresponding amides, carbamates, ureas, and sulfonamides. If the desired Rₐ is COOR_{y} or CONR_{w}Rₓ, these can be derived from the corresponding hydroxyl group. Oxidation of the hydroxyl group to the acid followed by ester or amide formation under conditions known in the art can provide examples wherein Rₐ is COOR_{y} or CONR_{w}Rₓ.

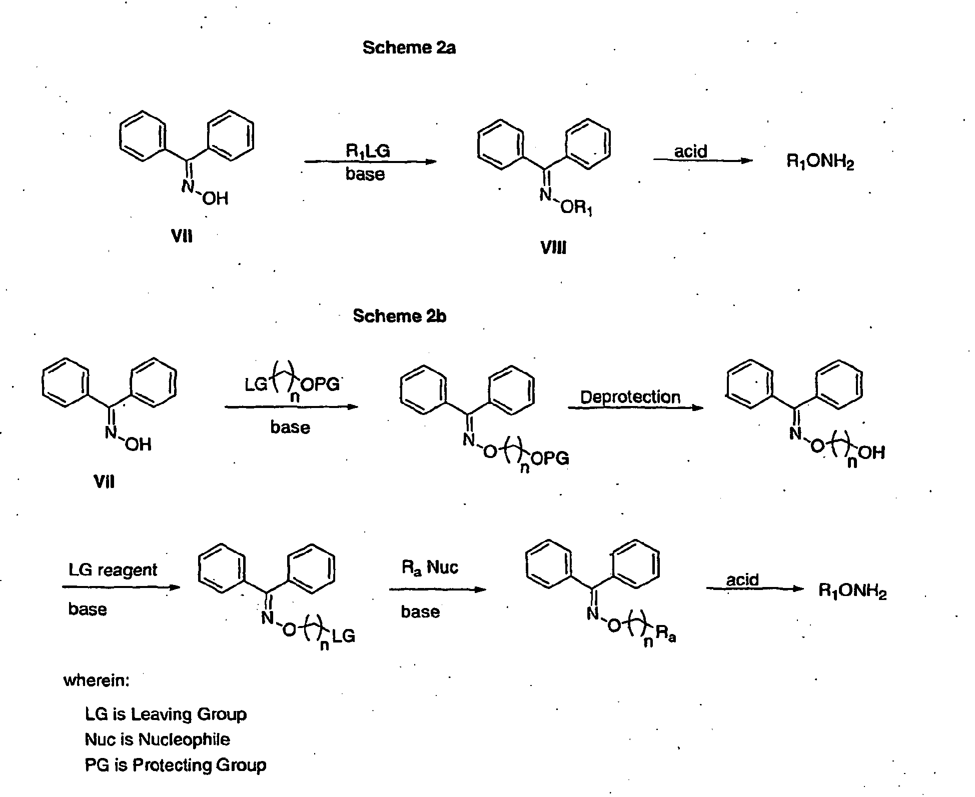

The amine reagents **V,** wherein Z is NH or N(alkyl) and B, r, and R₃ are defined as in Formula **I,'can** be prepared by the reaction sequence illustrated in Scheme 3a. Acylation of N-Boc diamine **IX** with an appropriate acylating agent **X,** where LG may be p-nitrophenoxy, chloride, bromide, or imidazole, can provide the acylated intermediate **XI.** Removal of the N-Boc protecting group under acidic conditions can provide the desired amine **V.** The acylating reagents **X** are either commercially available or can be prepared as illustrated in Scheme 3a. Treatment of an appropriate R₃BZH, wherein Z is NH or N(alkyl), with an appropriate acylating reagent such as carbonyldiimidazole or p-nitrophenylchloroformate (wherein LG may be chloride, imidazole, or p-nitrophenoxy) in the presence of a base such as triethylamine can provide **X**. Many R₃BZH reagents are either commercially available and can be prepared by a number of known methods (e.g.Tet Lett 1995, 36, 2411-2414). An alternative method of accessing **V**, wherein Z is CH₂ and B, r, and R₃ are defined as in Formula I, is outlined in Scheme 3b. Coupling of a cyclic amine **IX** with an appropriate R₃BCH₂CO₂H using a standard coupling reagent such as 1-(3-dimethylaminopropyl)-3-ethylcarbodiimide hydrochloride (EDC) or 1-hydroxybenzotriazole (HOBT) can provide the acylated intermediate **XI.** Removal of the N-Boc protecting group under acidic conditions can provide the desired amine **V.**

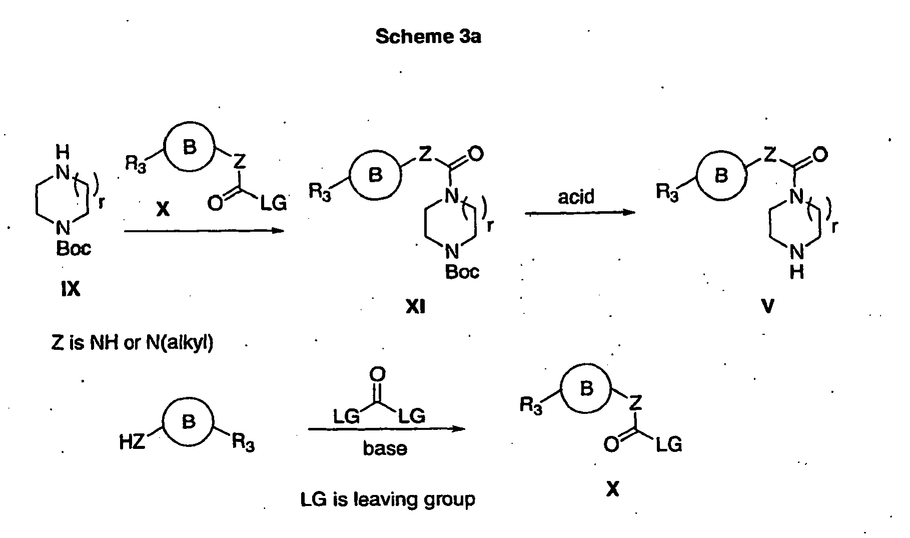

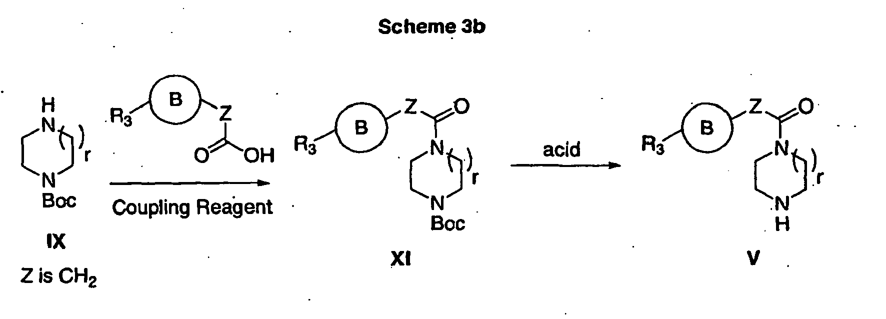

Alternatively compounds of formula **I,** wherein B, Z, r, R₁, and R₃ are defined as in Formula I, may be synthesized as outlined by the general synthetic route illustrated in Scheme 4. Treatment of 4-chloropyrimidine **IV** with an appropriate diamine **IX** in a solvent such as acetonitrile in the presence of a base such as diisopropylethylamine can provide the pyrimidine **XII.** Treatment of the 5-carbaldehyde pyrimidine **XII** with an appropriate R₁ONH₂ in a solvent such as MeOH can yield intermediate **XIII,** which upon subsequent deprotection of the N-Boc protecting group by acid treatment can provide the diamino pyrimidine **XIV.** Acylation of **XIV** in the presence of a base such as diisopropylethylamine with an appropriate reagent **X,** wherein Z is NH or N(alkyl) and LG may be chloride, imidazole, or p-nitrophenoxy, or, when Z is CH₂, via coupling with an appropriate R₃BCH₂CO₂H using a standard coupling reagent such as 1-(3-dimethylaminopropyl)-3-ethylcarbodiimide hydrochloride (EDC) or 1-hydroxybenzotriazole (HOBT), can provide the final product **I.** Although only the anti form of Formula **I** is pictured, it is expected that both the anti and syn geometric isomers may be formed in the reaction sequence. The isomers can be separated by column chromatography and are spectrascopically distinct.

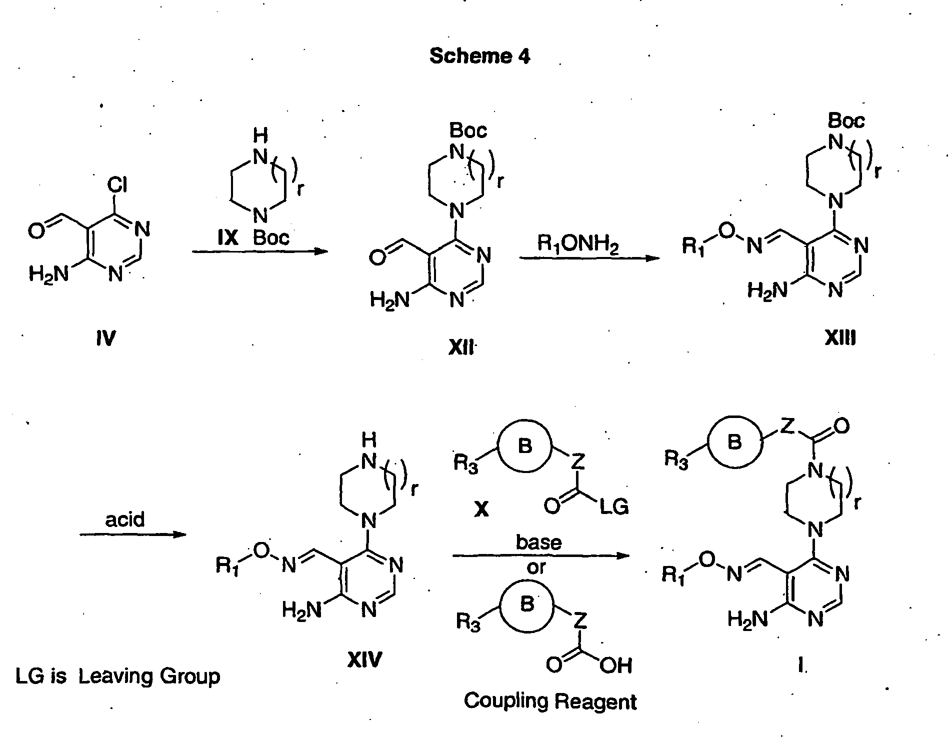

Alternatively compounds of formula **I,** wherein Z is NH and B, r, R₁, and R₃ are defined as in Formula I, may be synthesized as outlined by the general synthetic route illustrated in Scheme 5. Treatment of 4-chloropyrimidine **IV** with an appropriate diamine **IX** in a solvent such as acetonitrile in the presence of a base such as diisopropylethylamine can provide the pyrimidine **XII.** Treatment of the 5-carbaldehyde pyrimidine **XII** with an appropriate R₁ONH₂ in a solvent such as MeOH can yield intermediate **XIII,** which upon subsequent deprotection of the N-Boc protecting group by acid treatment can provide the diamino pyrimidine **XIV.** Acylation of **XIV** in the presence of a base such as diisopropylethylamine with an appropriate R₃BNCO can provide the final product **I**. Although only the anti form of Formula **I** is pictured, it is expected that both the anti and syn geometric isomers may be formed in the reaction sequence. The isomers can be separated by column chromatography and are spectrascopically distinct.

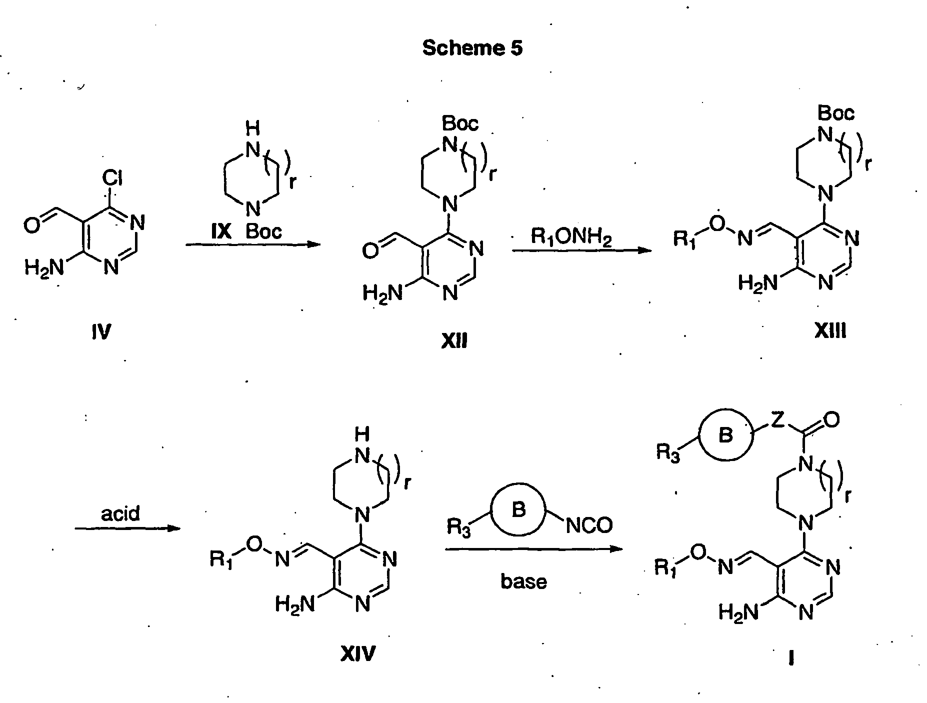

An alternative method to prepare compounds of formula **I,** wherein Z is NH or N(alkyl) and B, r, R₁, and R₃ are defined as in Formula **I,** is outlined by the general synthetic route illustrated in Scheme 6. Treatment of 4-chloropyrimidine **IV** with an appropriate diamine **IX** in a solvent such as acetonitrile in the presence of a base such as diisopropylethylamine can provide the pyrimidine **XII.** Deprotection of the N-Boc protecting group by acid treatment can provide the diamino pyrimidine **XV,** which can be subsequently acylated with an appropriate reagent **X,** wherein LG may be chloride, imidazole, or p-nitrophenoxy, in the presence of a base such as diisopropylethylamine to provide pyrimidine **XVI.** Treatment of the 5-carbaldehyde pyrimidine **XVI** with an appropriate R₁ONH₂ in a solvent such as MeOH can provide the final product **I.** Although only the anti form of Formula **I** is pictured, it is expected that both the anti and syn geometric isomers may be formed in the final reaction. The isomers are separable by column chromatography and are spectrascopically distinct.

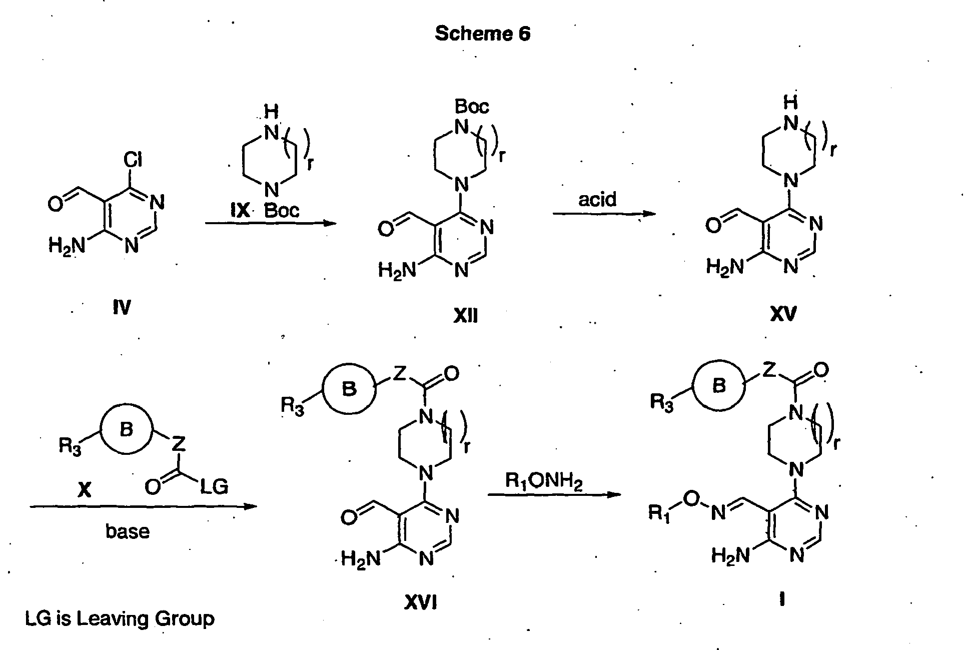

### REPRESENTATIVE COMPOUNDS

Representative compounds of the present invention synthesized by the aforementioned methods are presented below. Examples of the synthesis of specific compounds are presented thereafter. Preferred compounds are numbers 1, 2, 7, 12, 13, 16, 17, 18, 19, 27; particularly preferred are numbers 1, 2, 7, 12 and 17.

| **Number** | **Compound** |
|---|---|
| 1 | 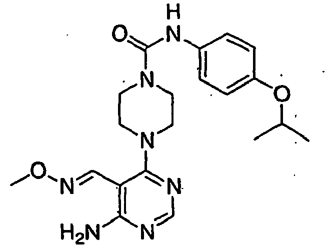 |
| 2 | 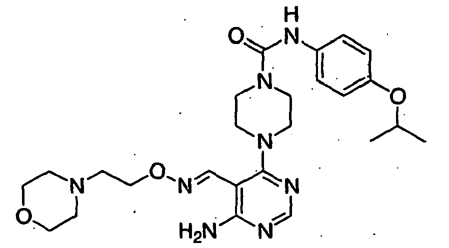 |
| 3 | 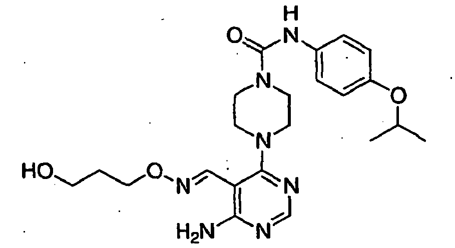 |
| 4 | 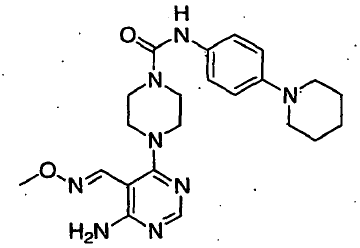 |
| 5 | 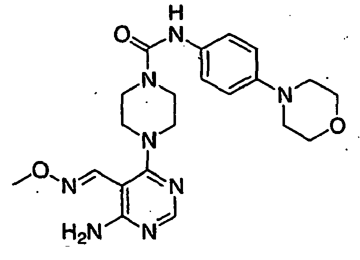 |
| 6 | 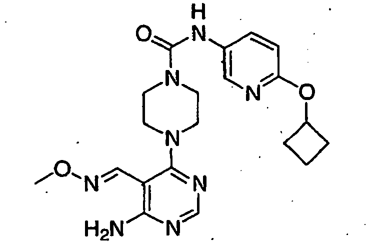 |
| 7 | 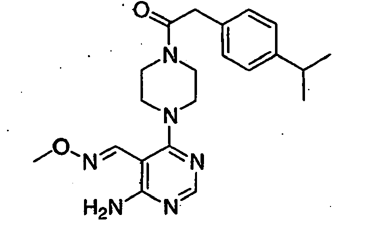 |
| 8 | 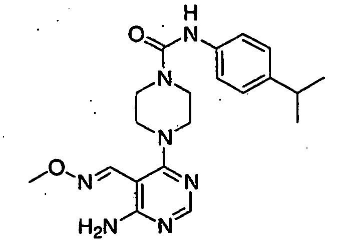 |
| 9 | 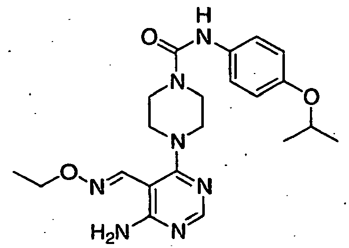 |
| 10 | 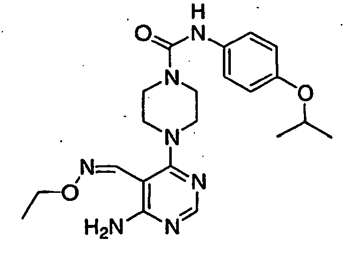 |
| 11 | 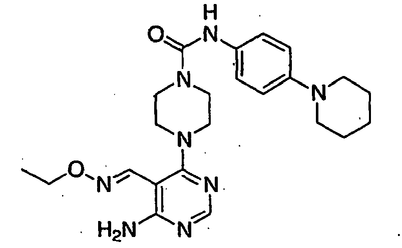 |
| 12 | 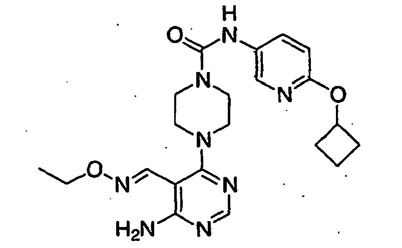 |
| 13 | 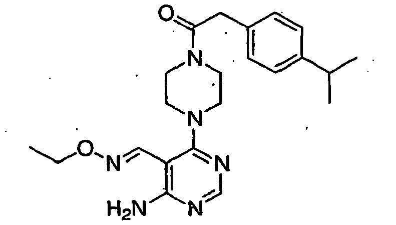 |
| 14 | 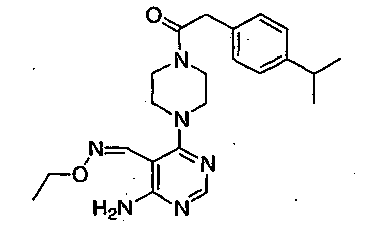 |
| 15 | 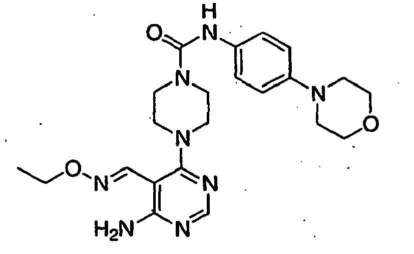 |
| 16 | 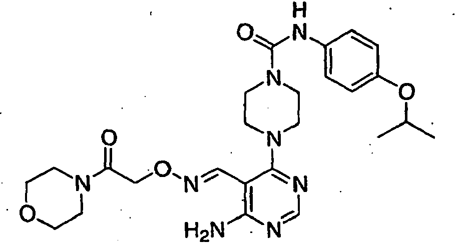 |
| 17 | 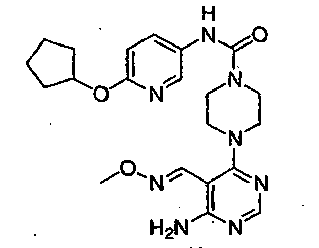 |
| 18 | 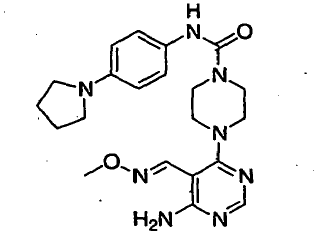 |
| 19 | 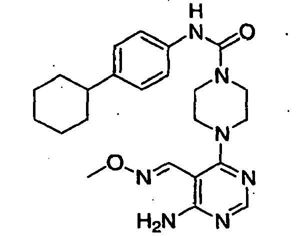 |
| 20 | 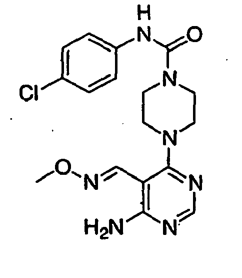 |
| 21 | 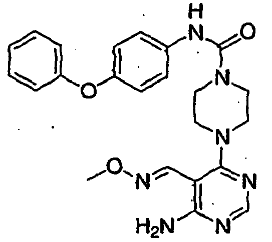 |
| 22 | 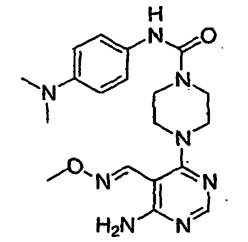 |
| 23 | 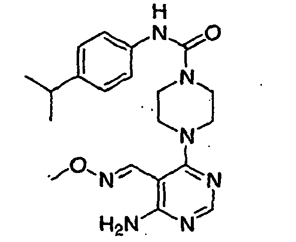 |
| 24 | 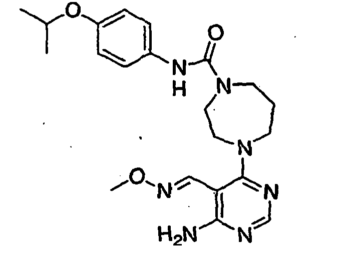 |
| 25 | 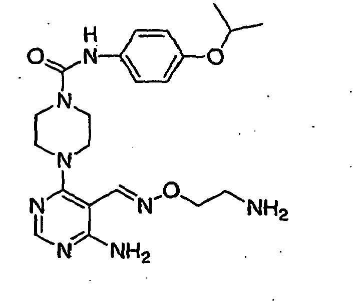 |
| 26 | 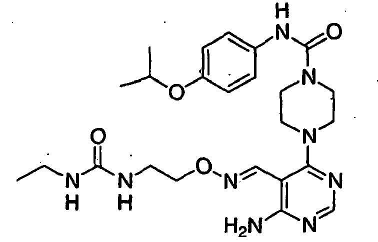 |
| 27 | 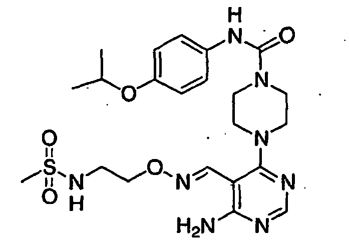 |
| 28 | 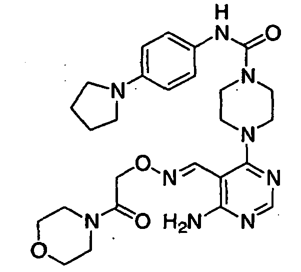 |
| 29 | 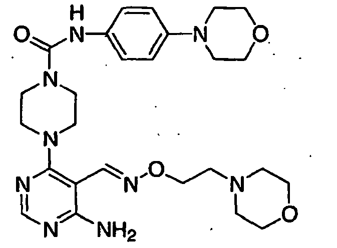 |
| 30 | 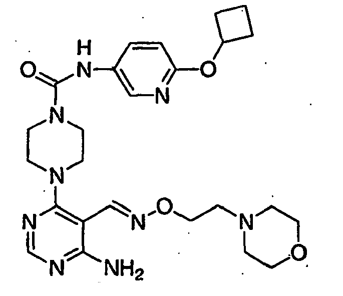 |
| 31 | 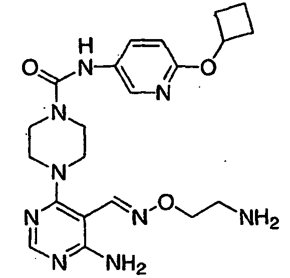 |
| 32 | 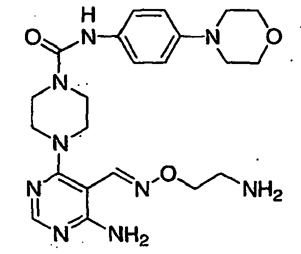 |
| 33 | 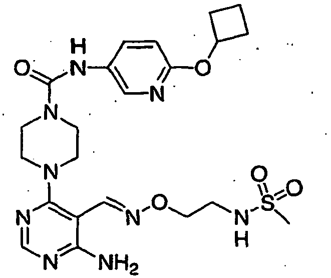 |
| 34 | 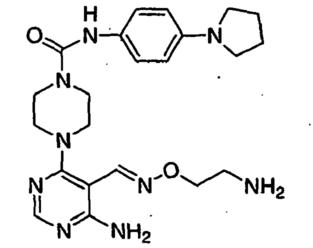 |
| 35 | 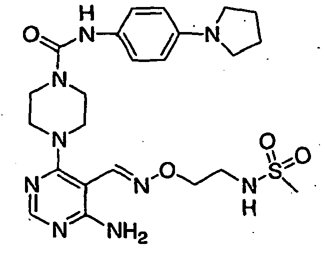 |
| 36 | 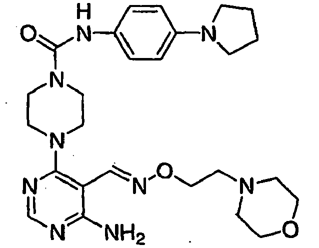 |
| 37 | 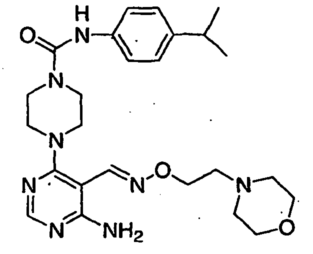 |

### EXAMPLE 1

### 4-[6-Amino-5-(methoxyimino-methyl)-pyrimidin-4-yl]-piperazine-1-carboxylic acid (4-isopropoxy-phenyl)-amide

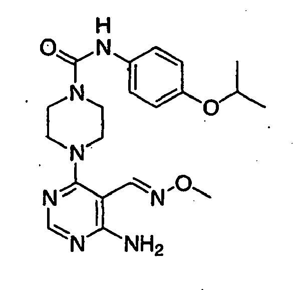

### a. 4,6-Dichloro-pyrimidine-5-carbaldehyde

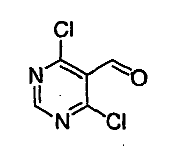

A mixture of DMF (3.2 mL) and POCl₃ (10 mL) at 0 °C was stirred for 1 h, treated with 4,6-dihydroxypyrimidine (2.5 g, 22.3 mmol), and stirred for 0.5 h at ambient temperature. The heterogeneous mixture was heated at reflux for 3 h and the volatiles were removed at reduced pressure. The residue was poured into ice water and extracted six times with ethyl ether. The organic phase was washed with aqueous NaHCO₃, dried over Na₂SO₄ and concentrated to afford a yellow solid (3.7 g, 95%). ¹H NMR (CDCl₃) δ 10.46 (s, 1H), 8.90 (s, 1H).

### b. 4-Amino-6-chloro-pyrimidine-5-carbaldehyde

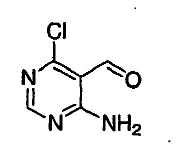

Ammonia was bubbled through a solution of 4,6-dichloro-pyrimidine-5-carbaldehyde (1g, 5.68 mmol) in toluene (100 mL) for 10 min and the solution was stirred at room temperature overnight. The yellow precipitate was filtered off, washed with EOAc and dried *in vacuo* to afford the pure product (880 mg, 99%). ¹H NMR (DMSO-d₆) δ 10.23 (s, 1H), 8.72 (br, 1H), 8.54 (br, 1H), 8.38 (s, 1H).

### Method A:

### a. 4-(6-Amino-5-formyl-pyrimidin-4-yl)-piperazine-1-carboxylic acid tert-butyl ester

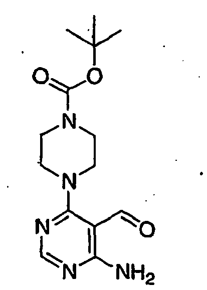

To a suspension of 4-amino-6-chloro-pyrimidine-5-carbaldehyde (446.8 mg, 2.85 mmol) in CH₃CN (2 mL) was added piperazine-1-carboxylic acid *tert*-butyl ester (583.1 mg, 3.13 mmol), followed by DIEA (736.7 mg, 5.7 mmol). The reaction mixture was stirred at 100 °C. After 2 h it was cooled to room temperature and the precipitate was filtered off, washed with CH₃CN (3 × 4 mL) and dried *in vacuo* to afford the title compound as a white powder (818 mg, 93.6%). ¹H NMR (DMSO-d₆) δ 9.75 (s, 1H), 8.28 (br, 1H), 8.07 (s, 1H), 7.83 (br, 1H), 3.59 (m, 4H), 3.43 (m, 4H), 1.41 (s, 9H); LC/MS (ESI) calcd for C₁₄H₂₂N₅O₃ (MH)⁺ 308.2, found 308.2.

### b. 4-[6-Amino-5-(methoxyimino-methyl)-pyrimidin-4-yl]-piperazine-1-carboxylic acid tert-butyl ester

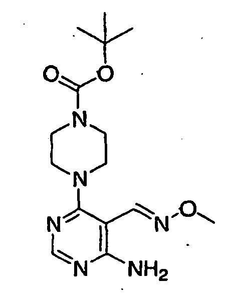

A mixture of 4-(6-amino-5-formyl-pyrimidin-4-yl)-piperazine-1-carboxylic acid *tert-*butyl ester (59.1 mg, 0.19 mmol) and MeONH₂.HCl (52 mg, 0.62 mmol) in MeOH (1.5 mL) was stirred at 75 °C for 0.5 h and the solvent was evaporated under reduced pressure. The crude residue was purified by flash column chromatography on silica gel (EtOAc as eluent) to afford the title compound as a white solid (48 mg, 74.6%). ¹H NMR (CDCl₃) δ 8.19 (s, 1H), 8.11 (s, 1H), 3.95 (s, 3H), 3.53 (t, *J* = 5.10 Hz, 4H), 3.33 (t, *J* = 5.10 Hz, 4H), 1.47 (s, 9H); LC/MS (ESI) calcd for C₁₅H₂₅N₆O₃ (MH)⁺ 337.2, found 337.3.

### c. 4-Amino-6-piperazin-1-yl-pyrimidine-5-carbaldehyde O-methyl-oxime trifluoroacetic acid salt

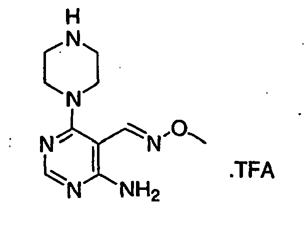

4-[6-Amino-5-(methoxyimino-methyl)-pyrimidin-4-yl]-piperazine-1-carboxylic acid *tert*-butyl ester (22.1 mg, 0.066 mmol) was treated with 50% TFA/ CH₂Cl₂ (4 mL). After 14 h, the mixture was evaporated and dried *in vacuo* to afford the title compound. ¹H NMR (CD₃OD) δ 8.29 (s, 1H), 8.15 (s, 1H), 4.00 (s, 3H), 3.93 (t, *J* = 5.16 Hz, 4H), 3.35 (t, *J* = 5.37 Hz, 4H); LC/MS (ESI) calcd for C₁₀H₁₇N₆O (MH)⁺ 237.1, found 237.2.

### d. (4-Isopropoxy-phenyl)-carbamic acid 4-nitro-phenyl ester

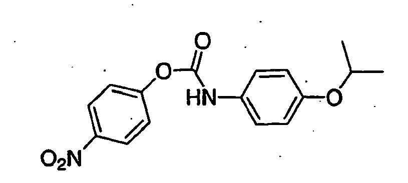

To a solution of 4-isopropoxyaniline (9.06 g, 60.0 mmol) in CH₂Cl₂ (120 mL) and pyridine (30 mL) was added 4-nitrophenyl chloroformate (10.9 g, 54.0 mmol) portionwise with stirring over -1 min with brief ice-bath cooling. After stirring at room temperature for 1 h, the homogeneous solution was diluted with CH₂Cl₂ (300 mL) and washed with 0.6 M HCl (1 × 750 mL) and 0.025 M HCl (1 × 1 L). The organic layer was dried (Na₂SO₄) and concentrated to give the title compound as a light violet-white solid (16.64 g, 98%). ¹H NMR (CDCl₃) δ 8.31-8.25 (m, 2H), 7.42-7.32 (m, 4H), 7.25-7.20 (m, 2H), 6.93 (br s, 1H), 2.90 (sep, *J* = 6.9 Hz, 1H), 1.24 (d, *J* = 6.9 Hz, 6H). LC/MS (ESI) calcd for C₁₆H₁₇N₂O₅ (MH)⁺ 317.1, found 633.2 (2MH)⁺.

### e. 4-[6-Amino-5-(methoxyimino-methyl)-pyrimidin-4-yl]-piperazine-1-carboxylic acid (4-isopropoxy-phenyl)-amide

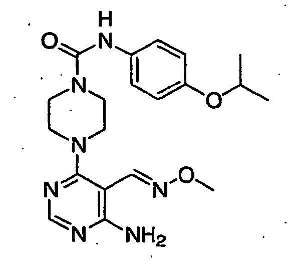

To a mixture of 4-amino-6-piperazin-1-yl-pyrimidine-5-carbaldehyde O-methyl-oxime trifluoroacetic acid salt (23 mg, 0.066 mmol) and (4-isopropoxy-phenyl)-carbamic acid 4-nitro-phenyl ester (22.8 mg, 0.072 mmol) in CH₃CN (1.5 mL) was added DIEA (17 mg, 0.13 mmol). The mixture was heated at reflux with stirring for 3 h and the solvents were evaporated under reduced pressure. The yellow residue was purified by flash column chromatography on silica gel (EtOAc as eluent) to afford the title compound as a white solid (12.7 mg, 46.8%). ¹H NMR (CDCl₃) δ 8.19 (s, 1H), 8.12 (s, 1H), 7.21 (d, *J* = 8.93 Hz, 2H), 6.81 (d, *J* = 8.94 Hz, 2H), 6.45 (br, 1H), 4.46 (m, 1H), 3.96 (s, 3H), 3.58 (m, 4H), 3.42 (m, 4H), 1.30 (d, *J* = 6.06 Hz, 6H); LC/MS (ESI) calcd for C₂₀H₂₈N₇O₃ (MH)⁺ 414.2, found 414.2.

### Method B:

### f. 4-(4-Isopropoxy-phenylcarbamoyl)-piperazine-1-carboxylic acid tert-butyl ester

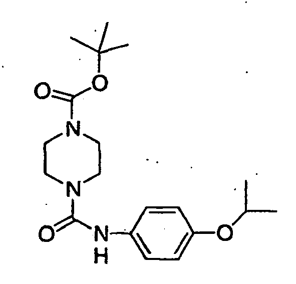

A mixture of piperazine-1-carboxylic acid *tert*-butyl ester (267.4 mg, 1.44 mmol) and (4-isopropoxy-phenyl)-carbamic acid 4-nitro-phenyl ester (432.1 mg, 1.36 mmol) in CH₃CN (2 mL) was heated at reflux for 2 h and cooled to room temperature. The precipitate was filtered off, washed with CH₃CN (3 × 3 mL) and dried *in vacuo* to yield the product as a white solid (459 mg, 93%). ¹H NMR (CD₃OD) δ 7.20 (d, J = 8.81 Hz, 2H), 6.82 (d, *J* = 8.93 Hz, 2H), 4.52 (sep, *J* = 6.03 Hz, 1H), 3.48 (m, 8H), 1.48 (s, 9H), 1.27 (d, *J* = 6.04 Hz, 6H); LC/MS (ESI) calcd for C₁₉H₃₀N₃O₄ (MH)⁺ 364.2, found 364.4.

### g. Piperazine-1-carboxylic acid (4-isopropoxy-phenyl)-amide

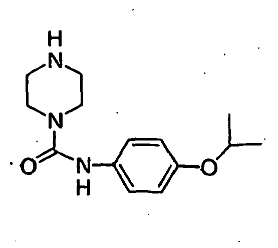

4-(4-Isopropoxy-phenylcarbamoyl)-piperazine-1-carboxylic acid *tert*-butyl ester (169 mg, 0.47 mmol) was treated with 50% TFA/ CH₂Cl₂ (15 mL). After 2h, it was evaporated under reduced pressure and the residue was neutralized with 2 M CH₃ in MeOH. Evaporation of the solvents under high vacuum yield the title compound (119 mg, 97%). ¹H NMR (CD₃OD) δ 7.22 (d, *J* = 8.83 Hz, 2H), 6.83 (d, *J* = 8.92 Hz, 2H), 4.52 (sep, *J* = 6.02 Hz, 1H), 3.76 (t, *J* = 4.98 Hz, 4H), 3.24 (t, *J* = 4.99 Hz, 4H), 1.27 (d, *J* = 6.03 Hz, 6H); LC/MS (ESI) calcd for C₁₄H₂₂N₃O₂ (MH)⁺ 264.2, found 264.3.

### h. 4-[6-Amino-5-(methoxyimino-methyl)-pyrimidin-4-yl]-piperazine-1-carboxylic acid (4-isopropoxy-phenyl)-amide

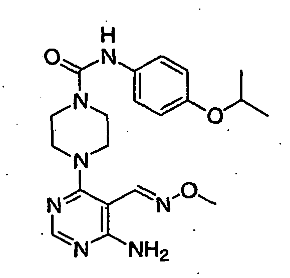

To a mixture of piperazine-1-carboxylic acid (4-isopropoxy-phenyl)-amide (302.1 mg, 1.15 mmol) and 4-amino-6-chloro-pyrimidine-5-carbaldehyde (157 mg, 1.0 mmol) in DMSO (2 mL) was added DIEA (258.5 mg, 2.0 mmol). The mixture was kept stirring at 100 °C for 2 h and MeONH₂.HCl (167 mg, 2.0 mmol) was added. The resulting mixture was heated at 100 °C for 0.5 h. It was diluted with water and extracted with CH₂Cl₂. The combined organic extracts were washed with brine, dried (Na₂SO₄) and concentrated under reduced pressure. The crude oil was subjected to flash column chromatography on silica gel (EtOAc as eluent) to yield the title compound (45 mg, 11%). ¹H NMR (CDCl₃) δ 8.19 (s, 1H), 8.12 (s, 1H), 7.21 (d, *J* = 8.93 Hz, 2H), 6.81 (d, *J* = 8.94 Hz, 2H), 6.45 (br, 1H), 4.46 (m, 1H), 3.96 (s, 3H), 3.58 (m, 4H), 3.42 (m, 4H), 1.30 (d, *J* = 6.06 Hz, 6H); LC/MS (ESI) calcd for C₂₀H₂₈N₇O₃ (MH)⁺ 414.2, found 414.4.

### EXAMPLE 2

### 4-{6-Amino-5-[(2-morpholin-4-yl-ethoxyimino)-methyl]-pyrimidin-4-yl}-piperazine-1-carboxylic acid (4-isopropoxy-phenyl)-amide

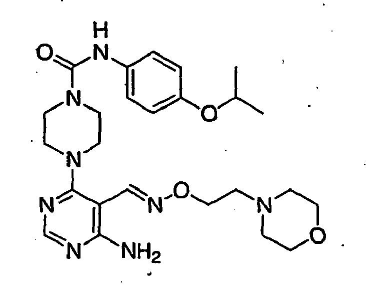

### a. 4-Amino-6-piperazin-1-yl-pyrimidine-5-carbaldehyde trifluoroacetic acid

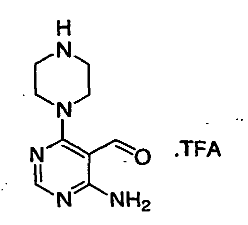

4-(6-Amino-5-formyl-pyrimidin-4-yl)-piperazine-1-carboxylic acid *tert*-butyl ester (235 mg, 0.76 mmol) was treated with 50% TFA/ CH₂Cl₂ (10 mL) and the mixture was stirred overnight. It was evaporated under reduced pressure to yield a white solid, which is pure and directly used for the next step reaction, ¹H NMR (CD₃OD) δ 9.83 (s, 1H), 8.29 (s, 1H), 4.22 (t, *J* = 5.23 Hz, 4H), 3.42 (t, *J* = 5.42 Hz, 4H); LC/MS (ESI) calcd for C₉H₁₄N₅O (MH)⁺ 208.1, found 208.1.

### b. 4-(6-Amino-5-formyl-pyrimidin-4-yl)-piperazine-1-carboxylic acid (4-isopropoxy-phenyl)-amide

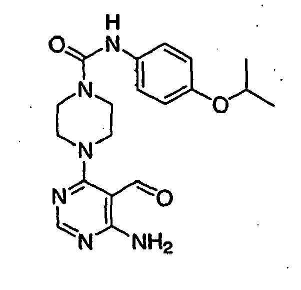

To a mixture of 4-Amino-6-piperazin-1-yl-pyrimidine-5-carbaldehyde trifluoroacetic acid salt (0.76 mmol) and (4-isopropoxy-phenyl)-carbamic acid 4-nitro-phenyl ester (253.7 mg, 0.80 mmol) in CH₃CN was added DIEA (396 mg, 3.06 mmol). The mixture was heated at 100 °C for 2 h, cooled to room temperature. The precipitate was filtered, washed with CH₃CN (2 × 2 mL) and EtOAc (2 × 1 mL) and dried *in vacuo* to afford the title compound as a light yellow solid (120 mg, 41%). ¹H NMR (CDCl₃) δ 9.88 (s, 1H), 8.73 (br, 1H), 8.17 (s, 1H), 7.22 (d, *J* = 8.97 Hz, 2H), 6.84 (d, *J* = 8.98 Hz, 2H), 6.50 (br, 1H), 6.25 (br, 1H), 4.49 (m, 1H), 3.85 (m, 4H), 3.66 (m, 4H), 1.31 (d, *J* = 6.06 Hz, 6H); LC/MS (ESI) calcd for C₁₉H₂₅N₆O₃ (MH)⁺ 385.2, found 385.2.

### c. Diphenyl-methanone O-(2-morpholin-4-yl-ethyl)-oxime

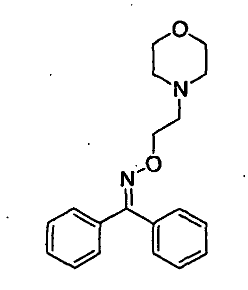

*N*-(2-Chloroethyl)morpholine hydrochloride (2.10 g, 11 mmol) was added, in portions, to a suspension of KOH powder (1.24 g, 22 mmol) and benzophenone oxime (1.97 g, 10 mmol) in DMSO (23 mL) at room temperature. The reaction mixture was kept stirring at room temperature for 3 days, diluted with water and extracted with ethyl ether. The organic phase was washed with brine, dried (Na₂SO₄) and evaporated to afford almost pure product. ¹H NMR (CDCl₃) δ 7.32-7.50 (m, 10H), 4.35 (t, *J* = 5.59 Hz, 2H), 3.69 (t, *J* = 4.52 Hz, 4H), 2.74 (m, 2H), 2.49 (m, 4H); LC/MS (ESI) calcd for C₁₉H₂₃N₂O₂ (MH)⁺ 311.2, found 311.2.

### d. O-(2-Morpholin-4-yl-ethyl)-hydroxylamine dihydrochloride

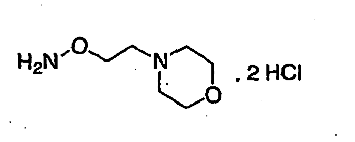

A suspension of diphenyl-methanone O-(2-morpholin-4-yl-ethyl)-oxime (2.5 g, 8.06 mmol) in 6N HCl (13.5 mL) was heated at reflux with stirring. After 2 h, the mixture was cooled to room temperature and extracted with EtOAc several times. The aqueous phase was evaporated to dryness *in vacuo* to afford the title compound (740 mg, 63%). ¹H NMR (DMSO-d₆) δ 4.45 (t, *J* = 4.49 Hz, 2H), 3.89 (t, *J* = 4.48 Hz, 4H), 3.47 (t, *J* = 4.64 Hz, 2H), 3.29 (m, 4H); LC/MS (ESI) calcd for C₆H₁₅N₂O₂ (MH)⁺ 147.1, found 147.1.

### e. 4-{6-Amino-5-[(2-morpholin-4-yl-ethoxyimino)-methyl]-pyrimidin-4-yl}-piperazine-1-carboxylic acid (4-isopropoxy-phenyl)-amide

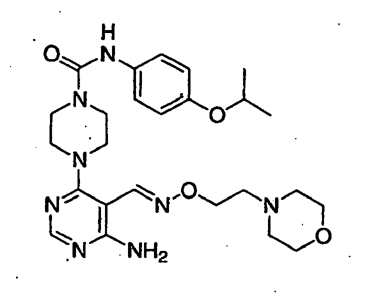

A mixture of 4-(6-amino-5-formyl-pyrimidin-4-yl)-piperazine-1-carboxylic acid (4-isopropoxy-phenyl)-amide (20.9 mg, 0.054 mmol) and O-(2-morpholin-4-yl-ethyl)-hydroxylamine dihydrochloride salt (12 mg, 0.054 mmol) in MeOH (1 mL) was heated at 100 °C for 0.5 h and the solvent was removed. The residue was partitioned between EtOAc and water. The organic extracts were dried (Na₂SO₄) and evaporated and the residue was purified by preparative TLC (5% MeOH/EtOAc) to yield the desired product as a white solid (16.4 mg, 58.9%). ¹H NMR (CD₃OD) δ 8.24 (s, 1H), 8.08 (s, 1H), 7.21 (d, *J* = 8.79 Hz, 2H), 6.83 (d, *J* = 9.03 Hz, 2H), 4.52 (m, 1H), 4.34 (t, *J* = 5.63 Hz, 2H), 3.71 (t, *J* = 4.84 Hz, 4H), 3.63 (m, 4H), 3.43 (m, 4H), 2.75 (t, *J* = 5.60 Hz, 2H), 2.57 (t, *J* = 4.96 Hz, 4H), 1.28 (d, *J* = 6.05 Hz, 6H); LC/MS (ESI) calcd for C₂₅H₃₇N₈O₄ (MH)⁺ 513.2, found 513.3.

### EXAMPLE 3

### 4-{6-Amino-5-[(3-hydroxy-propoxyimino)-methyl]-pyrimidin-4-yl}-piperazine-1-carboxylic acid (4-isopropoxy-phenyl)-amide

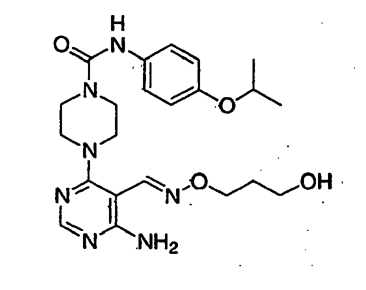

### a. Diphenyl-methanone O-(3-hydroxy-propyl)-oxime

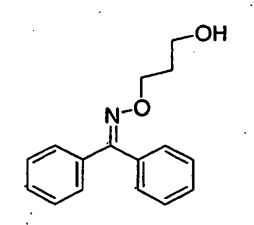

Following the procedure for the synthesis of Example 2c. ¹H NMR (CDCl₃) δ 7.30-7.52 (m, 10H), 4.35 (t, *J* = 5.83 Hz, 2H), 3.73 (t, J = 5.85 Hz, 2H), 1.95 (m, 2H).

### b. 3-Aminooxy-propan-1-ol hydrochloride

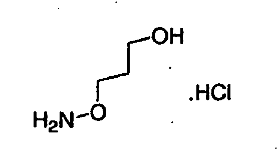

Following the procedure for the synthesis of Example 2d. ¹H NMR (CD₃OD) δ 4.26 (t, *J* = 6.75 Hz, 2H), 3.66 (t, *J* = 6.11 Hz, 2H), 2.51 (m, 2H).

### c. 4-{6-Amino-5-[(3-hydroxy-propoxyimino)-methyl]-pyrimidin-4-yl}-piperazine-1-carboxylic acid (4-isopropoxy-phenyl)-amide

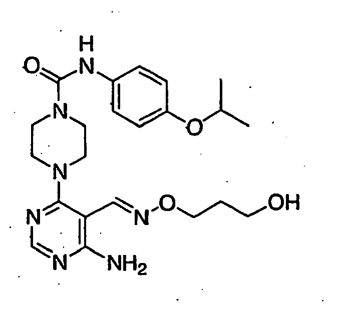

Prepared as described in Example 2e except that 3-aminooxy-propan-1-ol was used in place of O-(2-moipholin-4-yl-ethyl)-hydroxylamine. ¹H NMR (CD₃OD) δ 8.22 (s, 1H), 8.08 (s, 1H), 7.21 (d, *J* = 8.95 Hz, 2H), 6.83 (d, *J* = 9.01 Hz, 2H), 4.52 (m, 1H), 4.28 (t, *J* = 6.48 Hz, 2H), 3.69 (t, *J* = 6.35 Hz, 2H), 3.63 (m, 4H), 3.43 (m, 4H), 1.94 (m, 2H), 1.28 (d, *J* = 6.04 Hz, 6H). LC/MS (ESI) calcd for C₂₂H₃₂N₇O₄ (MH)⁺ 458.2, found 458.2.

### EXAMPLE 4

### 4-[6-Amino-5-(methoxyimino-methyl)-pyrimidin-4-yl]-piperazine-1-carboxylic acid (4-piperidin-1-yl-phenyl)-amide

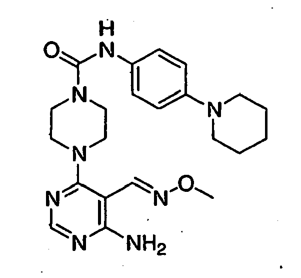

### a. (4-Piperidin-1-yl-phenyl)-carbamic acid 4-nitro-phenyl ester

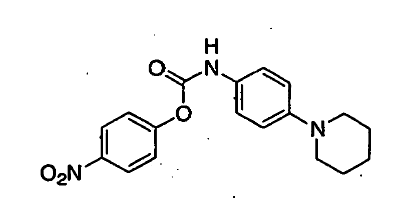

Prepared essentially as described in Example 1d, using 4-piperidinoaniline and toluene solvent. Silica flash chromatography (5:2 hex/EtOAc → EtOAc → 9:1 DCM/MeOH) provided the target compound as a grey powder (1.416 g, 73%). ¹H NMR (CDCl₃) δ 8.31-8.25 (m, 2H), 7.42-7.36 (m, 2H), 7.34-7.28 (m, 2H), 6.97-6.90 (m, 2H), 6.82 (br s, 1H), 3.17-3.09 (m, 4H), 1.77-1.66 (m, 4H), 1.63-1.54 (m, 2H). LC/MS (ESI) calcd for C₁₈H₁₉N₃O₄ (MH⁺) 342.1, found 342.2.

### b. 4-[6-Amino-5-(methoxyimino-methyl)-pyrimidin-4-yl]-piperazine-1-carboxylic acid (4-piperidin-1-yl-phenyl)-amide

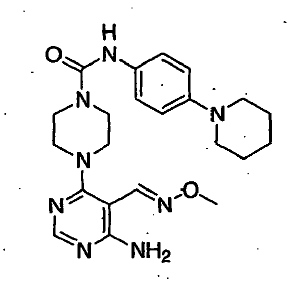

Prepared essentially as described in Example 1e except that (4-piperidin-1-yl-phenyl)-carbamic acid 4-nitro-phenyl ester was used in place of (4-isopropoxy-phenyl)-carbamic acid 4-nitro-phenyl ester. ¹H NMR (CDCl₃) δ 8.20 (s, 1H), 8.14 (s, 1H), 7.29 (m, 4H), 7.07 (br, 2H), 6.46 (br, 1H), 3.97 (s, 3H), 3.61 (m, 4H), 3.46 (m, 4H), 3.15 (m, 4H), 1.52-1.86 (m, 6H); LC/MS (ESI) calcd for C₂₀H₃₁N₈O₂ (MH)⁺ 439.3, found 439.2.

### EXAMPLE 5

### 4-[6-Amino-5-(methoxyimino-methyl)-pyrimidin-4-yl]-piperazine-1-carboxylic acid (4-morpholin-4-yl-phenyl)-amide

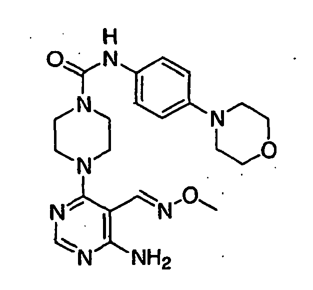

### a. (4-Morpholin-4-yl-phenyl)-carbamic acid 4-nitro-phenyl ester

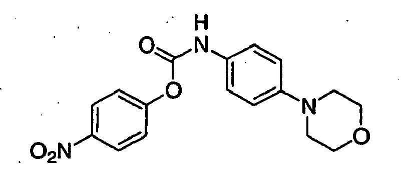

A mixture of 4-morpholinoaniline (1.01 g, 5.68 mmol) and CaCO₃ (743 mg, 7.42 mmol) (10 micron powder) was treated with a solution of 4-nitrophenyl chloroformate (1.49 g, 7.39 mmol) in CH₂Cl₂ (7.5 mL) under air on an ice bath. The thick, easily stirred reaction slurry was stirred for 1-2 min on the ice bath before stirring at room temperature for 1 h. The slurry was then diluted with 9:1 CH₂Cl₂/MeOH (7.5 mL) and directly applied to a flash silica column (95:5 CH₂Cl₂/MeOH) to provide 0.7 g of material. This was further purified by trituration with hot toluene (25 mL) to afford the title compound as a light olive green powder (444 mg, 23%). ¹H NMR (CDCl₃) δ 8.31-8.25 (m, 2H), 7.42-7.31 (m, 4H), 6.95-6.85 (m, 3H), 3.89-3.84 (m, 4H), 3.16-3.11 (m, 4H).

### b. 4-[6-Amino-5-(methoxyimino-methyl)-pyrimidin-4-yl]-piperazine-1-carboxylic acid (4-morpholin-4-yl-phenyl)-amide

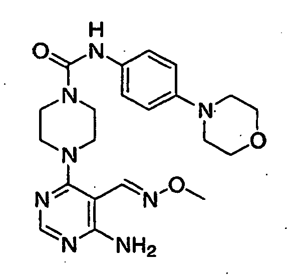

Prepared essentially as described in Example 1e except that (4-morpholin-4-yl-phenyl)-carbamic acid 4-nitro-phenyl ester was used in place of (4-isopropoxy-phenyl)-carbamic acid 4-nitro-phenyl ester. ¹H NMR (CDCl₃) δ 8.20 (s, 1H), 8.13 (s, 1H), 7.22 (m, 4H), 6.87 (br, 2H), 6.26 (br, 1H), 3.97 (s, 3H), 3.86 (t, *J* = 4.80 Hz, 4H), 3.60 (m, 4H), 3.47 (t, *J* = 4.47 Hz, 4H), 3.10 (m, 4H); LC/MS (ESI) calcd for C₂₁H₂₉N₈O₃(MH)⁺ 441.2, found 441.3.

### EXAMPLE 6

### 4-[6-Amino-5-(methoxyimino-methyl)-pyrimidin-4-yl]-piperazine-1-carboxylic acid (6-cyclobutoxy-pyridin-3-yl)-amide

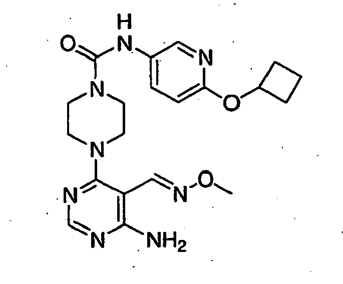

### a. 2-Cyclobutoxy-5-nitro-pyridine

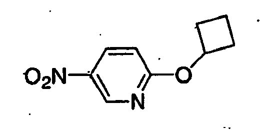

A mixture of 2-chloro-5-nitropyridine (7.12 g, 45.0 mmol) and cyclobutanol (3.40 g, 47.2 mmol) in THF (30 mL) was vigorously stirred at 0 °C while NaH (1.18 g, 46.7 mmol) was added in three portions over -10-20 s under air (Caution: Extensive gas evolution). Reaction residue was rinsed down with additional THF (5 mL), followed by stirring under positive argon pressure in the ice bath for 1-2 more minutes. The ice bath was then removed and the brown homogeneous solution was stirred for 1 h. The reaction mixture was concentrate under reduced pressure at 80 °C, taken up in 0.75 M EDTA (tetrasodium salt) (150 mL), and extracted with CH₂Cl₂ (1 × 100 mL, 1 × 50 mL). The combined organic layers were dried (Na₂SO₄), concentrated, taken up in MeOH (2 × 100 mL) and concentrated under reduced pressure at 60 °C to provide the title compound as a thick dark amber oil that crystallized upon standing (7.01 g, 80%). ¹H NMR (CDCl₃) δ 9.04 (dd, *J* = 2.84 and 0.40 Hz, 1H), 8.33 (dd, *J* = 9.11 and 2.85 Hz, 1H), 6.77 (dd, *J* = 9.11 and 0.50 Hz, 1H), 5.28 (m, 1H), 2.48 (m, 2H), 2.17 (m, 2H), 1.87 (m, 1H), 1.72 (m, 1H).

### b. 6-Cyclobutoxy-pyridin-3-ylamine

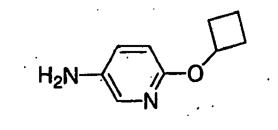

A flask containing 10% w/w Pd/C (485 mg) was gently flushed with argon while slowly adding MeOH (50 mL) along the sides of the flask, followed by the addition in -5 mL portions of a solution of 2-cyclobutoxy-5-nitro-pyridine (4.85 g, 25 mmol), as prepared in the previous step, in MeOH (30 mL). (Caution: Large scale addition of volatile organics to Pd/C in the presence of air can cause fire.) The flask was then evacuated one time and stirred under H₂ balloon pressure for 2 h at room temperature. The reaction was then filtered, and the clear amber filtrate was concentrated, taken up in toluene (2 × 50 mL) to remove residual MeOH, and concentrated under reduced pressure to provide the crude title compound as a translucent dark brown oil with a faint toluene smell (4.41 g). ¹H NMR (CDCl₃) δ 7.65 (d, *J* = 3.0 Hz, 1H), 7.04 (dd, *J* = 8.71 and 2.96 Hz, 1H), 6.55 (d, *J* = 8.74 Hz, 1H), 5.04 (m, 1H), 2.42 (m, 2H), 2.10 (m, 2H), 1.80 (m, 1H), 1.66 (m, 1H). LC-MS (ESI) calcd for C₉H₁₃N₂O (MH⁺) 165.1, found 165.2.

### c. (6-Cyclobutoxy-pyridin-3-yl)-carbamic acid 4-nitro-phenyl ester

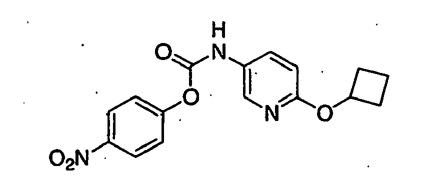

A. mixture of 6-cyclobutoxy-pyridin-3-ylamine (4.41 g, 25 mmol), as prepared in the previous step, and CaCO₃ (3.25 g, 32.5 mmol) (10 micron powder) was treated with a homogeneous solution of 4-nitrophenyl chloroformate (5.54 g, 27.5 mmol) in toluene (28 mL) in one portion at room temperature, and was stirred for 2 h. The reaction mixture was then directly loaded onto a flash silica column (95:5 DCM/MeOH → 9:1 DCM/MeOH) to afford 5.65 g of material, which was further purified by trituration with hot toluene (1 × 200 mL) to provide the title compound (4.45 g, 54%). ¹H NMR (CDCl₃) δ 8.32-8.25 (m, 2H), 8.12 (d, 1H), 7.81 (m, 1H), 7.42-7.36 (m, 2H), 6.85 (br s, 1H), 6.72 (d, 1H), 5.19-5.10 (m, 1H), 2.50-2.40 (m, 2H), 2.19-2.07 (m, 2H), 1.89-1.79 (m, 1H), 1.75-1.61 (m, 1H). LC-MS (ESI) calcd for C₁₆H₁₅N₃O₅ (MH⁺) 330.1, found 330.1.

### d. 4-[6-Amino-5-(methoxyimino-methyl)-pyrimidin-4-yl]-piperazine-1-carboxylic acid (6-cyclobutoxy-pyridin-3-yl)-amide

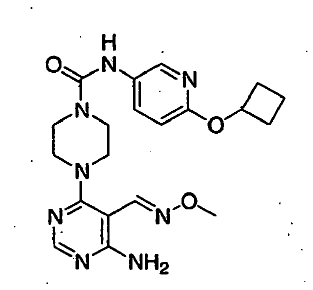

Prepared as described in Example 1e except that (6-cyclobutoxy-pyridin-3-yl)-carbamic acid 4-nitio-phenyl ester was used in place of (4-isopropoxy-phenyl)-carbamic acid 4-nitro-phenyl ester. ¹H NMR (DMSO-d₆) δ 8.55 (s, 1H), 8.14 (s, 1H), 8.12 (d, *J* = 2.74 Hz, 1H), 8.10 (s, 1H), 7.73 (dd, *J* = 8.72 and 2.72 Hz, 1H), 7.48 (br, 1H), 6.69 (d, *J* = 8.86 Hz, 1H), 5.05 (m, 1H), 3.91 (s, 3H), 3.54 (m, 4H), 3.34 (m, 4H), 2.36 (m, 2H), 2.00 (m, 2H), 1.75 (m, 1H), 1.61 (m, 1H); LC/MS (ESI) calcd for C₂₀H₂₇N₈O₃ (MH)⁺ 427.2, found 427.2.

### EXAMPLE 7

### 4-Amino-6-{4-[2-(4-isopropyl-phenyl)acetyl]-piperazin-1-yl}-pyrimidine-5-carbaldehyde O-methyl-oxim

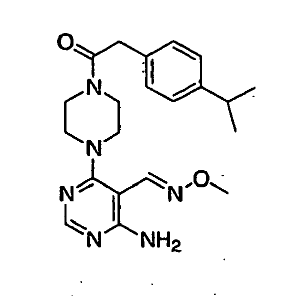

To a mixture of crude 4-amino-6-piperazin-1-yl-pyrimidine-5-carbaldehyde O-methyl-oxime trifluoroacetic acid salt (45.3 mg, 0.13 mmol), prepared as Example 1c, and (4-isopropyl-phenyl)-acetic acid (23 mg, 0.13 mmol) in anhydrous THF (2 mL) was added HOBT (25.7 mg, 0.17 mmol), followed by HBTU (63.6 mg, 0.17 mmol) and DIEA (83.4 mg, 0.65 mmol). The mixture was stirred at room temperature overnight and concentrated under reduced pressure. The crude material was directly loaded onto a preparative TLC plate for purification (5% MeOH/EtOAc) (8.6 mg, 16.7%). ¹H NMR (CDCl₃) δ 8.16 (s, 1H), 8.05 (s, 1H), 7.17 (m, 4H), 3.95 (s, 3H), 3.75 (m, 2H), 3.73 (s, 2H), 3.55 (t, *J* = 4.81 Hz, 2H), 3.38 (t, *J* = 4.98 Hz, 2H), 3.26 (t, J = 4.79 Hz, 2H), 2.89 (sep, *J* = 6.81 Hz, 1H), 1.24 (d, *J* = 6.92 Hz, 6H); LC/MS (ESI) calcd for C₂₁H₂₉N₆O₂ (MH)⁺ 397.2, found 397.3.

### EXAMPLE 8

### 4-[6-Amino-5-(methoxyimino-methyl)-pyrimidin-4-yl]-piperazine-1-carboxylic acid (4-isopropyl-phenyl)-amide

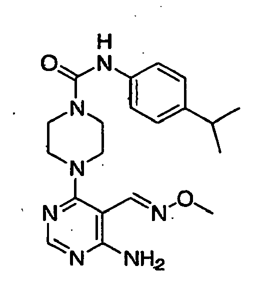

### a. (4-Isopropyl-phenyl)-carbamic acid 4-nitro-phenyl ester

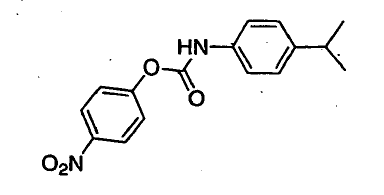

To a solution of 4-isopropylaniline (3.02 g, 22.3 mmol) in CH₂Cl₂ (40 mL) and pyridine (10 mL) was added 4-nitrophenyl chloroformate (4.09 g, 20.3 mmol) portionwise with stirring over -30 sec with brief ice-bath cooling. After stirring at room temperature for 1 h, the homogeneous solution was diluted with CH₂Cl₂ (100 mL) and washed with 0.6 M HCl (1 × 250 mL), 0.025 M HCl (1 × 400 mL), water (1 × 100 mL), and 1 M NaHCO₃ (1 ×100 mL). The organic layer was dried (Na₂SO₄) and concentrated to give the title compound as a light peach-colored solid (5.80 g, 95%). ¹H NMR (CDCl₃) δ 8.31-8.25 (m, 2H), 7.42-7.32 (m, 4H), 7.25-7.20 (m, 2H), 6.93 (br s, 1H), 2.90 (h, J = 6.9 Hz, 1H), 1.24 (d, J = 6.9 Hz, 6H). LC/MS (ESI) calcd for C₁₆H₁₆N₂O₄ (2MH)⁺ 601.2, found 601.3.

### b. Piperazine-1-carboxylic acid (4-isopropyl-phenyl)-amide

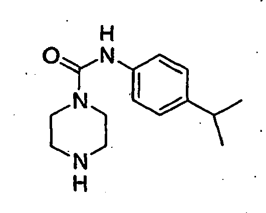

A mixture of piperazine-1-carboxylic acid *tert*-butyl ester (186 mg, 1.0 mmol) and (4-isopropyl-phenyl)-carbamic acid 4-nitro-phenyl ester (300 mg, 1.0 mmol) in CH₃CN (1.5 mL) was heated at reflux for 2 h and concentrated under reduced pressure. The residue was treated with 50% TFA/ CH₂Cl₂ (5 mL) and the solution was stirred overnight. The organic solvents were evaporated and the residue was neutralized with 2 M NH₃ in MeOH. After evaporation of the solvents, the residue was partitioned between EtOAc and water and the organic phase was dried and concentrated. The resulting material was purified by flash column chromatography on silica gel (EtOAc → 10% MeOH/EtOAc) to give the title compound (126 mg, 51%). ¹H NMR (CD₃OD) δ 7.25 (d, *J* = 8.53 Hz, 2H), 7.15 (d, *J* = 8.69 Hz, 2H), 3.75 (t, *J* = 5.17 Hz, 4H), 2.85 (sep, *J* = 6.91 Hz, 1H), 1.21 (d, *J* = 6.93 Hz, 6H); LC/MS (ESI) calcd for C₁₄H₂₂N₃O (MH)⁺ 248.2, found 248.2.

### c. 4-[6-Amino-5-(methoxyimino-methyl)-pyrimidin-4-yl]-piperazine-1-carboxylic acid (4-isopropyl-phenyl)-amide

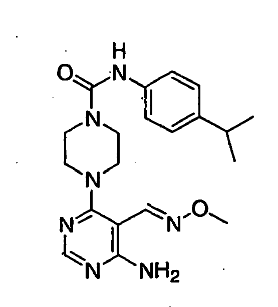

Following the procedure for the synthesis of 1h, using piperazine-1-carboxylic acid (4-isopropyl-phenyl)-amide instead of piperazine-1-carboxylic acid (4-isopropoxy-phenyl)-amide. ¹H NMR (CD₃OD) δ 8.20 (s, 1H), 8.08 (s, 1H), 7.25 (d, *J* = 8.63 Hz, 2H), 7.14 (d, *J* = 8.35 Hz, 2H), 3.96 (s, 3H), 3.64 (m, 4H), 3.42 (m, 4H), 2.85 (sep, *J* = 6.92 Hz, 1H), 1.22 (d, *J* = 6.93 Hz, 6H); LC/MS (ESI) calcd for C₂₀H₂₈N₇O₂ (MH)⁺ 398.2, found 398.3.

### EXAMPLE 9

### 4-[6-Amino-5-(ethoxyimino-methyl)-pyrimidin-4-yl]-piperazine-1-carboxylic acid (4-isopropoxy-phenyl) -amide (anti-configuration for -C=N-O-)

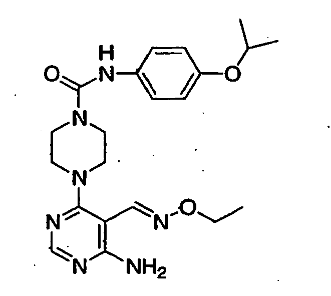

### a. 4-[6-Amino-5-(ethoxyimino-methyl)-pyrimidin-4-yl]-piperazine-1-carboxylic acid tert-butyl ester (anti-configuration for -C=N-O-)

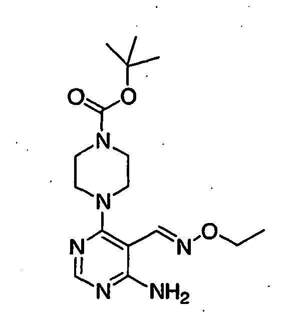

A mixture of 4-(6-amino-5-formyl-pyrimidin-4-yl)-piperazine-1-carboxylic acid *tert-*butyl ester (135.1 mg, 0.44 mmol) and EtONH₂.HCl (128.6 mg, 1.32 mmol) in MeOH (1.5 mL) was stirred at 90 °C for 0.5 h and the solvent was removed under reduced pressure. The residue was partitioned between CH₂Cl₂ and water and the organic phase was dried (Na₂SO₄). Evaporation of the solvent provided a white solid, which is shown to be a mixture of two isomers (2:1 ratio) by ¹H NMR (CDCl₃). Preparative TLC purification (EtOAc as eluent) provided two pure isomers. The major isomer is assigned to be the *anti*-isomer (for -C=N-O- configuration) (87.7 mg, 56.9%). ¹H NMR (CDCl₃) δ 8.13 (s, 1H), 8.04 (s, 1H), 4.21 (q, *J* = 7.06 Hz, 2H), 3.54 (m, 8H), 1.47 (s, 9H), 1.33 (t, *J* = 7.04 Hz, 3H); LC/MS (ESI) calcd for C₁₆H₂₇N₆O₃ (MH)⁺ 351.2, found 351.3.

### b. 4-[6-Amino-5-(ethoxyimino-methyl)-pyrimidin-4-yl]-piperazine-1-carboxylic acid tert-butyl ester (syn-configuration for -C=N-O-)

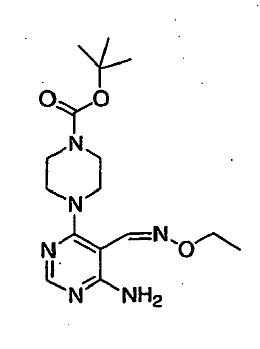

Prepared as described in Example 9a. It corresponds to the minor isomer and is assigned to be the *syn*-isomer (for -C=N-O- configuration) (40 mg, 26%). ¹H NMR (CDCl₃) δ 8.13 (s, 1H), 7.17 (s, 1H), 4.33 (q, *J* = 7.17 Hz, 2H), 3.65 (m, 4H), 3.53 (m, 4H), 1.48 (s, 9H), 1.35 (t, *J* = 7.04 Hz, 3H); LC/MS (ESI) calcd for C₁₆H₂₇N₆O₃ (MH)⁺ 351.2, found 351.3.

### c. 4-[6-Amino-5-(ethoxyimino-methyl)-pyrimidin-4-yl]-piperazine-1-carboxylic acid (4-isopropoxy-phenyl) -amide (anti-configuration for -C=N-O-)

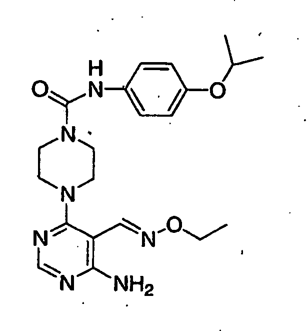

4-[6-Amino-5-(ethoxyimino-methyl)-pyrimidin-4-yl]-piperazine-1-carboxylic acid *tert*-butyl ester (*anti*-isomer) (36.8 mg, 0.105 mmol) was treated with 50% TFA/ CH₂Cl₂(1.3 mL) for 2 h and the solvents were removed under reduced pressure. The resulting material was re-dissolved in CH₃CN (2 mL), mixed with (4-isopropoxy-phenyl)-carbamic acid 4-nitro-phenyl ester (36.5 mg, 0.12 mmol) and DIEA (54.3 mg, 0.42 mmol). The reaction mixture was heated at 95 C for 1 h, concentrated and the residue was purified by flash column chromatography on silica gel (EtOAc → 5% MeOH/EtOAc) to afford the title compound as a white solid (14.4 mg, 32%). ¹H NMR (CDCl₃) δ 8.20 (s, 1H), 8.15 (s, 1H), 7.23 (d, *J* = 8.88 Hz, 2H), 6.84 (d, *J* = 8.92 Hz, 2H), 6.30 (br, 1H), 4.49 (sep, *J* = 6.08 Hz, 1H), 4.21 (q, *J* = 7.05 Hz, 2H), 3.61 (m, 4H), 3.45 (m, 4H), 1.34 (t, *J* = 7.18 Hz, 3H), 1.32 (d, *J* = 6.30 Hz, 6H); LC/MS (ESI) calcd for C₂₁H₃₀N₇O₃ (MH)⁺ 428.2, found 428.3.

### EXAMPLE 10

### 4-[6-Amino-5-(ethoxyimino-methyl)-pyrimidin-4-yl]-piperazine-1-carboxylic acid (4-isopropoxy-phenyl) -amide (syn-configuration for -C=N-O-)

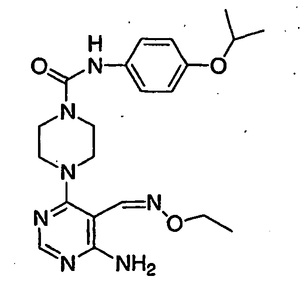

Prepared as described in Example 9c except that the *syn*-isomer of 4-[6-amino-5-(ethoxyimino-methyl)-pyrimidin-4-yl]-piperazine-1-carboxylic acid *tert*-butyl ester was used in place of its *anti*-isomer. ¹H NMR (CDCl₃) δ 8.24 (s, 1H), 7.27 (s, 1H), 7.22 (d, *J* = 8.97 Hz, 2H), 6.84 (d, *J* = 8.96 Hz, 2H), 6.22 (br, 1H), 5.60 (br, 2H), 4.48 (sep, *J* = 6.19 Hz, 1H), 4.33 (q, *J* = 7.06 Hz, 2H), 3.57 (m, 8H), 1.36 (t, *J* = 7.08 Hz, 3H), 1.31 (d, *J* = 6.05 Hz, 6H); LC/MS (ESI) calcd for C₂₁H₃₀N₇O₃ (MH)⁺ 428.2, found 428.3.

### EXAMPLE 11

### 4-[6-Amino-5-(ethoxyimino-methyl)-pyrimidin-4-yl]-piperazine-1-carboxylic acid (4-piperidin-1-yl-phenyl)-amide

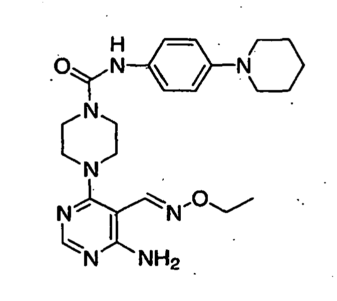

Prepared as described in Example 9c except that (4-piperidin-1-yl-phenyl)-carbamic acid 4-nitro-phenyl ester was used in place of (4-isopropoxy-phenyl)-carbamic acid 4-nitro-phenyl ester. ¹H NMR (CDCl₃) δ 8.20 (s, 1H), 8.15 (s, 1H), 7.27 (m, 4H), 7.04 (br, 2H), 6.43 (br, 1H), 4.21 (q, *J* = 7.07 Hz, 2H), 3.62 (m, 4H), 3.45 (t, *J* = 4.82 Hz, 4H), 3.13 (m, 4H), 1.54-1.84 (m, 6H), 1.34 (t, *J* = 7.06 Hz, 3H); LC/MS (ESI) calcd for C₂₃H₃₃N₈O₂ (MH)⁺ 453.3, found 453.3.

### EXAMPLE 12

### 4-[6-Amino-5-(ethoxyimino-methyl)-pyrimidin-4-yl]-piperazine-1-carboxylic acid (6-cyclobutoxy-pyridin-3-yl)-amide

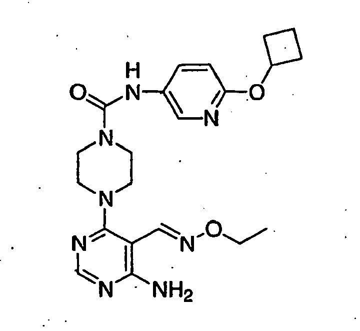

Prepared as described in Example 9c except that (6-cyclobutoxy-pyridin-3-yl)-carbamic acid 4-nitro-phenyl ester was used in place of (4-isopropoxy-phenyl)-carbamic acid 4-nitro-phenyl ester. ¹H NMR (CDCl₃) δ 8.20 (s, 1H), 8.15 (s, 1H), 7.96 (d, *J* = 2.65 Hz, 1H), 7.73 (dd, *J* = 8.84 and 2.74 Hz, 1H), 7.26 (br, 2H), 6.66 (d, *J* = 9.03 Hz, 1H), 6.27 (br, 1H), 5.10 (m, 1H), 4.21 (q, *J* = 7.05 Hz, 2H), 3.61 (m, 4H), 3.47 (m, 4H), 2.43 (m, 2H), 2.11 (m, 2H), 1.82 (m, 1H), 1.65 (m, 1H), 1.33 (t, *J* = 7.07 Hz, 3 H); LC/MS (ESI) calcd for C₂₁H₂₉N₈O₃ (MH)⁺ 441.2, found 441.3.

### EXAMPLE 13

### 4-Amino-6-{4-[2-(4-isopropyl-phenyl)-acetyl]-piperazin-1-yl}-pyrimidine-5-carbaldehyde O-ethyl-oxime (anti-configuration for -C=N-O-)

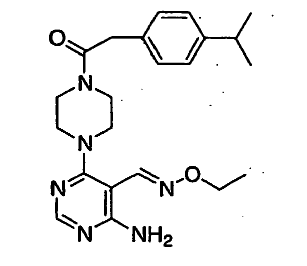

4-[6-Amino-5-(ethoxyimino-methyl)-pyrimidin-4-yl]-piperazine-1-carboxylic acid *tert*-butyl ester (a mixture of both *anti*- and *syn*-isomers, 37 mg, 0.11 mmol) was treated with 50% TFA/CH₂Cl₂ (1.5 mL) for 2 h and the organic solvents were removed under reduced pressure. The resulting material was used for the following coupling reaction without purification. To a mixture of the above material and (4-isopropyl-phenyl)-acetic acid (18.7 mg, 0.11 mmol) in THF (3 mL) was added HOBT (20.9 mg, 0.14 mmol), followed by HBTU (51.9 mg, 0.14 mmol) and DIEA (67.9 mg, 0.53 mmol). The reaction solution was stirred at room temperature overnight and concentrated. The residue was directly subjected to preparative TLC purification (5% MeOH/EtOAc) to give two products, which were shown to be a mixture of *anti*- and *syn*-isomers in terms of the -C=N-O- configuration). The major isomer is a white solid (5.3 mg, 12.3% isolated yield). ¹H NMR (CDCl₃) δ 8.16 (s, 1H), 8.07 (s, 1H), 7.18 (m, 4H), 4.20 (q, J = 7.08 Hz, 2H), 3.75 (m, 2H), 3.74 (s, 2H), 3.57 (t, J = 5.05 Hz, 2H), 3.37 (t, *J* = 5.08 Hz, 2H), 3.25 (t, *J* = 5.06 Hz, 2H), 2.89 (sep, *J* = 7.25 Hz, 1H), 1.32 (t, *J* = 7.05 Hz, 3H), 1.23 (d, *J* = 6.92 Hz, 6H); LC/MS (ESI) calcd for C₂₂H₃₁N₆O₂ (MH)⁺ 411.2, found 411.3.

### EXAMPLE 14

### 4-Amino-6-{4-[2-(4-isopropyl-phenyl)-acetyl]-piperazin-1-yl}-pyrimidine-5-carbaldehyde O-ethyl-oxime (syn-configuration for -C=N-O-)

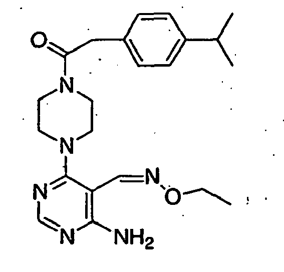

Prepared as described in Example 13. The minor isomer is a white solid (1.8 mg, 4.2% isolated yield). ¹H NMR (CDCl₃) δ 8.21 (s, 1H), 7.22 (s, 1H), 7.18 (m, 4H), 4.31 (q, *J* = 7.10 Hz, 2H), 3.74 (s, 2H), 3.73 (m, 2H), 3.54 (m, 2H), 3.39 (m, 2H), 3.30 (m, 2H), 2.89 (sep, *J* = 7.08 Hz, 1H), 1.34 (t, *J* = 7.07 Hz, 3H), 1.23 (d, *J* = 6.92 Hz, 6H); LC/MS (ESI) calcd for C₂₂H₃₁N₆O₂ (MH)⁺ 411.2, found 411.3.

### EXAMPLE 15

### 4-[6-Amino-5-(ethoxyimino-methyl)-pyrimidin-4-yl]-piperazine-1-carboxylic acid (4-morpholin-4-yl-phenyl)-amide

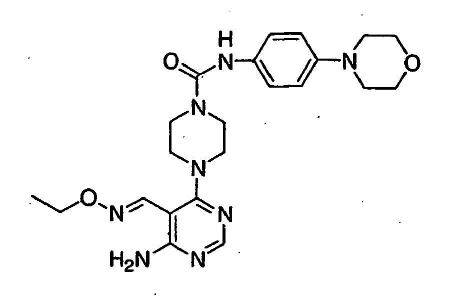

Prepared as described in Example 9c except that (4-morpholin-4-yl-phenyl)-carbamic acid 4-nitro-phenyl ester was used in place of (4-isopropoxy-phenyl)-carbamic acid 4-nitro-phenyl ester. ¹H NMR (300 MHz, CDCl₃) δ 8.16 (s, 1H), 8.10 (s, 1H), 7.20-7.27 (m, 4H), 6.85-6.91 (br, 2H), 6.23 (br, 1H), 4.22 (q, *J* = 7.08 Hz, 2H), 3.82-3.89 (m, 4H), 3.54-3.64 (m, 8H), 3.06-3.14 (m, 4H), 1.33 (t, *J* = 7.09 Hz, 3H). LC-MS (ESI) calcd for C₂₂H₃₁N₈O₃ (MH⁺) 455.2, found 455.2.

### EXAMPLE 16

### 4-{6-Amino-5-[(2-morpholin-4-yl-2-oxo-ethoxyimino)-methyl]-pyrimidin-4-yl}-piperazine-1-carboxylic acid (4-isopropoxy-phenyl)-amide

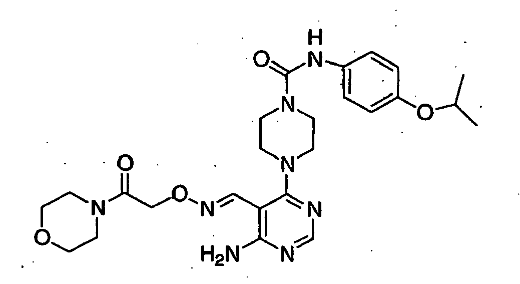

Prepared as described in Example 2c except that 2-aminooxy-1-morpholin-4-yl-ethanone hydrochloride was used in place of O-(2-morpholin-4-yl-ethyl)-hydroxylamine. ¹H NMR (300 MHz, DMSO-d₆) δ 8.45 (s, 1H), 8.24 (s, 1H), 8.23 (s, 1H), 7.82 (br, 2H), 7.31 (d, *J* = 8.95 Hz, 2H), 6.80 (d, *J* = 8.94 Hz, 2H), 4.91 (s, 2H), 4.50 (m, 1H), 3.55 (m, 4H), 3.32-3.46 (m, 8H), 3.31 (m, 4H), 1.22 (d, *J* = 6.03 Hz, 6H). LC-MS (ESI) calcd for C₂₁H₃₅N₈O₅ (MH⁺) 527.3, found 527.1.

### Example 17

### 4-[6-Amino-5-(methoxyimino-methyl)-pyrimidin-4-yl]-piperazine-1-carboxylic acid (6-cyclopentyloxy-pyridin-3-yl)-amide

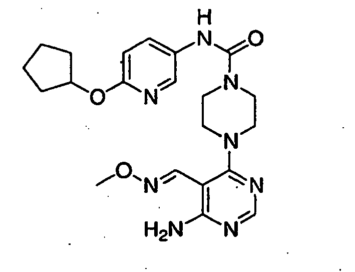

### a. 2-Cyclopentyloxy-5-nitro-pyridine

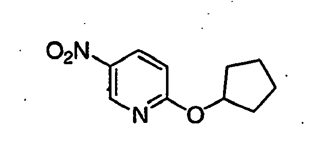

To a solution of 2-chloro-5-nitropyridine (7.01 g, 44.4 mmol) in THF (30 mL) and cyclopentanol (3.9 g, 45.3 mmol) was added sodium hydride (1.3 g, 54.2 mmol) portionwise with stirring over -30 sec with ice-bath cooling at 0 °C. After stirring at 0 °C for 5 min, the ice bath was removed and the reaction was stirred at rt for 3h. It was then concentrated in vacuo and the residue was dissolved in DCM and washed expensively with 1 M NaHCO₃ and then dried over anhydrous Na₂SO₄, filtered and concentrated in vacuo. The crude product was purified by flash column chromatography (silica gel, 9:1 Hexane:Ethyl Acetate) to obtain pure 2-cyclopentyloxy-5-nitro-pyridine (0.4 g, 4%). ¹H-NMR (300 MHz, CDCl₃): δ 9.07 (s, 1H), 8.32 (m, 1H), 6.74 (d, 1H), 5.53 (m, 1H), 2.00 (m, 2H), 1.81 (m, 4H), 1.66 (m, 2H).

### b. 6-Cyclopentyloxy-pyridin-3-ylamine

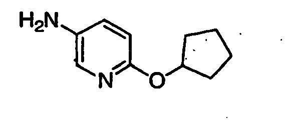

To a solution of 2-cyclopentyloxy-5-nitro-pyridine (0.3099 g, 1.49 mmol), in MeOH (2 mL) was added 10% Pd/C (90 mg). The solution was degassed and was kept stirring under hydrogen atmosphere for overnight. It was filtered through a pad of celite and the filtrate was evaporated to afford the desired product as a brown oil (248 mg, 94% yield). ¹H-NMR (300 MHz, CDCl₃): δ 7.69 (d, 1H), 7.04 (m, 1H), 6.56 (d, 1H), 5.25 (m, 1H), 1.93 (m, 2H), 1.78 (m, 4H), 1.60 (m, 2H). LC/MS (ESI) calcd for C₁₀H₁₄N₂O 178.23, found [M+41+1]⁺ 220.0.

### c. (6-Cyclopentyloxy-pyridin-3-yl)-carbamic acid 4-nitro-phenyl ester

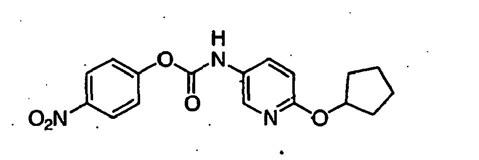

To a solution of 6-cyclopentyloxy-pyridin-3-ylamine (0.248 g, 1.39 mmol) in THF (2 mL) was added 4-nitrophenyl chloroformate (0.280 g, 1.39 mmol) portionwise. After stirring at rt for 1 h, a heavy precipitate formed in the organic layer. Filtration of the organic layer provided the title compound as a light pink solid (0.368 g, 77%). ¹H-NMR (400 MHz, CDCl₃): δ 11.1 (s, 1H), 9.11 (s, 1H), 9.04 (d, 1H), 8.26 (d, 2H), 7.40 (d, 2H), 7.14 (d, 1H), 5.36 (m, 1H), 2.11 (m, 2H), 1.97 (m, 2H), 1.84 (m, 2H), 1.71 (m, 2H).

### d. 4-[6-Amino-5-(methoxyimino-methyl)-pyrimidin-4-yl]-piperazine-1-carboxylic acid (6-cyclopentyloxy-pyridin-3-yl)-amide

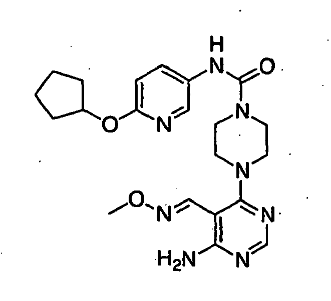

Prepared essentially as described in Example 6d except that (6-cyclopentyloxy-pyridin-3-yl)-carbamic acid 4-nitro-phenyl ester was used in place of (6-cyclocyclobutoxy-pyridin-3-yl)-carbamic acid 4-nitro-phenyl ester. ¹H NMR (CDCl₃) δ 8.20 (s, 1H), 8.13 (s, 1H), 7.97 (d, J = 2.74 Hz, 1H), 7.71 (dd, J = 8.87 and 2.82 Hz, 1H), 6.65 (d, *J* = 8.87 Hz, 1H), 6.31 (br, 1H), 5.30 (m, 1H), 3.96 (s, 3H), 3.61 (m, 4H), 3.45 (m, 4H), 1.93 (m, 2H), 1.78 (m, 4H), 1.60 (m, 2H); LC/MS (ESI) calcd for C₂₁H₂₉N₈O₃ (MH)⁺ 441.2, found 441.3.

### Example 18

### 4-[6-Amino-5-(methoxyimino-methyl)-pyrimidin-4-yl]-piperazine-1-carboxylic acid (4-pyrrolidin-1-yl-phenyl)-amide

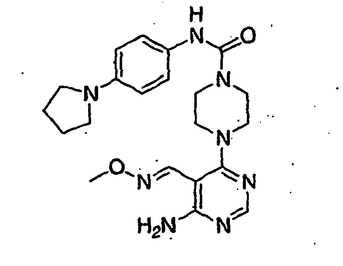

### a. (4-Pyrcolidin-1-yl-phenyl)-carbamic acid 4-nitro-phenyl ester hydrochloride

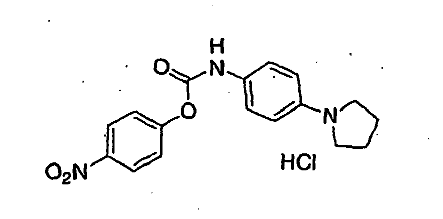

To a stirred solution of 4.9 g (30.4 mmol) of 4-pyrrolidin-1-yl-phenylamine in 70 mL of anhydrous THF at room temperature, was added dropwise a solution of 6.4 g (32 mmol) of 4-nitrophenyl chloroformate in 16 mL of anhydrous THF. After the addition was complete, the mixture was stirred for 1 h and then filtered. The precipitate was washed first with anhydrous THF (2 x 10 mL) and then with anhydrous DCM (3 x 10 mL) and dried *in vacuo* to yield 10 g of an off-white solid. ¹H-NMR (300 MHz, CD₃OD): 10.39 (s, 1H), 8.32 (d, 2H), 7.73 (d, 2H), 7.60 (d, 2H), 7.48 (d, 2H), 3.86-3.68 (bs, 4H), 2.35-2.24 (bs, 4H). LC/MS (ESI): 328 (MH)⁺.

### b. 4-[6-Amino-5-(methoxyimino-methyl)-pyrimidin-4-yl]-piperazine-1-carboxylic acid (4-pyrrolidin-1-yl-phenyl)-amide

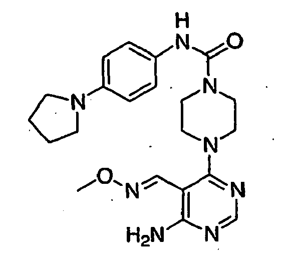

Prepared essentially as described in Example 1e except that (4-pyrrolidin-1-yl-phenyl)-carbamic acid 4-nitro-phenyl ester was used in place of (4-isopropoxy-phenyl)-carbamic acid 4-nitro-phenyl ester. ¹H NMR (CD₃OD) δ 8.20 (s, 1H), 8.08 (s, 1H), 7.11 (d, *J* = 8.77 Hz, 2H), 6.53 (d, *J* = 8.91 Hz, 2H), 3.96 (s, 3H), 3.61 (m, 4H), 3.42 (m, 4H), 3.24 (m, 4H), 2.01 (m, 4H); LC/MS (ESI) calcd for C₂₁H₂₉N₈O₂ (MH)⁺ 425.2, found 425.1.

### Example 19

### 4-[6-Amino-5-(methoxyimino-methyl)-pyrimidin-4-yl]-piperazine-1-carboxylic acid (4-cyclohexyl-phenyl)-amide

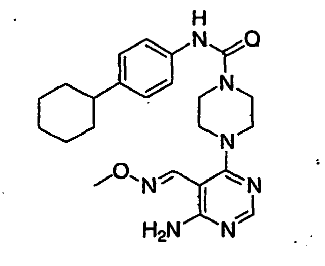

### a. (4-Cyclohexyl-phenyl)-carbamic acid 4-nitro-phenyl ester

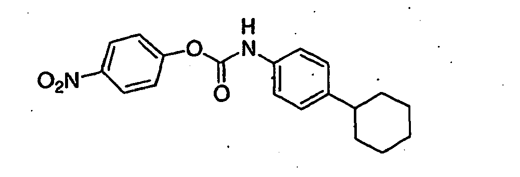

Prepared essentially as described as Example 8a except that 4-cyclohexylaniline was used in place of 4-isopropylaniline. ¹H NMR (DMSO-d₆) δ 10.37 (br, 1H), 8.30 (d, J = 9.30 Hz, 2H), 7.52 (d, *J* = 9.00 Hz, 2H), 7.41 (d, *J* = 8.10 Hz, 2H), 7.18 (d, *J* = 8.70 Hz, 2H), 1.18-1.82 (11H).

### b. 4-[6-Amino-5-(methoxyimino-methyl)-pyrimidin-4-yl]-piperazine-1-carboxylic acid (4-cyclohexyl-phenyl)-amide

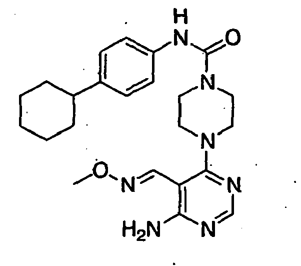

Prepared essentially as described in Example 1e except that (4-cyclohexyl-phenyl)-carbamic acid 4-nitro-phenyl ester was used in place of (4-isopropoxy-phenyl)-carbamic acid 4-nitro-phenyl ester. ¹H NMR (CDCl₃) δ 8.20 (s, 1H), 8.13 (s, 1H), 7.24 (d, *J* = 8.55 Hz, 2H), 7.13 (d, *J* = 8.50 Hz, 2H), 6.35 (br, 1H), 3.96 (s, 3H), 3.60 (m, 4H), 3.44 (m, 4H), 2.45 (m, 1H), 1.83 (m, 4H), 1.73 (m, 1H), 1.37 (m, 4H), 1.24 (m, 1H); LC/MS (ESI) calcd for C₂₃H₃₂N₇O₂ (MH)⁺ 438.3, found 438.3.

### Example 20

### 4-[6-Amino-5-(methoxyimino-methyl)-pyrimidin-4-yl]-piperazine-1-carboxylic acid (4-chloro-phenyl)-amide

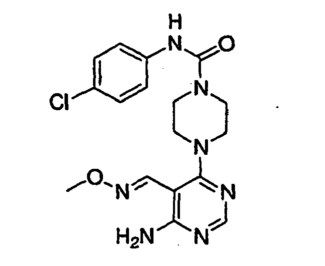

Prepared essentially as described in Example 1e except that 4-chlorophenyl isocyanate was used in place of (4-isopropoxy-phenyl)-carbamic acid 4-nitro-phenyl ester. ¹H NMR (CDCl₃) δ 8.20 (s, 1H), 8.13 (s, 1H), 7.30 (d, *J* = 9.00 Hz, 2H), 7.25 (d, *J* = 9.00 Hz, 2H), 6.42 (br, 1H), 3.96 (s, 3H), 3.61 (m, 4H), 3.46 (m, 4H); LC/MS (ESI) calcd for C₁₇H₂₁ClN₇O₂ (MH)⁺ 390.1, found 390.2.

### Example 21

### 4-[6-Amino-5-(methoxyimino-methyl)-pyrimidin-4-yl]-piperazine-1-carboxylic acid (4-phenoxy-phenyl)-amide

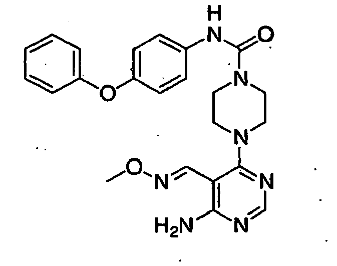

Prepared essentially as described in Example 1e except that 4-phenoxyphenyl isocyanate was used in place of (4-isopropoxy-phenyl)-carbamic acid 4-nitro-phenyl ester. ¹H NMR (CDCl₃) δ 8.20 (s, 1H), 8.14 (s, 1H), 7.31 (m, 4H), 7.07 (m, 1H), 6.97 (m, 4H), 6.35 (br, 1H), 3.97 (s, 3H), 3.62 (m, 4H), 3.47 (m, 4H); LC/MS (ESI) calcd for C₂₃H₂₆N₇O₃ (MH)⁺ 448.2, found 448.2.

### Example 22

### 4-[6-Amino-5-(methoxyimino-methyl)-pyrimidin-4-yl]-piperazine-1-carboxylic acid (4-dimethylamino-phenyl)-amide

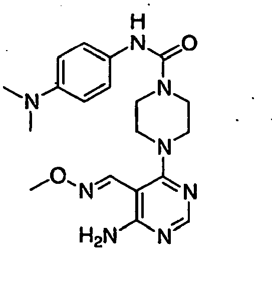

Prepared essentially as described in Example 1e except that *4*-*N,N-*dimethylaminophenyl isocyanate was used in place of (4-isopropoxy-phenyl)-carbamic acid 4-nitro-phenyl ester. ¹H NMR (CDCl₃) δ 8.21 (s, 1H), 8.14 (s; 1H), 7.18 (d, *J* = 9.04 Hz, 2H), 6.70 (d, *J* = 9.06 Hz, 2H), 6.16 (br, 1H), 3.97 (s, 3H), 3.59 (m, 4H), 3.45 (m, 4H), 2.91 (s, 6H); LC/MS (ESI) calcd for C₁₉H₂₇N₈O₂ (MH)⁺ 399.2, found 399.3.

### Example 23

### 4-[6-Amino-5-(methoxyimino-methyl)-pyrimidin-4-yl]-piperazine-1-carboxylic acid (4-isopropyl-phenyl)-amide

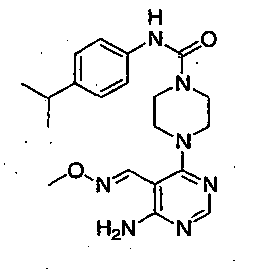

Prepared essentially as described in Example 1e except that 4-isopropylphenyl isocyanate was used in place of (4-isopropoxy-phenyl)-carbamic acid 4-nitro-phenyl ester. ¹H NMR (CDCl₃) δ 8.21 (s, 1H), 8.14 (s, 1H), 7.25 (d, *J* = 8.44 Hz, 2H), 7.16 (d, *J =* 8.38 Hz, 2H), 6.31 (br, 1H), 3.97 (s, 3H), 3.61 (m, 4H), 3.45 (m, 4H), 2.87 (m, 1H), 1.22 (d, *J* = 6.92 Hz, 6H); LC/MS (ESI) calcd for C₂₀H₂₈N₇O₂ (MH)⁺ 398.2, found 398.3.

### Example 24

### 4-[6-Amino-5-(methoxyimino-methyl)-pyrimidin-4-yl]-[1,4]diazepane-1-carboxylic acid (4-isopropoxy-phenyl)-amide

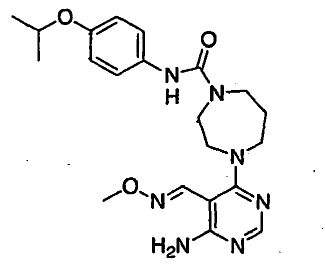

Prepared essentially as described in Example 1e except that 4-amino-6-[1,4]diazepan-1-yl-pyrimidine-5-carbaldehyde O-methyl-oxime was used in place of 4-amino-6-piperazin-1-yl-pyrimidine-5-carbaldehyde O-methyl-oxime. ¹H NMR (CDCl₃) δ 8.09 (2H), 7.20 (d, *J* = 8.99 Hz, 2H), 6.82 (d, *J* = 8.97 Hz, 2H), 6.29 (br, 1H), 4.47 (m, 1H), 3.95 (s, 3H), 3.79 (m, 2H), 3.75 (m, 2H), 3.68 (t, *J* = 5.57 Hz, 2H), 3.57 (t, *J* = 6.01 Hz, 2H), 2.06 (m, 2H), 1.30 (d, *J* = 6.06 Hz, 6H); LC/MS (ESI) calcd for C₂₁H₃₀N₇O₃ (MH)⁺ 428.2, found 428.3.

### Example 25

### 4-{6-Amino-5-[(2-amino-ethoxyimino)-methyl]-pyrimidin-4-yl}-piperazine-1-carboxylic acid (4-isopropoxy-phenyl)-amide

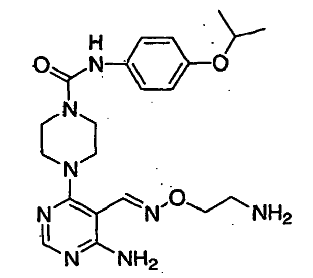

Prepared essentially as described in Example 2e except that O-(2-amino-ethyl)-hydroxylamine dihydrochloride was used in place of O-(2-morpholin-4-yl-ethyl)-hydroxylamine dihydrochloride. ¹H NMR (CDCl₃) δ 8.20 (2H), 7.22 (d, *J* = 8.96 Hz, 2H), 6.83 (d, *J* = 8.99 Hz, 2H), 6.32 (br, 1H), 4.48 (m, 1H), 4.19 (t, *J* = 5.18 Hz, 2H), 3.60 (m, 4H), 3.45 (m, 4H), 3.04 (t, *J* = 5.17 Hz, 2H), 1.31 (d, *J* = 6.06 Hz, 6H); LC/MS (ESI) calcd for C₂₁H₃₁N₈O₃ (MH)⁺ 443.2, found 443.3.

### Example 26

### 4-(6-Amino-5-{[2-(3-ethyl-ureido)-ethoxyimino]-methyl}-pyrimidin-4-yl)-piperazine-1-carboxylic acid (4-isopropoxy-phenyl)-amide

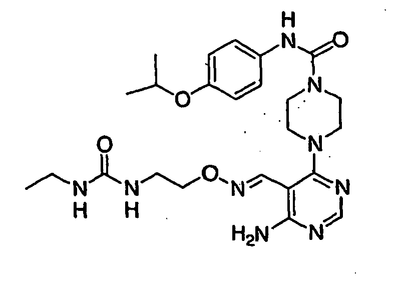

To a solution of 4-{6-amino-5-[(2-amino-ethoxyimino)-methyl]-pyrimidin-4-yl}-piperazine-1-carboxylic acid (4-isopropoxy-phenyl)-amide (44.7 mg, 0.101 mmol) in CH₃CN (1.5 mL) was added ethyl isocyanate (10.8 mg, 0.152 mmol). The mixture was stirred for 1 h and the solvents were evaporated. The residue was washed with water and MeOH, dried *in vacuo* to afford the desired product as a white solid. ¹H NMR (DMSO-d₆) δ 8.40 (br, 1H), 8.14 (s, 1H), 8.08 (s, 1H), 7.45 (br, 2H), 7.28 (d, *J* = 9.03 Hz, 2H), 6.77 (d, *J* = 9.08 Hz, 2H), 5.92 (t, *J* = 5.99 Hz, 1H), 5.85 (t, *J* = 5.02 Hz, 1H), 4.48 (m, 1H), 4.07 (t, *J* = 5.53 Hz, 2H), 3.22-3.54 (10H), 2.97 (m, 2H), 1.20 (d, *J* = 6.02 Hz, 6H), 0.94 (t, *J* = 7.14 Hz, 3H); LC/MS (ESI) calcd for C₂₄H₃₆N₉O₄ (MH)⁺ 514.3, found 514.3.

### Example 27

### 4-{6-Amino-5-[(2-methanesulfonylamino-ethoxyimino)-methyl]-pyrimidin-4-yl}-piperazine-1-carboxylic acid (4-isopropoxy-phenyl)-amide

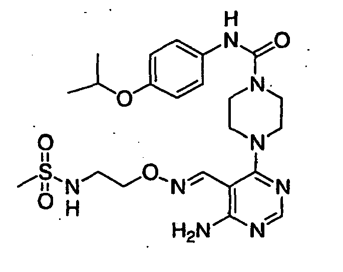

To a solution of 4-{6-amino-5-[(2-amino-ethoxyimino)-methyl]-pyrimidin-4-yl}-piperazine-1-carboxylic acid (4-isopropoxy-phenyl)-amide (70.8 mg, 0.16 mmol) in CH₂Cl₂ (2 mL) was added MsCl (45.8 mg, 0.4 mmol) and DIEA ((77.6 mg, 0.6 mmol). The reaction was stirred for 1 h, partitioned between CH₂Cl₂ and water. The CH₂Cl₂ extracts were evaporated and the crude residue was purified by flash column chromatography on silica gel (5% MeOH/EtOAc as eluent) to afford the desired product. ¹H NMR (CDCl₃) δ 8.20 (s, 1H), 8.16 (s, 1H), 7.24 (d, *J =* 8.92 Hz, 2H), 6.83 (d, *J* = 8.99 Hz, 2H), 6.45 (br, 1H), 5.23 (m, 1H), 4.47 (m, 1H), 4.29 (t, *J* = 5.36 Hz, 2H), 3.60 (m, 4H), 3.47 (m, 4H), 3.32 (m, 2H), 3.00 (s, 3H), 1.30 (d, *J* = 6.05 Hz, 6H); LC/MS (ESI) calcd for C₂₂H₃₃N₈O₄S (MH)⁺ 521.2, found 521.3.

### Example 28

### 4-{6-Amino-5-[(2-morpholin-4-yl-2-oxo-ethoxyimino)-methyl]-pyrimidin-4-yl}-piperazine-1-carboxylic acid (4-pyrrolidin-1-yl-phenyl)-amide

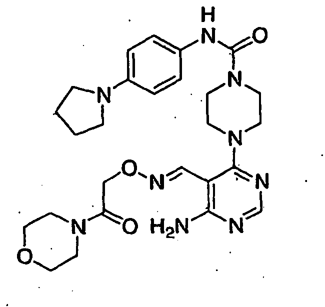

Prepared essentially as described in Example 2e except that 2-aminooxy-1-morpholin-4-yl-ethanone hydrochloride was used in place of O-(2-morpholin-4-yl-ethyl)-hydroxylamine dihydrochloride. ¹H NMR (DMSO-d₆) δ 8.26 (br, 1H), 8.20 (s, 1H), 8.14 (s, 1H), 7.60 (br, 2H), 7.19 (d, *J* = 8.97 Hz, 2H), 6.48 (d, *J* = 9.59 Hz, 2H), 4.88 (s, 2H), 3.54 (m, 8H), 3.30-3.47 (8H), 3.16 (m, 4H), 1.92 (m, 4H); LC/MS (ESI) calcd for C₂₆H₃₆N₉O₄ (MH)⁺ 538.3, found 538.3.

### Example 29

### 4-{6-Amino-5-[(2-morpholin-4-yl-ethoxyimino)-methyl]-pyrimidin-4-yl}-piperazine-1-carboxylic acid (4-morpholin-4-yl-phenyl)-amide

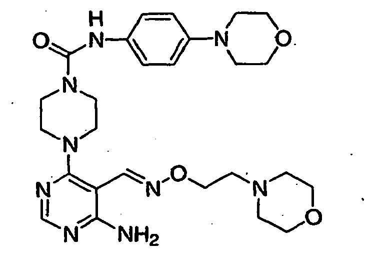

Prepared essentially as described in Example 5b except that O-(2-morpholin-4-yl-ethyl)-hydroxylamine dihydrochloride was used in place of methoxyamine hydrochloride. ¹H NMR (CDCl₃) δ 8.21 (s, 1H), 8.18 (s, 1H), 7.25 (d, *J* = 9.07 Hz, 2H), 6.88 (d, *J* = 9.07 Hz, 2H), 6.22 (br, 1H), 4.30 (t, *J* = 5.84 Hz, 2H), 3.86 (t, *J* = 4.66 Hz, 4H), 3.74 (t, *J* = 4.60 Hz, 4H), 3.60 (m, 4H), LC/MS (ESI) calcd for C₂₆H₃₈N₉O₄ (MH)⁺ 540.3, found 540.3.

### Example 30

### 4-{6-Amino-5-[(2-morpholin-4-yl-ethoxyimino)-methyl]-pyrimidin-4-yl}-piperazine-1-carboxylic acid (6-cyclobutoxy-pyridin-3-yl)-amide

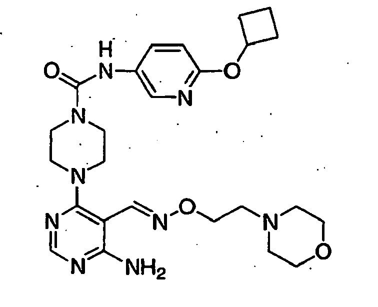

Prepared essentially as described in Example 6d except that O-(2-morpholin-4-yl-ethyl)-hydroxylamine dihydrochloride was used in place of methoxyamine hydrochloride. ¹H NMR (CDCl₃) δ 8.21 (s, 1H), 8.18 (s, 1H), 7.96 (d, *J* = 2.68 Hz, 1H), 7.74 (dd, *J* = 8.83 and 2.79 Hz, 1H), 6.67 (d, *J* = 9.16 Hz, 1H), 6.24 (br, 1H), 5.11 (m, 1H), 4.30 (t, *J* = 5.64 Hz, 2H); 3.74 (m, 4H), 3.61 (m, 4H), 3.45 (m, 4H), 2.73 (t, *J* = 5.71 Hz, 2H), 2.54 (m, 4H), 2.44 (m, 2H), 2.12 (m, 2H), 1.59-1.82 (2H); LC/MS (ESI) calcd for C₂₅H₃₆N₉O₄ (MH)⁺ 526.3, found 526.2.

### Example 31

### 4-{6-Amino-5-[(2-amino-ethoxyimino)-methyl]-pyrimidin-4-yl}-piperazine-1-carboxylic acid (6-cyclobutoxy-pyridin-3-yl)-amide

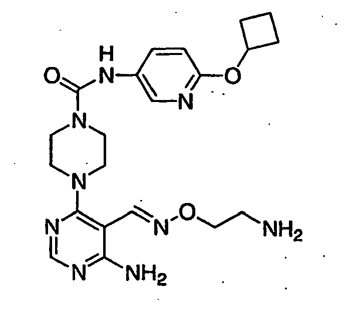

Prepared essentially as described in Example 6d except that O-(2-amino-ethyl)-hydroxylamine dihydrochloride was used in place of methoxyamine hydrochloride. ¹H NMR (CDCl₃) δ 8.21 (s, 1H), 8.20 (s, 1H), 7.96 (d, *J* = 2.26 Hz, 1H), 7.74 (dd, *J* = 8.83 and 2.78 Hz, 1H), 6.67 (d, *J* = 8.86 Hz, 1H), 6.31 (br, 1H), 5.10 (m, 1H), 4.20 (t, *J* = 5.22 Hz, 2H), 3.61 (m, 4H), 3.45 (m, 4H), 3.04 (m, 2H), 2.42 (m, 2H), 2.11 (m, 2H), 1.59-1.87 (2H); LC/MS (ESI) calcd for C₂₁H₃₀N₉O₃ (MH)⁺ 456.2, found 456.2.

### Example 32

### 4-{6-Amino-5-[(2-amino-ethoxyimino)-methyl]-pyrimidin-4-yl}-piperazine-1-carboxylic acid (4-morpholin-4-yl-phenyl)-amide

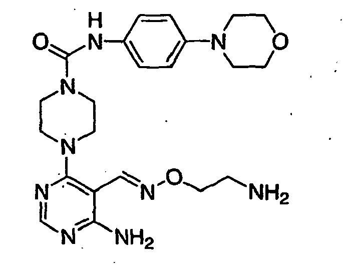

Prepared essentially as described in Example 5b except that O-(2-amino-ethyl)-hydroxylamine dihydrochloride was used in place of methoxyamine hydrochloride. ¹H NMR (CDCl₃) δ 8.21 (s, 1H), 8.20 (s, 1H), 7.25 (d, *J* = 9.05 Hz, 2H), 6.87 (d, *J* = 9.05 Hz, 2H), 6.23 (br, 1H), 4.20 (t, *J* = 5.25 Hz, 2H), 3.86 (t, *J* = 4.69 Hz, 4H), 3.62 (m, 4H), 3.46 (m, 4H), 3.11 (t, *J* = 4.86 Hz, 4H), 3.04 (t, *J* = 5.62 Hz, 2H); LC/MS (ESI) calcd for C₂₂H₃₂N₉O₃ (MH)⁺ 470.3, found 470.2.

### Example 33

### 4-{6-Amino-5-[(2-methanesulfonylamino-ethoxyimino)-methyl]-pyrimidin-4-yl}-piperazine-1-carboxylic acid (6-cyclobutoxy-pyridin-3-yl)-amide

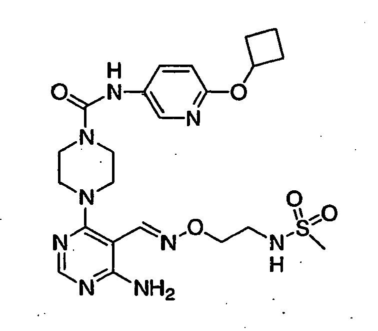

Prepared essentially as described in Example 27 except that 4-(6-amino-5-formyl-pyrimidin-4-yl)-piperazine-1-carboxylic acid (6-cyclobutoxy-pyridin-3-yl)-amide was used in place of 4-(6-amino-5-formyl-pyrimidin-4-yl)-piperazine-1-carboxylic acid (4-isopropoxy-phenyl)-amide. ¹H NMR (CDCl₃) δ 8.20 (s, 1H), 8.16 (s, 1H), 7.99 (d, *J* = 3.19 Hz, 1H), 7.74 (dd, *J* = 8.82 and 2.78 Hz, 1H), 6.65 (d, *J* = 8.83 Hz, 1H), 6.57 (s, 1H), 5.28 (br, 1H), 5.08 (m, 1H), 4.30 (t, *J* = 4.68 Hz, 2H), 3.61 (m, 4H), 3.45 (m, 6H), 3.00 (s, 3H), 2.42 (m, 2H), 2.11 (m, 2H), 1.59-1.87 (2H); LC/MS (ESI) calcd for C₂₂H₃₂N₉O₅S (MH)⁺ 534.2, found 534.2.

### Example 34

### 4-{6-Amino-5-[(2-amino-ethoxyimino)-methyl]-pyrimidin-4-yl}-piperazine-1-carboxylic acid (4-pyrrolidin-1-yl-phenyl)-amide

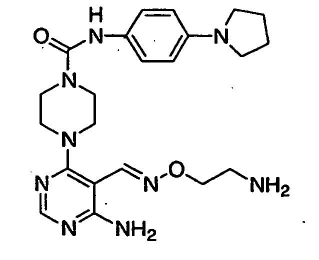

Prepared essentially as described in Example 2e except that O-(2-amino-ethyl)-hydroxylamine dihydrochloride was used in place of O-(2-morpholin-4-yl-ethyl)-hydroxylamine dihydrochloride. ¹H NMR (CDCl₃) δ 8.21 (s, 1H), 8.20 (s, 1H), 7.16 (d, *J* = 8.85 Hz, 2H), 6.51 (d, *J* = 8.89 Hz, 2H), 4.19 (t, J = 5.08 Hz, 2H), 3.58 (m, 4H), 3.45 (m, 4H), 3.26 (m, 4H), 3.04 (t, *J* = 5.30 Hz, 2H), 1.99 (m, 4H); LC/MS (ESI) calcd for C₂₂H₃₂N₉O₂ (MH)⁺ 454.3, found 454.2.

### Example 35

### 4-{6-Amino-5-[(2-methanesulfonylamino-ethoxyimino)-methyl]-pyrimidin-4-yl}-piperazine-1-carboxylic acid (4-pyrrolidin-1-yl-phenyl)-amide

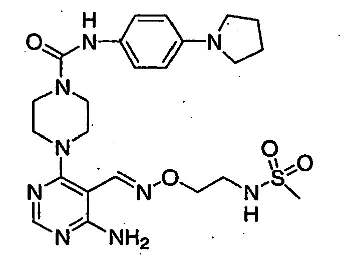

Prepared essentially as described in Example 27 except that 4-(6-amino-5-formyl-pyrimidin-4-yl)-piperazine-1-carboxylic acid (4-pyrrolidin-1-yl-phenyl)-amide was used in place of 4-(6-amino-5-formyl-pyrimidin-4-yl)-piperazine-1-carboxylic acid (4-isopropoxy-phenyl)-amide. ¹H NMR (CD₃OD) δ 8.26 (s, 1H), 8.08 (s, 1H), 7.11 (d, *J* = 8.94 Hz, 2H), 6.53 (d, *J* = 9.00 Hz, 2H), 4.26 (t, *J* = 5.22 Hz, 2H), 3.62 (m, 4H), 3.50 (m, 2H), 3.44 (m, 4H), 3.24 (m, 4H), 2.97 (s, 3H), 2.00 (m, 4H); LC/MS (ESI) calcd for C₂₃H₃₄N₉O₄S (MH)⁺ 532.2, found 532.1.

### Example 36

### 4-{6-Amino-5-[(2-morpholin-4-yl-ethoxyimino)-methyl]-pyrimidin-4-yl}-piperazine-1-carboxylic acid (4-pyrrolidin-1-yl-phenyl)-amide

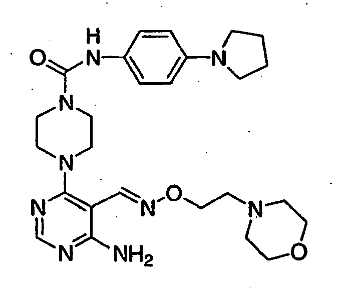

Prepared essentially as described in Example 2e except that 4-(6-amino-5-formyl-pyrimidin-4-yl)-piperazine-1-carboxylic acid (4-pyrrolidin-1-yl-phenyl)-amide was used in place of 4-(6-amino-5-formyl-pyrimidin-4-yl)-piperazine-1-carboxylic acid (4-isopropoxy-phenyl)-amide. ¹H NMR (CD₃OD) δ 8.24 (s, 1H), 8.08 (s, 1H), 7.11 (d, *J* = 8.96 Hz, 2H), 6.53 (d, *J* = 8.97 Hz, 2H), 4.34 (t, *J* = 5.53 Hz, 2H), 3.71 (t, *J* = 4.86 Hz, 4H), 3.62 (m, 4H), 3.43 (m, 4H), 3.24 (m, 4H), 2.75 (t, *J* = 5.70 Hz, 2H), 2.57 (m, 4H), 2.01 (m, 4H); LC/MS (ESI) calcd for C₂₆H₃₈N₉O₃ (MH)⁺ 524.3, found 524.3.

### Example 37

### 4-{6-Amino-5-[(2-morpholin-4-yl-ethoxyimino)-methyl]-pyrimidin-4-yl}-piperazine-1-carboxylic acid (4-isopropyl-phenyl)-amide

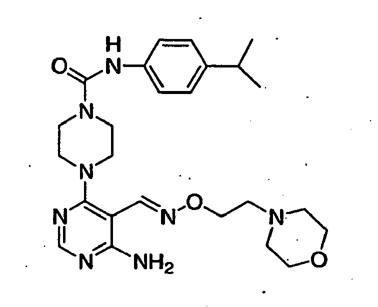

Prepared essentially as described in Example 2e except that 4-(6-amino-5-formyl-pyrimidin-4-yl)-piperazine-1-carboxylic acid (4-isopropyl-phenyl)-amide was used in place of 4-(6-amino-5-formyl-pyrimidin-4-yl)-piperazine-1-carboxylic acid (4-isopropoxy-phenyl)-amide. ¹H NMR (CD₃OD) δ 8.25 (s, 1H), 8.09 (s, 1H), 7.26 (d, J = 8.57 Hz, 2H), 7.14 (d, *J* = 8.43 Hz, 2H), 4.37 (t, *J* = 6.36 Hz, 2H), 3.74 (t, *J* = 4.75 Hz, 4H), 3.65 (m, 4H), 3.44 (m, 4H), 2.84 (m, 3H), 2.66 (m, 4H), 1.22 (d, *J* = 6.92 Hz, 6H); LC/MS (ESI) calcd for C₂₅H₃₇N₈O₃ (MH)⁺ 497.2, found 497.3.

### BIOLOGICAL ACTIVITY

### In Vitro Assays

The following representative *in vitro* assays were performed in determining.the biological activities of compounds within the scope of the invention. They are given to illustrate the invention in a non-limiting fashion.

Inhibition of FLT3 enzyme activity, MV4-11 proliferation and Baf3-FLT3 phosphorylation exemplify the specific inhibition of the FLT3 enzyme and cellular processes that are dependent on FLT3 activity. Inhibition of Baf3 cell proliferation is used as a test of FLT3, c-Kit and TrkB independent cytotoxicity of compounds within the scope of the invention. All of the examples herein show significant and specific inhibition of the FLT3 kinase and FLT3-dependent cellular responses. Examples herein also show specific inhibition of the TrkB and c-kit kinase in an enzyme activity assay. The compounds of the present invention are also cell permeable.

### FLT3 Fluorescence Polarization Kinase Assay

To determine the activity of the compounds of the present invention in an *in vitro* kinase assay, inhibition of the isolated kinase domain of the human FLT3 receptor (a.a. 571-993) was performed using the following fluorescence polarization (FP) protocol. The FLT3 FP assay utilizes the fluorescein-labeled phosphopeptide and the anti-phosphotyrosine antibody included in the Panvera Phospho-Tyrosine Kinase Kit (Green) supplied by Invitrogen. When FLT3 phosphorylates polyGlu₄Tyr, the fluorescein-labeled phosphopeptide is displaced from the anti-phosphotyrosine antibody by the phosphorylated poly Glu₄Tyr, thus decreasing the FP value. The FLT3 kinase reaction is incubated at room temperature for 30 minutes under the following conditions: 10nM FLT3 571-993, 20ug/mL poly Glu₄Tyr, 150uM ATP, 5mM MgCl₂, 1% compound in DMSO. The kinase reaction is stopped with the addition of EDTA. The fluorescein-labeled phosphopeptide and the anti-phosphotyrosine antibody are added and incubated for 30 minutes at room temperature.

All data points are an average of triplicate samples. Inhibition and IC₅₀ data analysis was done with GraphPad Prism using a non-linear regression fit with a multiparamater, sigmoidal dose-response (variable slope) equation. The IC₅₀ for kinase inhibition represents the dose of a compound that results in a 50% inhibition of kinase activity compared to DMSO vehicle control.

### c-Kit Fluorescence Polarization Kinase Assay

The compounds of the present invention are also specific inhibitors of c-Kit. Selection of preferred compounds of Formula I for use as c-Kit inhibitors was performed in the following manner using an *in vitro* kinase assay to measure inhibition of the isolated kinase domain of the human c-kit receptor in a fluorescence polarization (FP) protocol. The c-kit assay utilized the fluorescein-labeled phosphopeptide and the anti-phosphotyrosine antibody included in the Panvera Phospho-Tyrosine Kinase Kit (Green) supplied by Invitrogen. When c-kit phosphorylated the poly Glu₄Tyr, the fluorescein-labeled phosphopeptide was displaced from the anti-phosphotyrosine antibody by the phosphorylated poly Glu₄Tyr, thus decreasing the FP value. The c-kit kinase reaction was incubated at room temperature for 45 minutes under the following conditions: 1nM c-kit (ProQinase, lot SP005), 100ug/mL poly Glu₄Tyr, 50uM ATP, 5mM MgCl₂, 1mM DTT, 0.01%Tween-20, 1% DMSO or compound in 100nM Hepes, pH 7.5. The kinase reaction was stopped with the addition of EDTA. The fluorescein-labeled phosphopeptide and the anti-phosphotyrosine antibody were added and incubated for 30 minutes at room temperature and fluorescence polarization was read. Data points were an average of triplicate samples. Inhibition and IC₅₀ data analysis were done with GraphPad Prism using a non-linear regression fit with a multiparamater, sigmoidal dose-response (variable slope) equation. The IC₅₀ for kinase inhibition represents the dose of a compound that resulted in a 50% inhibition of kinase activity compared to DMSO vehicle control.

### Trk B Fluorescence Polarization Kinase Assay (TrkB IC₅₀ Data)

The compounds of the present invention are also specific inhibitors of TrkB. Selection of preferred compounds of Formula I for use as TrkB inhibitors was performed in the following manner. The TrkB assay utilized the fluorescein-labeled phosphopeptide and the anti-phosphotyrosine antibody included in the Panvera Phospho-Tyrosine Kinase Kit (Green) supplied by Invitrogen. When TrkB phosphorylated poly Glu₄Tyr, the fluorescein-labeled phosphopeptide was displaced from the anti-phosphotyrosine antibody by the phosphorylated poly Glu₄Tyr, thus decreasing the FP value. The TrkB kinase reaction was incubated at room temperature for 30 minutes under the following conditions: 50nM TrkB (Upstate, catalog # 14-507M), 20ug/mL poly Glu₄Tyr, 150uM ATP, 5mM MgCl₂, 1% compound in DMSO. The kinase reaction was stopped with the addition of EDTA. The fluorescein-labeled phosphopeptide and the anti-phosphotyrosine antibody were added and incubated for 30 minutes at room temperature. Data points were an average of triplicate samples. Inhibition and IC₅₀ data analysis were done with GraphPad Prism using a non-linear regression fit with a multiparamater, sigmoidal dose-response (variable slope) equation. The IC₅₀ for kinase inhibition represents the dose of a compound that resulted in a 50% inhibition of kinase activity compared to DMSO vehicle control.

### Inhibition Of MV4-11 and Baf3 Cell Proliferation

To assess the cellular potency of the compounds of the present invention, FLT3 specific growth inhibition was measured in the leukemic cell line MV4-11 (ATCC Number: CRL-9591). MV4-11 cells are derived from a patient with childhood acute myelomonocytic leukemia with an 11q23 translocation resulting in a MLL gene rearrangement and containing an FLT3-ITD mutation (AML subtype M4)(1,2). MV4-11 cells cannot grow and survive without active FLT3ITD.

The IL-3 dependent, murine b-cell lymphoma cell line, Baf3, were used as a control to confirm the selectivity of the compounds of the present invention by measuring non-specific growth inhibition by the compounds of the present invention.

To measure proliferation inhibition by test compounds, the luciferase based CellTiterGlo reagent (Promega), which quantifies total cell number based on total cellular ATP concentration, was used. Cells are plated at 10,000 cells per well in 100ul of in RPMI media containing penn/strep, 10% FBS and 1ng/ml GM-CSF or 1ng/ml IL-3 for MV4-11 and Baf3 cells respectively.

Compound dilutions or 0.1% DMSO (vehicle control) are added to cells and the cells are allowed to grow for 72 hours at standard cell growth conditions (37 °C, 5%CO₂). For activity measurements in MV4-11 cells grown in 50% plasma, cells were plated at 10,000 cells per well in a 1:1 mixture of growth media and human plasma (final volume of 100 µL). To measure total cell growth an equal volume of CellTiterGlo reagent was added to each well, according to the manufacturer's instructions, and luminescence was quantified. Total cell growth was quantified as the difference in luminescent counts (relative light units, RLU) of cell number at Day 0 compared to total cell number at Day 3 (72 hours of growth and/or compound treatment). One hundred percent inhibition of growth is defined as an RLU equivalent to the Day 0 reading. Zero percent inhibition was defined as the RLU signal for the DMSO vehicle control at Day 3 of growth. All data points are an average of triplicate samples. The IC₅₀ for growth inhibition represents the dose of a compound that results in a 50% inhibition of total cell growth at day 3 of the DMSO vehicle control. Inhibition and IC₅₀ data analysis was done with GraphPad Prism using a non-linear regression fit with a multiparamater, sigmoidal dose-response (variable slope) equation.

MV4-11 cells express the FLT3 internal tandem duplication mutation, and thus are entirely dependent upon FLT3 activity for growth. Strong activity against the MV4-11 cells is anticipated to be a desirable quality of the invention. In contrast, the Baf3 cell proliferation is driven by the cytokine IL-3 and thus are used as a non-specific toxicity control for test compounds. All compound examples in the present invention showed < 50% inhibition at a 3uM dose (data is not included), suggesting that the compounds are not cytotoxic and have good selectivity for FLT3.

### Cell-Based FLT3 Receptor Elisa

Specific cellular inhibition of FLT ligand-induced wild-type FLT3 phosphorylation was measured in the following manner: Baf3 FLT3 cells overexpressing the FLT3 receptor were obtained from Dr. Michael Heinrich (Oregon Health and Sciences University). The Baf3 FLT3 cell lines were created by stable transfection of parental Baf3 cells (a murine B cell lymphoma line dependent on the cytokine IL-3 for growth) with wild-type FLT3. Cells were selected for their ability to grow in the absence of IL-3 and in the presence of FLT3 ligand.

Baf3 cells were maintained in RPMI 1640 with 10% FBS, penn/strep and 10ng/ml FLT ligand at 37 °C, 5%CO₂. To measure direct inhibition of the wild-type FLT3 receptor activity and phosphorylation a sandwich ELISA method was developed similar to those developed for other RTKs (3,4). 200µL of Baf3FLT3 cells (1x10⁶/mL) were plated in 96 well dishes in RPMI 1640 with 0.5% serum and 0.01ng/mL IL-3 for 16 hours prior to 1 hour compound or DMSO vehicle incubation. Cells were treated with 100ng/mL Flt ligand (R&D Systems Cat# 308-FK) for 10 min. at 37°C. Cells were pelleted, washed and lysed in 100ul lysis buffer (50 mM Hepes, 150 mM NaCl, 10% Glycerol, 1% Triton -X-100, 10 mM NaF, 1 mM EDTA, 1.5 mM MgCl₂, 10 mM NaPyrophosphate) supplemented with phosphatase (Sigma Cat# P2850) and protease inhibitors (Sigma Cat #P8340). Lysates were cleared by centrifugation at 1000xg for 5 minutes at 4 °C. Cell lysates were transferred to white wall 96 well microtiter (Costar #9018) plates coated with 50ng/well anti-FLT3 antibody (Santa Cruz Cat# sc-480) and blocked with SeaBlock reagent (Pierce Cat#37527). Lysates were incubated at 4°C for 2 hours. Plates were washed 3x with 200ul/well PBS/0.1% Triton-X-100. Plates were then incubated with 1:8000 dilution of HRP-conjugated anti-phosphotyrosine antibody (Clone 4G10, Upstate Biotechnology Cat#16-105) for 1 hour at room temperature. Plates were washed 3x with 200ul/well PBS/0.1% Triton-X-100. Signal detection with Super Signal Pico reagent (Pierce Cat#37070) was done according to manufacturer's instruction with a Berthold microplate luminometer. All data points are an average of triplicate samples. The total relative light units (RLU) of Flt ligand stimulated FLT3 phosphorylation in the presence of 0.1% DMSO control was defined as 0% inhibition and 100% inhibition was the total RLU of lysate in the basal state. Inhibition and IC₅₀ data analysis was done with GraphPad Prism using a non-linear regression fit with a multiparamater, sigmoidal dose-response (variable slope) equation.

### BIOLOGICAL PROCEDURE REFERENCES

1. Drexler HG. The Leukemia-Lymphoma Cell Line Factsbook Academic Pres: San Diego, CA, 2000.
2. Quentmeier H, Reinhardt J, Zaborski M, Drexler HG. FLT3 mutations in acute myeloid leukemia cell lines. Leukemia. 2003 Jan;17:120-124.
3. Sadick, MD, Sliwkowski, MX, Nuijens, A, Bald, L, Chiang, N, Lofgren, JA, Wong WLT. Analysis of Heregulin-Induced ErbB2 Phosphorylation with a High-Throughput Kinase Receptor Activation Enzyme-Linked Immunsorbent Assay, Analytical Biochemistry. 1996; 235:207-214.
4. Baumann CA, Zeng L, Donatelli RR, Maroney AC. Development of a quantitative, high-throughput cell-based enzyme-linked immunosorbent assay for detection of colony-stimulating factor-1 receptor tyrosine kinase inhibitors. J Biochem Biophys Methods. 2004; 60:69-79.

### BIOLOGICAL DATA

### Biological Data for FLT3

The activity of representative compounds of the present invention is presented in the charts below. All activities are in µM and have the following uncertainties: FLT3 - kinase: ±10%; MV4-11 and Baf3-FLT3: ± 20%.

| **Number** | **Compound** | **FLT3 Kinase (uM)** | **MV4-11 (uM)** | **BaF3 ELISA (uM)** |
|---|---|---|---|---|
| 1 | 4-[6-Amino-5-(methoxyimino-methyl)-pyrimidin-4-yl]-piperazine-1-carboxylic acid (4-isopropoxy-phenyl)-amide | 0.05 | 0.079 | 0.017 |
| 2 | 4-{6-Amino-5-[(2-morpholin-4-yl-ethoxyimino)-methyl]-pyrimidin-4-yl}-piperazine-1-carboxylic acid (4-isopropoxy-phenyl)-amide | 0.036 | 0.177 | 0.081 |
| 3 | 4-{6-Amino-5-[(3-hydroxy-propoxyimino)-methyl]-pyrimidin-4-yl}-piperazine-1-carboxylic acid (4-isopropoxy-phenyl)-amide | 0.26 | 0.283 | 0.072 |
| 4 | 4-[6-Amino-5-(methoxyimino-methyl)-pyrimidin-4-yl]-piperazine-1-carboxylic acid (4-piperidin-1-yl-phenyl)-amide | 0.05 | 0.089 | 0.155 |
| 5 | 4-[6-Amino-5-(methoxyimino-methyl)-pyrimidin-4-yl]-piperazine-1-carboxylic acid (4-morpholin-4-yl-phenyl)-amide | 0.34 | 0.515 | 0.105 |
| 6 | 4-[6-Amino-5-(methoxyimino-methyl)-pyrimidin-4-yl]-piperazine-1-carboxylic acid (6-cyclobutoxy-pyridin-3-yl)-amide | 0.075 | 0.111 | 0.104 |
| 7 | 4-Amino-6-{4-[2-(4-isopropyl-phenyl)-acetyl]-piperazin-1-yl}-pyrimidine-5-carbaldehyde O-methyl-oxime | 0.014 | 0.024 | 0.002 |
| 8 | 4-[6-Amino-5-(methoxyimino-methyl)-pyrimidin-4-yl]-piperazine-1-carboxylic acid (4-isopropyl-phenyl)-amide | 0.082 | 0.147 | 0.189 |
| 9 | 4-[6-Amino-5-(ethoxyimino-methyl)-pyrimidin-4-yl]-piperazine-1-carboxylic acid (4-isopropoxy-phenyl) -amide (*anti*-configuration for -C=N-O-) | 0.018 | 0.077 | 0.022 |
| 10 | 4-[6-Amino-5-(ethoxyimino-methyl)-pyrimidin-4-yl]-piperazine-1-carboxylic acid (4-isopropoxy-phenyl) -amide (*syn*-configuration for -C=N-O-) | 0.12 | 0.075 | 0.267 |
| 11 | 4-[6-Amino-5-(ethoxyimino-methyl)-pyrimidin-4-yl]-piperazine-1-carboxylic acid (4-piperidin-1-yl-phenyl)-amide | 0.049 | 0.058 | 0.026 |
| 12 | 4-[6-Amino-5-(ethoxyimino-methyl)-pyrimidin-4-yl]-piperazine-1-carboxylic acid (6-cyclobutoxy-pyridin-3-yl)-amide | 0.058 | 0.065 | 0.063 |
| 13 | 4-Amino-6-{4-[2-(4-isopropyl-phenyl)-acetyl]-piperazin-1-yl}-pyrimidine-5-carbaldehyde O-ethyl-oxime (*anti*-configuration for -C=N-O-) | 0.008 | 0.013 | 0.116 |
| 14 | 4-Amino-6-{4-[2-(4-isopropyl-phenyl)-acetyl]-piperazin-1-yl}-pyrimidine-5-carbaldehyde O-ethyl-oxime (*syn-*configuration for -C=N-O-) | 0.024 | 0.029 | 0.158 |
| 15 | 4-[6-Amino-5-(ethoxyimino-methyl)-pyrimidin-4-yl]-piperazine-1-carboxylic acid (4-morpholin-4-yl-phenyl)-amide | 0.126 | 0.35 | 0.735 |
| 16 | 4-{6-Amino-5-[(2-morpholin-4-yl-2-oxo-ethoxyimino)-methyl]pyrimidin-4-yl}-piperazine-1-carboxylic acid (4-isopropoxy-phenyl)-amide | 0.486 | 0.268 | 0.187 |
| 17 | 4-[6-Amino-5-(methoxyimino-methyl)-pyrimidin-4-yl]-piperazine-1-carboxylic acid (6-cyclopentyloxy-pyridin-3-yl)-amide | 0.018 | 0.112 | 0.068 |
| 18 | 4-[6-Amino-5-(methoxyimino-methyl)-pyrimidin-4-yl]-piperazine-1-carboxylic acid (4-pyrrolidin-1-yl-phenyl)-amide | 0.003 | 0.099 | 0.312 |
| 19 | 4-[6-Amino-5-(methoxyimino-methyl)-pyrimidin-4-yl]-piperazine-1-carboxylic acid (4-cyclohexyl-phenyl)-amide | 0.099 | 0.052 | 0.012 |
| 20 | 4-[6-Amino-5-(methoxyimino-methyl)-pyrimidin-4-yl]-piperazine-1-carboxylic acid (4-chloro-phenyl)-amide | 1.37 | >1 | 0.11 |
| 21 | 4-[6-Amino-5-(methoxyimino-methyl)-pyrimidin-4-yl]-piperazine-1-carboxylic acid (4-phenoxy-phenyl)-amide | 0.496 | 0.086 | 0.102 |
| 22 | 4-[6-Amino-5-(methoxyimino-methyl)-pyrimidin-4-yl]-piperazine-1-carboxylic acid (4-dimethylamino-phenyl)-amide | 1.87 | 0.472 | 0.058 |
| 23 | 4-[6-Amino-5-(methoxyimino-methyl)-pyrimidin-4-yl]-piperazine-1-carboxylic acid (4-isopropyl-phenyl)-amide | 0.015 | 0.098 | 0.008 |
| 24 | 4-[6-Amino-5-(methoxyimino-methyl)-pyrimidin-4-yl]-[1,4]diazepane-1-carboxylic acid (4-isopropoxy-phenyl)-amide | 0.122 | 0.66 | 0.016 |
| 25 | 4-{6-Amino-5-[(2-amino-ethoxyimino)-methyl]-pyrimidin-4-yl}-piperazine-1-carboxylic acid (4-isopropoxy-phenyl)-amide | 1.15 | 2.0 | nd |
| 26 | 4-(6-Amino-5-{[2-(3-ethyl-ureido)-ethoxyimino]-methyl}-pyrimidin-4-yl)-piperazine-1-carboxylic acid (4-isopropoxy-phenyl)-amide | nd | >1 | nd |
| 27 | 4-{6-Amino-5-[(2-methanesulfonylamino-ethoxyimino)-methyl]-pyrimidin-4-yl}-piperazine-1-carboxylic acid (4-isopropoxy-phenyl)-amide | 0.146 | 0.415 | 0.028 |
| 28 | 4-{6-Amino-5-[(2-morpholin-4-yl-2-oxo-ethoxyimino)-methyl]-pyrimidin-4-yl}-piperazine-1-carboxylic acid (4-pyrrolidin-1-yl-phenyl)-amide | 0.3 | 0.458 | 0.066 |
| 29 | 4-{6-Amino-5-[(2-morpholin-4-yl-ethoxyimino)-methyl]-pyrimidin-4-yl}-piperazine-1-carboxylic acid (4-morpholin-4-yl-phenyl)-amide | >10 | 3.0 | nd |
| 30 | 4-{6-Amino-5-[(2-morpholin-4-yl-ethoxyimino)-methyl]-pyrimidin-4-yl}-piperazine-1-carboxylic acid (6-cyclobutoxy-pyridin-3-yl)-amide | 1.74 | 2.5 | nd |
| 31 | 4-{6-Amino-5-[(2-amino-ethoxyimino)-methyl]-pyrimidin-4-yl}-piperazine-1-carboxylic acid (6-cyclobutoxy-pyridin-3-yl)-amide | 1.45 | 1.3 | 0.206 |
| 32 | 4-{6-Amino-5-[(2-amino-ethoxyimino)-methyl]-pyrimidin-4-yl}-piperazine-1-carboxylic acid (4-morpholin-4-yl-phenyl)-amide | 5.15 | 0.79 | 0.077 |
| 33 | 4-{6-Amino-5-[(2-methanesulfonylamino-ethoxyimino)-methyl]-pyrimidin-4-yl}-piperazine-1-carboxylic acid (6-cyclobutoxy-pyridin-3-yl)-amide | 1.15 | 2.8 | nd |
| 34 | 4-{6-Amino-5-[(2-amino-ethoxyimino)-methyl]-pyrimidin-4-yl}-piperazine-1-carboxylic acid (4-pyrrolidin-1-yl-phenyl)-amide | 1.51 | 2.5 | nd |
| 35 | 4-{6-Amino-5-[(2-methanesulfonylamino-ethoxyimino)-methyl]-pyrimidin-4-yl}-piperazine-1-carboxylic acid (4-pyrrolidin-1-yl-phenyl)-amide | 0.15 | 0.554 | 0.025 |
| 36 | 4-{6-Amino-5-[(2-morpholin-4-yl-ethoxyimino)-methyl]-pyrimidin-4-yl}-piperazine-1-carboxylic acid (4-pyrrolidin-1-yl-phenyl)-amide | 4.04 | 0.362 | 0.530 |
| 37 | 4-{6-Amino-5-[(2-morpholin-4-yl-ethoxyimino)-methyl]-pyrimidin-4-yl}-piperazine-1-carboxylic acid (4-isopropyl-phenyl)-amide | nd | nd | nd |

### Biological Data for TrkB

The activity of representative compounds of the present invention is presented in the charts below. All activities are in µM and have the following uncertainties: TrkB IC₅₀: ±10 %.

| **Number** | **Compound** | **TrkB IC₅₀**µ**M** |
|---|---|---|
| 1 | 4-[6-Amino-5-(methoxyimino-methyl)-pyrimidin-4-yl]-piperazine-1-carboxylic acid (4-isopropoxy-phenyl)-amide | 0.8 |
| 2 | 4-{6-Amino-5-[(2-morpholin-4-yl-ethoxyimino)-methyl]-pyrimidin-4-yl}-piperazine-1-carboxylic acid (4-isopropoxy-phenyl)-amide | 10.1 |
| 3 | 4-{6-Amino-5-[(3-hydroxy-propoxyimino)-methyl]-pyrimidin-4-yl}-piperazine-1-carboxylic acid (4-isopropoxy-phenyl)-amide | 1.3 |
| 4 | 4-[6-Amino-5-(methoxyimino-methyl)-pyrimidin-4-yl]-piperazine-1-carboxylic acid (4-piperidin-1-yl-phenyl)-amide | 4 |
| 5 | 4-[6-Amino-5-(methoxyimino-methyl)-pyrimidin-4-yl]-piperazine-1-carboxylic acid (4-morpholin-4-yl-phenyl)-amide | 8.4 |
| 6 | 4-[6-Amino-5-(methoxyimino-methyl)-pyrimidin-4-yl]-piperazine-1-carboxylic acid (6-cyclobutoxy-pyridin-3-yl)-amide | 3.5 |
| 7 | 4-Amino-6-{4-[2-(4-isopropyl-phenyl)-acetyl]-piperazin-1-yl}-pyrimidine-5-carbaldehyde O-methyl-oxime | 0.5 |
| 8 | 4-[6-Amino-5-(methoxyimino-methyl)-pyrimidin-4-yl]-piperazine-1-carboxylic acid (4-isopropyl-phenyl)-amide | 1.1 |
| 9 | 4-[6-Amino-5-(ethoxymino-methyl)-pyrimidin-4-yl]-piperazine-1-carboxylic acid (4-isopropoxy-phenyl) -amide (*anti*-configuration for -C=N-O-) | 0.6 |
| 10 | 4-[6-Amino-5-(ethoxyimino-methyl)-pyrimidin-4-yl]-piperazine-1-carboxylic acid (4-isopropoxy-phenyl)-amide (*syn*-configuration for -C=N-O-) | 11 |
| 11 | 4-[6-Amino-5-(ethoxyimino-methyl)-pyrimidin-4-yl]-piperazine-1-carboxylic acid (4-piperidin-1-yl-phenyl)-amide | 2.6 |
| 12 | 4-[6-Amino-5-(ethoxyimino-methyl)-pyrimidin-4-yl]-piperazine-1-carboxylic acid (6-cyclobutoxy-pyridin-3-yl)-amide | 1.6 |
| 13 | 4-Amino-6-{4-[2-(4-isopropyl-phenyl)-acetyl]-piperazin-1-yl}-pyrimidine-5-carbaldehyde O-ethyl-oxime (*anti*-configuration for -C=N-O-) | 2.2 |
| 14 | 4-Amino-6-{4-[2-(4-isopropyl-phenyl)-acetyl]-piperazin-1-yl}-pyrimidine-5-carbaldehyde O-ethyl-oxime (*syn*-configuration for -C=N-O-) | 1.4 |
| 15 | 4-[6-Amino-5-(ethoxyimino-methyl)-pyrimidin-4-yl]-piperazine-1-carboxylic acid (4-morpholin-4-yl-phenyl)-amide | 7.76 |
| 16 | 4-{6-Amino-5-[(2-morpholin-4-yl-2-oxo-ethoxyimino)-methyl]-pyrimidin-4-yl}-piperazine-1-carboxylic acid (4-isopropoxy-phenyl)-amide | 11.1 |
| 17 | 4-[6-Amino-5-(methoxyimino-methyl)-pyrimidin-4-yl]-piperazine-1-carboxylic acid (6-cyclopentyloxy-pyridin-3-yl)-amide | 0.8 |
| 18 | 4-[6-Amino-5-(methoxyimino-methyl)-pyrimidin-4-yl]-piperazine-1-carboxylic acid (4-pyrrolidin-1-yl-phenyl)-amide | 2.6 |
| 19 | 4-[6-Amino-5-(methoxyimino-methyl)-pyrimidin-4-yl]-piperazine-1-carboxylic acid (4-cyclohexyl-phenyl)-amide | 1.68 |
| 20 | 4-[6-Amino-5-(methoxyimino-methyl)-pyrimidin-4-yl]-piperazine-1-carboxylic acid (4-chloro-phenyl)-amide | 15.6 |
| 21 | 4-[6-Amino-5-(methoxyimino-methyl)-pyrimidin-4-yl]-piperazine-1-carboxylic acid (4-phenoxy-phenyl)-amide | 7.4 |

### Biological Data for c-kit

The activity of representative compounds of the present invention is presented in the charts below. All activities are in nM and have the following uncertainties: C-Kit IC50: ±10%.

| **Number** | **Compound** | **c-kit IC₅₀nM** |
|---|---|---|
| 1 | 4-[6-Amino-5-(methoxyimino-methyl)-pyrimidin-4-yl]-piperazine-1-carboxylic acid (4-isopropoxy-phenyl)-amide | 10.5 |
| 2 | 4-{6-Amino-5-[(2-morpholin-4-yl-ethoxyimino)-methyl]-pyrimidin-4-yl}-piperazine-1-carboxylic acid (4-isopropoxy-phenyl)-amide | 26 |
| 11 | 4-[6-Amino-5-(ethoxyimino-methyl)-pyrimidin-4-yl]-piperazine-1-carboxylic acid (4-piperidin-1-yl-phenyl)-amide | 5.1 |

### METHODS OF TREATMENT / PREVENTION

In another aspect of this invention, compounds of the invention can be used to inhibit tyrosine kinase activity, including Flt3 activity, and/or c-kit activity, and/or TrkB activity, or reduce kinase activity, including Flt3 activity, and/or c-kit activity, and/or TrkB activity, in a cell or a subject, or to treat disorders related to FLT3, and/or c-kit and/or TrkB kinase activity or expression in a subject.

In one embodiment to this aspect, the present invention provides compounds for reducing or inhibiting the kinase activity of FLT3 and/or c-kit and/or TrkB in a cell The present invention also provides compounds for reducing or inhibiting the kinase activity of FLT3, and/or c-kit and/or TrkB in a subject. Also described is a method of inhibiting cell proliferation in a cell comprising the step of contacting the cell with a compound of Formula I.

The kinase activity of FLT3, c-kit or TrkB in a cell or a subject can be determined by procedures well known in the art, such as the FLT3 kinase assay described herein, the c-kit kinase assay described herein, and the TrkB kinase assay described herein.

The term "subject" as used herein, refers to an animal, preferably a mammal, most preferably a human, who has been the object of treatment, observation or experiment.

The term "contacting" as used herein, refers to the addition of compound to cells such that compound is taken up by the cell.

Also described are prophylactic and therapeutic methods for treating a subject at risk of (or susceptible to) developing a cell proliferative disorder or a disorder related to FLT3 and/or c-kit and/or TrkB.

In one example, the invention provides pharmaceutical compositions for preventing in a subject a disorder related to FLT3 and/or c-kit and/or TrkB, said pharmaceutical compositions comprising a compound of Formula I and a pharmaceutically acceptable carrier. Administration of said prophylactic agent can occur prior to the manifestation of symptoms characteristic of the cell proliferative disorder or disorder related to FLT3 and/or c-kit and/or TrkB, such that a disease or disorder is prevented or, alternatively, delayed in its progression.

In another example, the invention pertains to pharmaceutical compositions for treating in a subject a disorder related to FLT3 and/or c-kit and/or TrkB said pharmaceutical compositions comprising a compound of Formula I and a pharmaceutically acceptable carrier. Administration of said therapeutic agent can occur concurrently with the manifestation of symptoms characteristic of the disorder, such that said therapeutic agent serves as a therapy to compensate for the cell proliferative disorder or disorders related to FLT3 and/or c-kit and/or TrkB.

The term "prophylactically effective amount" refers to an amount of an active compound or pharmaceutical agent that inhibits or delays in a subject the onset of a disorder as being sought by a researcher, veterinarian, medical doctor or other clinician.

The term "therapeutically effective amount" as used herein, refers to an amount of active compound or pharmaceutical agent that elicits the biological or medicinal response in a subject that is being sought by a researcher, veterinarian, medical doctor or other clinician, which includes alleviation of the symptoms of the disease or disorder being treated.

Methods are known in the art for determining therapeutically and prophylactically effective doses for the instant pharmaceutical composition.

As used herein, the term "composition" is intended to encompass a product comprising the specified ingredients in the specified amounts, as well as any product which results, directly or indirectly, from combinations of the specified ingredients in the specified amounts.

As used herein, the terms "disorders related to FLT3", or "disorders related to FLT3 receptor", or "disorders related to FLT3 receptor tyrosine kinase " shall include diseases associated with or implicating FLT3 activity, for example, the overactivity of FLT3, and conditions that accompany with these diseases. The term "overactivity of FLT3 " refers to either 1) FLT3 expression in cells which normally do not express FLT3; 2) FLT3 expression by cells which normally do not express FLT3; 3) increased FLT3 expression leading to unwanted cell proliferation; or 4) mutations leading to constitutive activation of FLT3. Examples of "disorders related to FLT3" include disorders resulting from over stimulation of FLT3 due to abnormally high amount of FLT3 or mutations in FLT3, or disorders resulting from abnormally high amount of FLT3 activity due to abnormally high amount of FLT3 or mutations in FLT3. It is known that overactivity of FLT3 has been implicated in the pathogenesis of a number of diseases, including the cell proliferative disorders, neoplastic disorders and cancers listed below.

The term "cell proliferative disorders" refers to unwanted cell proliferation of one or more subset of cells in a multicellular organism resulting in harm (i.e., discomfort or decreased life expectancy) to the multicellular organisms. Cell proliferative disorders can occur in different types of animals and humans. For example, as used herein "cell proliferative disorders" include neoplastic and other cell proliferative disorders.

As used herein, a "neoplastic disorder" refers to a tumor resulting from abnormal or uncontrolled cellular growth. Examples of neoplastic disorders include, but are not limited to, hematopoietic disorders such as, for instance, the myeloproliferative disorders, such as thrombocythemia, essential thrombocytosis (ET), agnogenic myeloid metaplasia, myelofibrosis (MF), myelofibrosis with myeloid metaplasia (MMM), chronic idiopathic myelofibrosis (IMF), and polycythemia vera (PV), the cytopenias, and pre-malignant myelodysplastic syndromes; cancers such as glioma cancers, lung cancers, breast cancers, colorectal cancers, prostate cancers, gastric cancers, esophageal cancers, colon cancers, pancreatic cancers, ovarian cancers, and hematoglogical malignancies, including myelodysplasia, multiple myeloma, leukemias and lymphomas. Examples of hematological malignancies include, for instance, leukemias, lymphomas (non-Hodgkin's lymphoma), Hodgkin's disease (also called Hodgkin's lymphoma), and myeloma -- for instance, acute lymphocytic leukemia (ALL), acute myeloid leukemia (AML), acute promyelocytic leukemia (APL), chronic lymphocytic leukemia (CLL), chronic myeloid leukemia (CML), chronic neutrophilic leukemia (CNL), acute undifferentiated leukemia (AUL), anaplastic large-cell lymphoma (ALCL), prolymphocytic leukemia (PML), juvenile myelomonocyctic leukemia (JMML), adult T-cell ALL, AML with trilineage myelodysplasia (AMI/TMDS), mixed lineage leukemia (MLL), myelodysplastic syndromes (MDSs), myeloproliferative disorders (MPD), and multiple myeloma, (MM).

Examples of other cell proliferative disorders, include but are not limited to, atherosclerosis (Libby P, 2003, "Vascular biology of atherosclerosis: overview and state of the art", Am J Cardiol 91(3A):3A-6A) transplantation-induced vasculopathies (Helisch A, Schaper W. 2003, Arteriogenesis: the development and growth of collateral arteries. Microcirculation, 10(1):83-97), macular degeneration (Holz FG et al., 2004, "Pathogenesis of lesions in late age-related macular disease", Am J Ophthalmol. 137(3):504-10), neointima hyperplasia and restenosis (Schiele TM et. al., 2004, "Vascular restenosis - striving for therapy." Expert Opin Pharmacother. 5(11):2221-32) , pulmonary fibrosis (Thannickal VJ et al., 2003, "Idiopathic pulmonary fibrosis: emerging concepts on pharmacotherapy, Expert Opin Pharmacother. 5(8):1671-86), glomerulonephritis (Cybulsky AV, 2000, "Growth factor pathways in proliferative glomerulonephritis", Curr Opin Nephrol Hypertens " 9(3):217-23), glomerulosclerosis (Harris RC et al, 1999, "Molecular basis of injury and progression in focal glomerulosclerosis" Nephron 82(4):289-99), renal dysplasia and kidney fibrosis (Woolf AS et al., 2004, "Evolving concepts in human renal dysplasia", J Am Soc Nephrol.15(4):998-1007), diabetic retinopathy (Grant MB et al., 2004, "The role of growth factors in the pathogenesis of diabetic retinopathy", Expert Opin Investig Drugs 13(10):1275-93) and rheumatoid arthritis (Sweeney SE, Firestein GS, 2004, Rheumatoid arthritis: regulation of synovial inflammation, Int J Biochem Cell Biol. 36(3):372-8).

As used herein, the terms "disorders related to TrkB", or "disorders related to the TrkB receptor", or "disorders related to the TrkB receptor tyrosine kinase" shall include diseases associated with or implicating TrkB activity, for example, the overactivity of TrkB, and conditions that accompany these diseases. The term "overactivity of TrkB " refers to either 1) TrkB expression in cells which normally do not express TrkB; 2) TrkB expression by cells which normally do not express TrkB; 3) increased TrkB expression leading to unwanted cell proliferation; or 4) increased TrkB expression leading to adhesion independent cell survival; 5) mutations leading to constitutive activation of TrkB. Examples of "disorders related to TrkB" include 1) disorders resulting from over stimulation of TrkB due to abnormally high amount of TrkB or mutations in TrkB, or 2) disorders resulting from abnormally high amount of TrkB activity due to abnormally high amount of TrkB or mutations in TrkB.

Disorders related to TrkB include a number of diseases, including cancers, such as, but not limited to, neuroblastoma, wilm's tumor, breast, colon, prostate, and lung. See, e.g., Brodeur GM, (2003) "Neuroblastoma: biological insights into a clinical enigma." Nat RevCancer; 3(3):203-16; Eggerl A et. al. (2001) "Expression of the neurotrophin receptor TrkB is associated with unfavorable outcome in Wilms' tumor" J Clin Oncol. 19(3):689-96; Descamps S et.al.(2001) "Nerve growth factor stimulates proliferation and survival of human breast cancer cells through two distinct signaling pathways." J Biol Chem. 276(21):17864-70; Bardelli A, et, al. (2003) "Mutational analysis of the tyrosine kinome in colorectal cancers." Science 300(5621):949; Weeraratna AT et, al. (2000) "Rational basis for Trk inhibition therapy for prostate cancer." Prostate 45(2):140-8.19(3):689-96; Ricci et. al., (2001) "Neurotrophins and neurotrophin receptors in human lung cancer." Am J Respir Cell Mol Biol. 25(4):439-46.

As used herein, the terms "disorders related to c-kit", or "disorders related to c-kit receptor", or "disorders related to c-kit receptor tyrosine kinase " shall include diseases associated with or implicating c-kit activity, for example, the overactivity of c-kit, and conditions that accompany with these diseases. The term "overactivity of c-kit" refers to either 1) c-kit expression in cells which normally do not express c-kit; 2) c-kit expression by cells which normally do not express c-kit; 3) increased c-kit expression leading to unwanted cell proliferation; or 4) mutations leading to constitutive activation of c-kit. Examples of "disorders related to c-kit" include disorders resulting from over stimulation of c-kit due to abnormally high amount of c-kit or mutations in c-kit, or disorders resulting from abnormally high amount of c-kit activity due to abnormally high amount of c-kit or mutations in c-kit.

Disorders related to c-Kit include, a number of diseases, such as mastocytosis, must cell leukemia, gastrointestinal stromal tumour, sinonasal natural killer/T-cell lymphoma, seminoma, dysgerminoma, thyroid carcinoma; small-cell lung carcinoma, malignant melanoma, adenoid cystic carcinoma, ovarian carcinoma, acute myelogenous leukemia, anaplastic large cell lymphoma, angiosarcoma, endometrial carcinoma, pediatric T-cell ALL, lymphoma, breast carcinoma and prostate carcinoma. See Heinrich, Michael C. et al. Review Article: Inhibition of KIT Tyrosine Kinase Activity: A Novel Molecular Approach to the Treatment of KIT-Positive Malignancies.

In a further embodiment to this aspect, the invention encompasses a combination therapy for treating or preventing a disorder related to FLT3 and/or c-kit and/or TrkB in a subject. The combination therapy comprises administering to the subject a therapeutically or prophylactically effective amount of a compound of Formula I, and one or more other anti-cell proliferation therapy including chemotherapy, radiation therapy, gene therapy and immunotherapy.

In an embodiment of the present invention, the compound of the present invention may be administered in combination with chemotherapy. As used herein, chemotherapy refers to a therapy involving a chemotherapeutic agent. A variety of chemotherapeutic agents may be used in the combined treatment methods disclosed herein. Chemotherapeutic agents contemplated as exemplary, include, but are not limited to: platinum compounds (e.g.,cisplatin, carboplatin, oxaliplatin); taxane compounds (e.g., paclitaxcel, docetaxol); campotothecin compounds (irinotecan, topotecan); ; vinca alkaloids (e.g., vincristine, vinblastine, vinorelbine); anti-tumor nucleoside derivatives (e.g., 5-fluorouracil, leucovorin, gemcitabine, capecitabine) ; alkylating agents (e.g., cyclophosphamide, carmustine, lomustine, thiotepa); epipodophyllotoxins / podophyllotoxins (e.g. etoposide, teniposide); aromatase inhibitors (e.g.; anastrozole, letrozole, exemestane); anti-estrogen compounds (e.g., tamoxifen, fulvestrant), antifolates (e.g., premetrexed disodium); hypomethylating agents (e.g., azacitidine); biologics (e.g., gemtuzamab, cetuximab, rituximab, pertuzumab, trastuzumab, bevacizumab, erlotinib); antibiotics/anthracyclines (e.g. idarubicin, actinomycin D, bleomycin, daunorubicin, doxorubicin, mitomycin C, dactinomycin, carminomycin, daunomycin); antimetabolites (e.g., aminopterin, clofarabine, cytosine arabinoside, methotrexate); tubulin-binding agents (e.g. combretastatin, colchicine, nocodazole); topoisomerase inhibitors (e.g., camptothecin). Further useful agents include verapamil, a calcium antagonist found to be useful in combination with antineoplastic agents to establish chemosensitivity in tumor cells resistant to accepted chemotherapeutic agents and to potentiate the efficacy of such compounds in drug-sensitive malignancies. See Simpson WG, The calcium channel blocker verapamil and cancer chemotherapy. Cell Calcium. 1985 Dec;6(6):449-67. Additionally, yet to emerge chemotherapeutic agents are contemplated as being useful in combination with the compound of the present invention.

In another embodiment of the present invention, the compound of the present invention may be administered in combination with radiation therapy. As used herein, "radiation therapy" refers to a therapy comprising exposing the subject in need thereof to radiation. Such therapy is known to those skilled in the art. The appropriate scheme of radiation therapy will be similar to those already employed in clinical therapies wherein the radiation therapy is used alone or in combination with other chemotherapeutics.

In another embodiment of the present invention, the compound of the present invention may be administered in combination with a gene therapy. As used herein, "gene therapy" refers to a therapy targeting on particular genes involved in tumor development. Possible gene therapy strategies include the restoration of defective cancer-inhibitory genes, cell transduction or transfection with antisense DNA corresponding to genes coding for growth factors and their receptors, RNA-based strategies such as ribozymes, RNA decoys, antisense messenger RNAs and small interfering RNA (siRNA) molecules and the so-called 'suicide genes'.

In other embodiments of this invention, the compound of the present invention may be administered in combination with an immunotherapy. As used herein, "immunotherapy" refers to a therapy targeting particular protein involved in tumor development via antibodies specific to such protein. For example, monoclonal antibodies against vascular endothelial growth factor have been used in treating cancers.

Where a second pharmaceutical is used in addition to a compound of the present invention, the two pharmaceuticals may be administered simultaneously (e.g. in separate or unitary compositions) sequentially in either order, at approximately the same time, or on separate dosing schedules. In the latter case, the two compounds will be administered within a period and in an amount and manner that is sufficient to ensure that an advantageous or synergistic effect is achieved. It will be appreciated that the preferred method and order of administration and the respective dosage amounts and regimes for each component of the combination will depend on the particular chemotherapeutic agent being administered in conjunction with the compound of the present invention, their route of administration, the particular tumor being treated and the particular host being treated.

As will be understood by those of ordinary skill in the art, the appropriate doses of chemotherapeutic agents will be generally similar to or less than those already employed in clinical therapies wherein the chemotherapeutics are administered alone or in combination with other chemotherapeutics.

The optimum method and order of administration and the dosage amounts and regime can be readily determined by those skilled in the art using conventional methods and in view of the information set out herein.

By way of example only, platinum compounds are advantageously administered in a dosage of 1 to 500 mg per square meter (mg/m²) of body surface area, for example 50 to 400 mg/m², particularly for cisplatin in a dosage of about 75 mg/m² and for carboplatin in about 300mg/m² per course of treatment. Cisplatin is not absorbed orally and must therefore be delivered via injection intravenously, subcutaneously, intratumorally or intraperitoneally.

By way of example only, taxane compounds are advantageously administered in a dosage of 50 to 400 mg per square meter (mg/m²) of body surface area, for example 75 to 250 mg/m², particularly for paclitaxel in a dosage of about 175 to 250 mg/m² and for docetaxel in about 75 to 150 mg/m² per course of treatment.

By way of example only, camptothecin compounds are advantageously administered in a dosage of 0.1 to 400 mg per square meter (mg/m²) of body surface area, for example 1 to 300 mg/m², particularly for irinotecan in a dosage of about 100 to 350 mg/m² and for topotecan in about 1 to 2 mg/m² per course of treatment.

By way of example only, vinca alkaloid may be advantageously administered in a dosage of 2 to 30 mg per square meter (mg/m²) of body surface area, particularly for vinblastine in a dosage of about 3 to 12 mg/m² , for vincristine in a dosage of about 1 to 2 mg/m² , and for vinorelbine in dosage of about 10 to 30 mg/m² per course of treatment.

By way of example only, anti-tumor nucleoside derivatives may be advantageously administered in a dosage of 200 to 2500 mg per square meter (mg/m²) of body surface area, for example 700 to 1500 mg/m². 5-fluorouracil (5-FU) is commonly used via intravenous administration with doses ranging from 200 to 500mg/m² (preferably from 3 to 15 mg/kg/day). Gemcitabine is advantageously administered in a dosage of about 800 to 1200 mg/m² and capecitabine is advantageously administered in about 1000 to 2500 mg/m² per course of treatment.

By way of example only, alkylating agents may be advantageously administered in a dosage of 100 to 500 mg per square meter (mg/m²) of body surface area, for example 120 to 200 mg/m², particularly for cyclophosphamide in a dosage of about 100 to 500 mg/m², for chlorambucil in a dosage of about 0.1 to 0.2 mg/kg of body weight, for carmustine in a dosage of about 150 to 200 mg/m² , and for lomustine in a dosage of abut 100 to 150 mg/m² per course of treatment.

By way of example only, podophyllotoxin derivatives may be advantageously administered in a dosage of 30 to 300 mg per square meter (mg/m²) of body surface area, for example 50 to 250 mg/m², particularly for etoposide in a dosage of about 35 to 100 mg/m² and for teniposide in about 50 to 250 mg/m² per course of treatment.

By way of example only, anthracycline derivatives may be advantageously administered in a dosage of 10 to 75 mg per square meter (mg/m²) of body surface area, for example 15 to 60 mg/m², particularly for doxorubicin in a dosage of about 40 to 75 mg/m², for daunorubicin in a dosage of about 25 to 45mg/m², and for idarubicin in a dosage of about 10 to 15 mg/m² per course of treatment.

By way of example only, anti-estrogen compounds may be advantageously administered in a dosage of about 1 to 100mg daily depending on the particular agent and the condition being treated. Tamoxifen is advantageously administered orally in a dosage of 5 to 50 mg, preferably 10 to 20 mg twice a day, continuing the therapy for sufficient time to achieve and maintain a therapeutic effect. Toremifene is advantageously administered orally in a dosage of about 60mg once a day, continuing the therapy for sufficient time to achieve and maintain a therapeutic effect. Anastrozole is advantageously administered orally in a dosage of about 1mg once a day. Droloxifene is advantageously administered orally in a dosage of about 20-100mg once a day. Raloxifene is advantageously administered orally in a dosage of about 60mg once a day. Exemestane is advantageously administered orally in a dosage of about 25mg once a day.

By way of example only, biologics may be advantageously administered in a dosage of about 1 to 5 mg per square meter (mg/m²) of body surface area, or as known in the art, if different. For example, trastuzumab is advantageously administered in a dosage of 1 to 5 mg/m² particularly 2 to 4mg/m² per course of treatment.

Dosages may be administered, for example once, twice or more per course of treatment, which may be repeated for example every 7, 14, 21 or 28 days.

The compounds of the present invention can be administered to a subject systemically, for example, intravenously, orally, subcutaneously, intramuscular, intradermal, or parenterally. The compounds of the present invention can also be administered to a subject locally. Non-limiting examples of local delivery systems include the use of intraluminal medical devices that include intravascular drug delivery catheters, wires, pharmacological stents and endoluminal paving. The compounds of the present invention can further be administered to a subject in combination with a targeting agent to achieve high local concentration of the compound at the target site. In addition, the compounds of the present invention may be formulated for fast-release or slow-release with the objective of maintaining the drugs or agents in contact with target tissues for a period ranging from hours to weeks.

The present invention also provides a pharmaceutical composition comprising a compound of Formula I in association with a pharmaceutically acceptable carrier. The pharmaceutical composition may contain between about 0.1 mg and 1000 mg, preferably about 100 to 500 mg, of the compound, and may be constituted into any form suitable for the mode of administration selected.

The phrases "pharmaceutically acceptable" refer to molecular entities and compositions that do not produce an adverse, allergic or other untoward reaction when administered to an animal, or a human, as appropriate. Veterinary uses are equally included within the invention and "pharmaceutically acceptable" formulations include formulations for both clinical and/or veterinary use.

Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixirs, emulsions, and suspensions. Forms useful for parenteral administration include sterile solutions, emulsions and suspensions.

The pharmaceutical composition of the present invention also includes a pharmaceutical composition for slow release of a compound of the present invention. The composition includes a slow release carrier (typically, a polymeric carrier) and a compound of the present invention.

Slow release biodegradable carriers are well known in the art. These are materials that may form particles that capture therein an active compound(s) and slowly degrade/dissolve under a suitable environment (e.g., aqueous, acidic, basic, etc) and thereby degrade/dissolve in body fluids and release the active compound(s) therein. The particles are preferably nanoparticles (i.e., in the range of about 1 to 500 nm in diameter, preferably about 50-200 nm in diameter, and most preferably about 100 nm in diameter).

The present invention also provides methods to prepare the pharmaceutical compositions of this invention. The compound of Formula I, as the active ingredient, is intimately admixed with a pharmaceutical carrier according to conventional pharmaceutical compounding techniques, which carrier may take a wide variety of forms depending on the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, though other ingredients, for example, for purposes such as aiding solubility or for preservation, may be included. Injectable suspensions may also be prepared, in which case appropriate liquid carriers, suspending agents and the like may be employed. In preparation for slow release, a slow release carrier, typically a polymeric carrier, and a compound of the present invention are first dissolved or dispersed in an organic solvent. The obtained organic solution is then added into an aqueous solution to obtain an oil-in-water-type emulsion. Preferably, the aqueous solution includes surface-active agent(s). Subsequently, the organic solvent is evaporated from the oil-in-water-type emulsion to obtain a colloidal suspension of particles containing the slow release carrier and the compound of the present invention.

The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, from about 0.01 mg to 200 mg/kg of body weight per day. Preferably, the range is from about 0.03 to about 100 mg/kg of body weight per day, most preferably, from about 0.05 to about 10 mg/kg of body weight per day. The compounds may be administered on a regimen of 1 to 5 times per day. The dosages, however, may be varied depending upon the requirement of the patients, the severity of the condition being treated and the compound being employed. The use of either daily administration or post-periodic dosing may be employed.

Preferably these compositions are in unit dosage forms such as tablets, pills, capsules, powders, granules, sterile parenteral solutions or suspensions, metered aerosol or liquid sprays, drops, ampoules, auto-injector devices or suppositories; for oral parenteral, intranasal, sublingual or rectal administration, or for administration by inhalation or insulation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, acetyl alcohol and cellulose acetate.

The liquid forms in which the compound of Formula I may be incorporated for administration orally or by injection include, aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin. The liquid forms in suitably flavored suspending or dispersing agents may also include the synthetic and natural gums, for example, tragacanth, acacia, methylcellulose and the like. For parenteral administration, sterile suspensions and solutions are desired. Isotonic preparations which generally contain suitable preservatives are employed when intravenous administration is desired.

Advantageously, compounds of Formula I may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The daily dosage of the products of the present invention may be varied over a wide range from 1 to 5000 mg per adult human per day. For oral administration, the compositions are preferably provided in the form of tablets containing, 0.01,0.05, 0.1, 0.5, 1.0, 2.5, 5.0, 10.0, 15.0, 25.0, 50.0, 100, 150, 200, 250 and 500 milligrams of the active ingredient for the symptomatic adjustment of the dosage to the patient to be treated. An effective amount of the drug is ordinarily supplied at a dosage level of from about 0.01 mg/kg to about 200 mg/kg of body weight per day. Particularly, the range is from about 0.03 to about 15 mg/kg of body weight per day, and more particularly, from about 0:05 to about 10 mg/kg of body weight per day. The compound of the present invention may be administered on a regimen up to four or more times per day, preferably of 1 to 2 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

The compounds of the present invention can also be administered in the form of liposome delivery systems, such as small unilamellar vesicles, large unilamellar vesicles, and multilamellar vesicles. Liposomes can be formed from a variety of lipids, including but not limited to amphipathic lipids such as phosphatidylcholines, sphingomyelins, phosphatidylethanolamines, phophatidylcholines, cardiolipins, phosphatidylserines, phosphatidylglycerols, phosphatidic acids, phosphatidylinositols, diacyl trimethylammonium propanes, diacyl dimethylammonium propanes, and stearylamine, neutral lipids such as triglycerides, and combinations thereof. They may either contain cholesterol or may be cholesterol-free.

The compounds of the present invention can also be administered locally. Any delivery device, such as intravascular drug delivery catheters, wires, pharmacological stents and endoluminal paving, may be utilized. The delivery system for such a device may comprise a local infusion catheter that delivers the compound at a rate controlled by the administor.

The present invention provides a drug delivery device comprising an intraluminal medical device, preferably a stent, and a therapeutic dosage of a compound of the invention.

The term "stent" refers to any device capable of being delivered by a catheter. A stent is routinely used to prevent vascular closure due to physical anomalies such as unwanted inward growth of vascular tissue due to surgical trauma. It often has a tubular, expanding lattice-type structure appropriate to be left inside the lumen of a duct to relieve an obstruction. The stent has a lumen wall-contacting surface and a lumen-exposed surface. The lumen-wall contacting surface is the outside surface of the tube and the lumen-exposed surface is the inner surface of the tube. The stent can be polymeric, metallic or polymeric and metallic, and it can optionally be biodegradable.

Commonly, stents are inserted into the lumen in a non-expanded form and are then expanded autonomously, or with the aid of a second device in situ. A typical method of expansion occurs through the use of a catheter-mounted angioplastry balloon which is inflated within the stenosed vessel or body passageway in order to shear and disrupt the obstructions associated with the wall components of the vessel and to obtain an enlarged lumen. Self-expanding stents as described in U.S. 6,776,796 (Falotico et al.) may also be utilized. The combination of a stent with drugs, agents or compounds which prevent inflammation and proliferation, may provide the most efficacious treatment for post-angioplastry restenosis.

Compounds of the invention can be incorporated into or affixed to the stent in a number of ways and in utilizing any number of biocompatible materials. In one exemplary embodiment, the compound is directly incorporated into a polymeric matrix, such as the polymer polypyrrole, and subsequently coated onto the outer surface of the stent. The compound elutes from the matrix by diffusion through the polymer. Stents and methods for coating drugs on stents are discussed in detail in the art. In another exemplary embodiment, the stent is first coated with as a base layer comprising a solution of the compound, ethylene-co-vinylacetate, and polybutylmethacrylate. Then, the stent is further coated with an outer layer comprising only polybutylmethacrylate. The outlayer acts as a diffusion barrier to prevent the compound from eluting too quickly and entering the surrounding tissues. The thickness of the outer layer or topcoat determines the rate at which the compound elutes from the matrix. Stents and methods for coating are discussed in detail in WIPO publication WO9632907, U.S. Publicadon No. 2002/0016625 and references disclosed therein.

The solution of the compound of the invention and the biocompatible materials/polymers may be incorporated into or onto a stent in a number of ways. For example, the solution may be sprayed onto the stent or the stent may be dipped into the solution. In a preferred embodiment, the solution is sprayed onto the stent arid then allowed to dry. In another exemplary embodiment, the solution may be electrically charged to one polarity and the stent electrically changed to the opposite polarity. In this manner, the solution and stent will be attracted to one another. In using this type of spraying process, waste may be reduced and more control over the thickness of the coat may be achieved. Compound is preferably only affixed to the outer surface of the stent which makes contact with one tissue. However, for some compounds, the entire stent may be coated. The combination of the dose of compound applied to the stent and the polymer coating that controls the release of the drug is important in the effectiveness of the drug. The compound preferably remains on the stent for at least three days up to approximately six months and more, preferably between seven and thirty days.

Any number of non-erodible biocompatible polymers may be utilized in conjunction with the compound of the invention. It is important to note that different polymers may be utilized for different stents. For example, the above-described ethylene-co-vinylacetate and polybutylmethacrylate matrix works well with stainless steel stents. Other polymers may be utilized more effectively with stents formed from other materials, including materials that exhibit superelastic properties such as alloys of nickel and titanium.

Restensosis is responsible for a significant morbidity and mortality following coronary angioplasty. Restenosis occurs through a combination of four processes including elastic recoil, thrombus formation, intima hyperplasia and extracellular matrix remodeling. Several growth factors have been recently identified to play a part in these processes leading to restenosis (see, Schiele TM et. al., 2004, "Vascular restenosis - striving for therapy." Expert Opin Pharmacother. 5(11):2221-32.). Of note, TrkB ligands BDNF and neurotrophins as well as TrkB are expressed by vascular smooth muscle cells and endothelial cells (see,Ricci A, et. al. 2003 ", Neurotrophins and neurotrophin receptors in human pulmonary arteries." J Vasc Res. 37(5):355-63; see also, Kim H, et. al., 2004 "Paracrine and autocrine functions of brain-derived neurotrophic factor (BDNF) and nerve growth factor (NGF) in brain-derived endothelial cells", J Biol Chem. 279(32):33538-46). Additionally, TrkB may play a role in peripheral angiogenesis and intima hyperplasia because of its ability to prevent anoikis and prolong cell survival (see, Douma S, et. al.,2004, "Suppression of anoikis and induction of metastasis by the neurotrophic receptor TrkB", Nature. 430(7003):1034-9.). Therefore, inhibition of TrkB during and following coronary angioplasty using a coated stent presents a viable therapeutic strategy.

Also described is a method for the treatment of disorders related to TrkB, including restenosis, intimal hyperplasia or inflammation, in blood vessel walls, in a subject comprising administering to the subject a compound of the invention in a therapeutically effective amounts by the controlled delivery, by release from an intraluminal medical device, such as a stent, of the compound of the invention.

Methods for introducing a stent into a lumen of a body are well known and the compound-coated stents of this invention are preferably introduced using a catheter As will be appreciated by those of ordinary skill in the art, methods will vary slightly based on the location of stent implantation. For coronary stent implantation, the balloon catheter bearing the stent is inserted into the coronary artery and the stent is positioned at the desired site. The balloon is inflated, expanding the stent. As the stent expands, the stent contacts the lumen wall. Once the stent is positioned, the balloon is deflated and removed. The stent remains in place with the lumen-contacting surface bearing the compound directly contacting the lumen wall surface. Stent implantation may be accompanied by anticoagulation therapy as needed.

Optimum conditions for delivery of the compounds for use in the stent of the invention may vary with the different local delivery systems used, as well as the properties and concentrations of the compounds used. Conditions that may be optimized include, for example, the concentrations of the compounds, the delivery volume, the delivery rate, the depth of penetration of the vessel wall, the proximal inflation pressure, the amount and size of perforations and the fit of the drug delivery catheter balloon. Conditions may be optimized for inhibition of smooth muscle cell proliferation at the site of injury such that significant arterial blockage due to restenosis does not occur, as measured, for example, by the proliferative ability of the smooth muscle cells, or by changes in the vascular resistance or lumen diameter. Optimum conditions can be determined based on data from animal model studies using routine computational methods.

Another alternative method for administering compounds of this invention may be by conjugating the compound to a targeting agent which directs the conjugate to its intended site of action, i.e., to vascular endothelial cells, or to tumor cells. Both antibody and non-antibody targeting agents may be used. Because of the specific interaction between the targeting agent and its corresponding binding partner, a compound of the present invention can be administered with high local concentrations at or near a target site and thus treats the disorder at the target site more effectively.

The antibody targeting agents include antibodies or antigen-binding fragments thereof, that bind to a targetable or accessible component of a tumor cell, tumor vasculature, or tumor stroma. The "targetable or accessible component" of a tumor cell, tumor vasculature or tumor stroma, is preferably a surface-expressed, surface-accessible or surface-localized component. The antibody targeting agents also include antibodies or antigen-binding fragments thereof, that bind to an intracellular component that is released from a necrotic tumor cell. Preferably such antibodies are monoclonal antibodies, or antigen-binding fragments thereof, that bind to insoluble intracellular antigen(s) present in cells that may be induced to be permeable, or in cell ghosts of substantially all neoplastic and normal cells, but are not present or accessible on the exterior of normal living cells of a mammal.

As used herein, the term "antibody" is intended to refer broadly to any immunologic binding agent such as IgG, IgM, IgA, IgE, F(ab')2, a univalent fragment such as Fab', Fab, Dab, as well as engineered antibodies such as recombinant antibodies, humanized antibodies, bispecific antibodies, and the like. The antibody can be either the polyclonal or the monoclonal, although the monoclonal is preferred. There is a very broad array of antibodies known in the art that have immunological specificity for the cell surface of virtually any solid tumor type (see, Summary Table on monoclonal antibodies for solid tumors in US Patent No. 5,855,866 to Thorpe et al). Methods are known to those skilled in the art to produce and isolate antibodies against tumor (see, US Patent No.5,855,866 to Thorpe et al., and US Patent No.6,34,2219 to Thorpe et al.).

Techniques for conjugating therapeutic moiety to antibodies are well known. (See, e.g., Amon et al., "Monoclonal Antibodies For Immunotargeting Of Drugs In Cancer Therapy", in Monoclonal Antibodies And Cancer Therapy, Reisfeld et al. (eds.), pp. 243- 56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985)). Similar techniques can also be applied to attach compounds of the invention to non-antibody targeting agents. Those skilled in the art will know, or be able to determine, methods of forming conjugates with non-antibody targeting agents, such as small molecules, oligopeptides, polysaccharides, or other polyanionic compounds.

Although any linking moiety that is reasonably stable in blood, can be used to link the compounds of the present invention to the targeting agent, biologically-releasable bonds and/or selectively cleavable spacers or linkers are preferred. "Biologically-releasable bonds" and "selectively cleavable spacers or linkers" still have reasonable stability in the circulation, but are releasable, cleavable or hydrolyzable only or preferentially under certain conditions, i.e., within a certain environment, or in contact with a particular agent. Such bonds include, for example, disulfide and trisulfide bonds and acid-labile bonds, as described in U.S. Pat. Nos. 5, 474,765 and 5,762,918 and enzyme-sensitive bonds, including peptide bonds, esters, amides, phosphodiesters and glycosides as described in U.S. Pat. Nos. 5,474,765 and 5,762,918. Such selective-release design features facilitate sustained release of the compounds from the conjugates at the intended target site.

The present invention provides a pharmaceutical composition comprising an effective amount of a compound of the present invention conjugated to a targeting agent and a pharmaceutically acceptable carrier.

The present invention further provides compounds conjugated to a targeting agent for treating a cell proliferative disorder or a disorder related to FL'1'3 and/or c-kit and/or TrkB.

When proteins such as antibodies or growth factors, or polysaccharides are used as targeting agents, they are preferably administered in the form of injectable compositions. The injectable antibody solution will be administered into a vein, artery or into the spinal fluid over the course of from 2 minutes to about 45 minutes, preferably from 10 to 20 minutes. In certain cases, intradermal and intracavitary administration are advantageous for tumors restricted to areas close to particular regions of the skin and/or to particular body cavities. In addition, intrathecal administrations may be used for tumors located in the brain.

Therapeutically effective dose of the compound of the present invention conjugated to a targeting agent depends on the individual, the discase type, the disease state, the method of administration and other clinical variable. The effective dosages are readily determinable using data from an animal model. Experimental animals bearing solid tumors are frequently used to optimize appropriate therapeutic doses prior to translating to a clinical environment. Such models are known to be very reliable in predicting effective anti-cancer strategies. For example, mice bearing solid tumors, are widely used in pre-clinical testing to determine working ranges of therapeutic agents that give beneficial anti-tumor effects with minimal toxicity.

## Claims

1. A compound of Formula I: 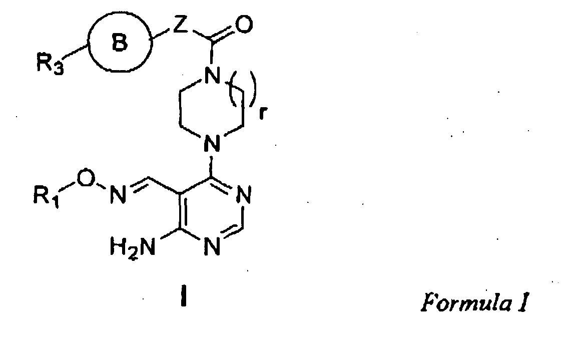 and N-oxides, pharmaceutically acceptable salts, solvates, geometric isomers and stereochemical isomers thereof, wherein:
**r** is 1 or 2;
**Z** is NH, N(alkyl), or CH₂;
**B** is phenyl, heteroaryl, or a nine to ten membered benzo-fused heteroaryl;
**R₁** is: 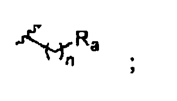
wherein n is 1,2, 3 or 4;
**Rₐ** is hydrogen, alkoxy, phenoxy, phenyl, heteroaryl optionally substituted with R₅, hydroxyl, amino, alkylamino, dialkylamino, oxazolidinonyl optionally substituted with R₅, pyrrolidinonyl optionally substituted with R₅, piperidinonyl optionally substituted with R₅, cyclic heterodionyl optionally substituted with R₅, heterocyclyl optionally substituted with R₅, -COOR_{y}, -CONR_{w}Rₓ, -N(R_{w})CON(R_{y})(Rₓ), -N(R_{y})CON(R_{w})(Rₓ), -N(R_{w})C(O)ORₓ, -N(R_{w})COR_{y}, -SR_{y}, -SOR_{y}, -SO₂R_{y}, -NR_{w}SO₂R_{y.} -NR_{w}SO₂Rₓ, -SO₃R_{y}, -OSO₂NR_{w}Rₓ, or -SO₂NR_{w}Rₓ;
**R_{w}** and **Rₓ** are independently selected from: hydrogen, alkyl, alkenyl, aralkyl, or heteroaralkyl, or **R_{w}** and **Rₓ** may optionally be taken together to form a 5 to 7 membered ring, optionally containing a heteromoiety selected from O, NH, N(alkyl), SO₂, SO, or S;
**R_{y}** is selected from: hydrogen, alkyl, alkenyl, cycloalkyl, phenyl, aralkyl, heteroaralkyl, or heteroaryl;
**R₅** is one, two, or three substituents independently selected from: halogen, cyano, trifluoromethyl, amino, hydroxyl, alkoxy, -C(O)alkyl, -SO₂alkyl, -C(O)N(alkyl)₂, alkyl, C(₁₋₄)alkyl-OH, or alkylamino; and
**R₃** is one or more substituents independently selected from: hydrogen, alkyl, alkoxy, halogen, alkoxyether, hydroxyl, thio, nitro, cycloalkyl optionally substituted with R₄, heteroaryl optionally substituted with R₄, alkylamino, heterocyclyl optionally substituted with R₄, -O(cycloalkyl), pyrrolidinonyl optionally substituted with R₄, phenoxy optionally substituted with R₄, -CN, -OCHF₂, -OCF₃, -CF₃, halogenated alkyl, hetcroaryloxy optionally substituted with R₄, dialkylamino, -NHSO₂alkyl, thioalkyl, or -SO₂alkyl; wherein **R₄** is independently selected from: halogen, cyano, trifluoromethyl, amino, hydroxyl, alkoxy, -C(O)alkyl, -CO₂alkyl, -SO₂alkyl, -C(O)N(alkyl)₂, alkyl, or alkylamino.

2. A compound of claim 1, wherein: **R_{w}** and **Rₓ** are independently selected from: hydrogen, alkyl, alkenyl, aralkyl, or heteroaralkyl, or **R_{w}** and **Rₓ** may optionally be taken together to form a 5 to 7 membered ring selected from the group consisting of:

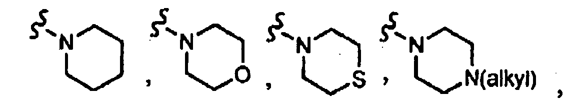

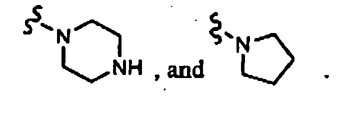

3. A compound of claim 2, wherein
**B** is phenyl or heteroaryl.

4. A compound of claim 1, wherein:
**B** is phenyl or heteroaryl.

5. A compound of claim 4, wherein:
**Z** is NH or CH₂; and
**R₃** is one or more substituents independently selected from: hydrogen, alkyl, alkoxy, halogen, alkoxyether, hydroxyl, cycloalkyl optionally substituted with R₄, heteroaryl optionally substituted with R₄, heterocyclyl optionally substituted with R₄, -O(cycloallcyl), phenoxy optionally substituted with R₄, heteroaryloxy optionally substituted with R₄, dialkylamino, or -SO₂alkyl.

6. A compound of claim 5, wherein: **Rₐ** is hydrogen, alkoxy, heteroaryl optionally substituted with R₅, hydroxyl, amino, alkylamino, dialkylamino, oxazolidinonyl optionally substituted with R₅, pyrrolidinonyl optionally substituted with R₅, heterocyclyl optionally substituted with **R₅,** -CONR_{w}Rₓ, -N(R_{w})CON(R_{y})(Rₓ), -N(R_{y})CON(R_{w})(Rₓ), -N(R_{w})C(O)ORₓ, -N(R_{w})COR_{y}, -SO₂R, -NR_{w}SO₂R_{y}, or -SO₂NR_{w}Rₓ.

7. A compound of claim 6, wherein:
**r** is 1;
**Rₐ** is hydrogen, hydroxyl, amino, alkylamino, dialkylamino, heteroaryl, heterocyclyl optionally substituted with R₅, -CONR_{w}Rₓ, -SO₂R_{y}, -NR_{w}SO₂R_{y}. -N(R_{y})CON(R_{w})(Rₓ), or -N(R_{w})C(O)ORₓ;
**R₅** is one substituent independently selected from: -C(O)alkyl, -SO₂alkyl, -C(O)N(alkyl)₂, alkyl, or -C(₁₄)alkyl-OH; and
**R₃** is one substituent independently selected from: alkyl, alkoxy, halogen, cycloalkyl, heterocyclyl, -O(cycloalkyl), phenoxy, or dialkylamino.

8. A compound of claim 7, wherein:
**B** is phenyl or pyridinyl;
**Rₐ** is hydrogen, dialkylamino, heterocyclyl optionally substituted with R₅, -CONR_{w}Rₓ, -N(R_{y})CON(R_{w})(Rₓ), or -NR_{w}SO₂R_{y}; and
**R₃** is one substituent independently selected from: alkyl, alkoxy, heterocyclyl; cycloalkyl, or -O(cycloalkyl).

9. A compound of claim 1 selected from the group consisting of: 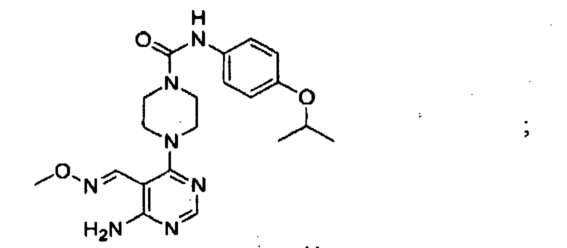 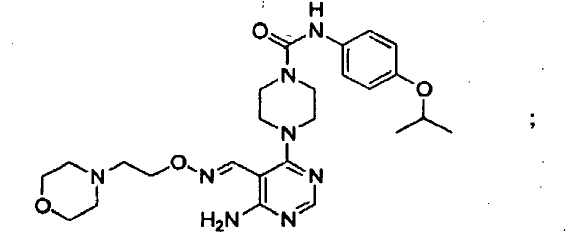 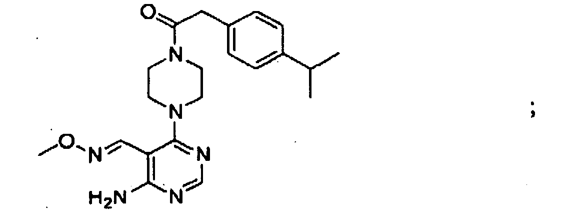 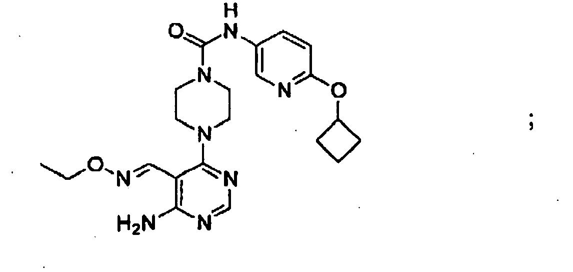 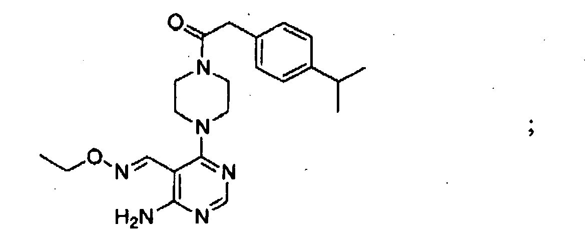 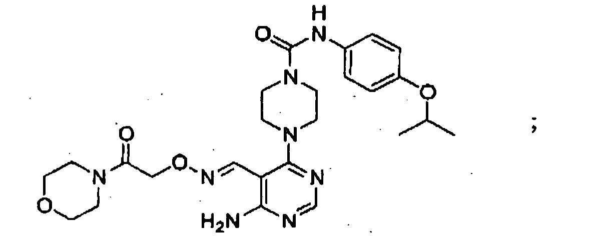 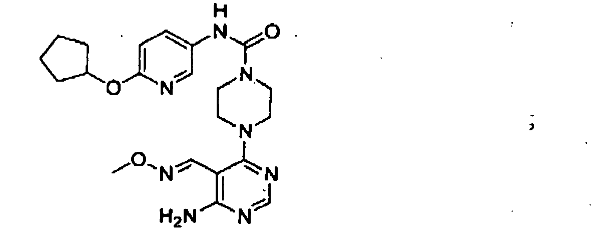 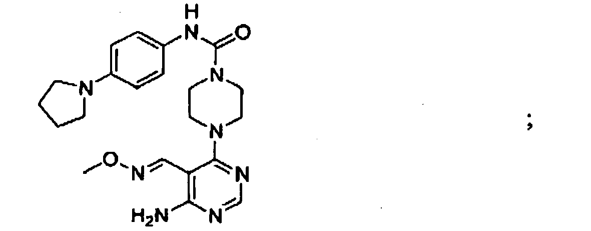 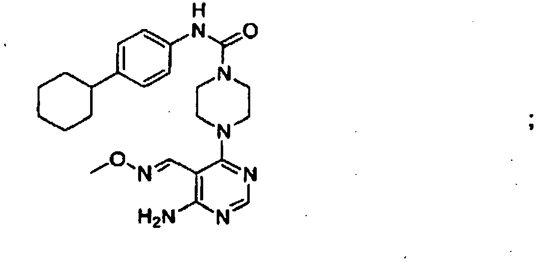 and

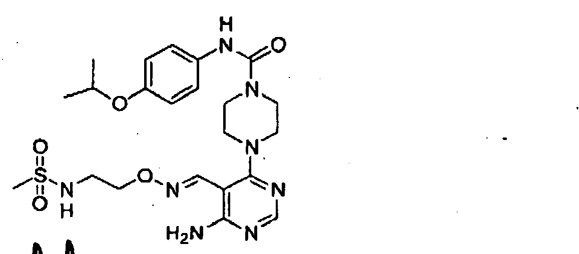

10. A compound of claim 1 selected from the group consisting of: 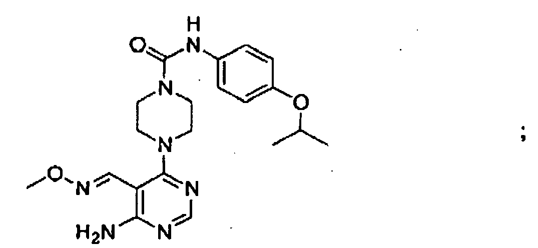 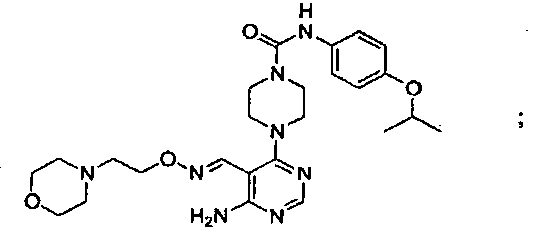 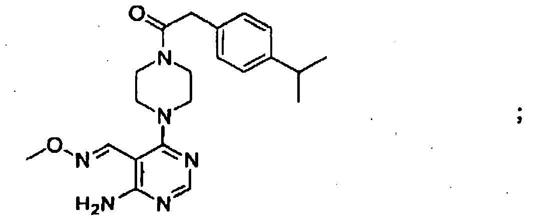 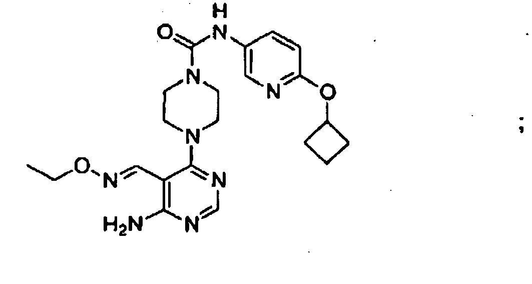 and

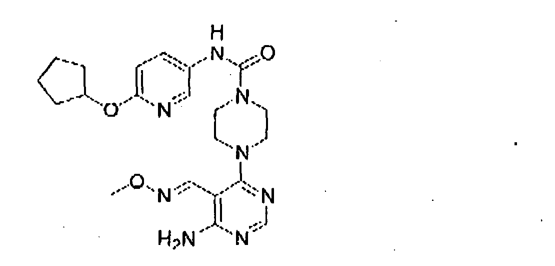

11. A pharmaceutical composition comprising a compound of claims 1-10 and a pharmaceutically acceptable carrier.

12. A compound as claimed in any of claims 1 to 10 for use as a medicine.

13. Use of a compound as claimed in any of claims 1 to 10 for the manufacture of a medicament for the treatment of a cell proliferative disorder.

14. A compound as claimed in any of claims 1 to 10 for treating a cell proliferative disorder.

15. A compound of Claims 1-10 for reducing or inhibiting, kinase activity of any one of TrkB, c-Kit and FLT3 in a cell.

16. A compound of Claims 1-10 for reducing or inhibiting kinase activity of any one of TrkB, c-Kit and FLT3 in a subject.

17. A pharmaceutical composition comprising a compound of Claims 1-10 and a pharmaceutically acceptable carrier for preventing or treating in a subject a disorder related to any one of TrkB, c-Kit and FLT3.

18. The pharmaceutical composition of claim 17 further comprising administration of any one of gene therapy, immunotherapy, radiation therapy and a chemotherapeutic agent.

19. A compound of Claims 1-10 for the treatment of a cell proliferative disorder or a disorder related to any one of TrkB, c-Kit and FLT3 in a subject wherein said compound is to be administered to the subject by controlled delivery by release from an intraluminal medical device.

20. The compound of claim 19, wherein said intraluminal medical device comprises a stent.

21. A pharmaceutical composition comprising a compound of claims 1-10 conjured to a targeting agent and a pharmaceutically acceptable carrier.

22. A compound of claims 1-10 conjugate to a targeting agent for treating a cell proliferative disorder or a disorder reflated to any one of TrkB, c-Kit and FLT3.

23. A combination of a chemotherapeutic agent and a compound as claimed in any of claims 1 to 10.

24. A process for the preparation of a compound of a compound of Claim 1, said process comprising reacting a compound of Formula IV: 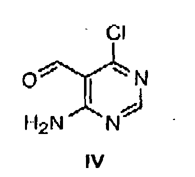 with a compound of Formula V: 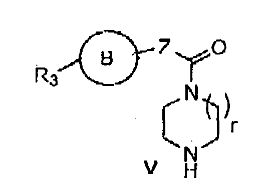 in the presence of a base.

25. A process for the preparation of a compound of Claim 1, said process comprising reacting a compound of formula XII: 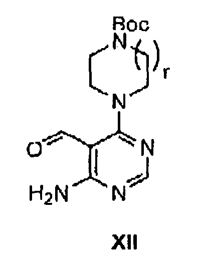 with a compound comprising R₁ONH₂.

26. A pharmaceutical composition comprising the product made by the process of claims 24-25.

## Patentansprüche

1. Verbindung von Formel I: 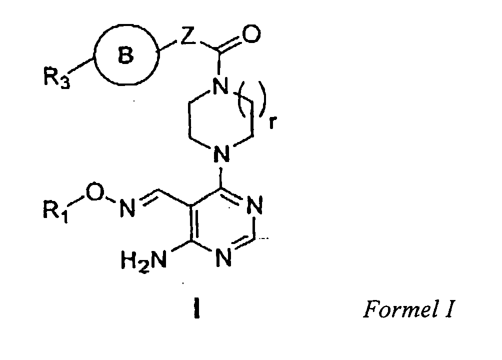 und N-Oxide, pharmazeutisch verträgliche Salze, Solvate, geometrische Isomere und stereochemische Isomere davon, worin:
**r** 1 oder 2 ist;
**Z** NH, N(Alkyl) oder CH₂ ist;
**B** Phenyl, Heteroaryl oder ein neun- bis zehngliedriges benzokondensiertes Heteroaryl ist;
**R₁:** 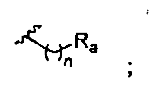
wobei **n** 1, 2, 3 oder 4 ist;
**Rₐ** Wasserstoff, Alkoxy, Phenoxy, Phenyl, Heteroaryl, gegebenenfalls substituiert mit R₅, Hydroxyl, Amino, Alkylamino, Dialkylamino, Oxazolidinonyl, gegebenenfalls substituiert mit R₅, Pyrrolidinonyl, gegebenenfalls substituiert mit R₅, Piperidinonyl, gegebenenfalls substituiert mit R₅, cyclisches Heterodionyl, gegebenenfalls substituiert mit R₅, Heterocyclyl, gegebenenfalls substituiert mit R₅, -COOR_{y}, -CONR_{w}Rₓ, -N(R_{w})CON(R_{y})(Rₓ), -N(R_{y})CON(R_{w})(Rₓ), -N(R_{w})C(O)ORₓ, -N(R_{w})COR_{y}, -SR_{y}, -SOR_{y}, -SO₂R_{y}, -NR_{w}SO₂R_{y}, -NR_{w}SO₂Rₓ, -SO₃R_{y}, -OSO₂NR_{w}Rₓ oder -SO₂NR_{w}Rₓ ist;
**R_{w}** und **Rₓ** unabhängig ausgewählt sind aus: Wasserstoff, Alkyl, Alkenyl, Aralkyl oder Heteroaralkyl oder **R_{w}** und **Rₓ** gegebenenfalls zusammengenommen sein können, um einen 5- bis 7-gliedrigen Ring zu bilden, der gegebenenfalls eine Heteroeinheit enthält, die ausgewählt ist aus O, NH, N(Alkyl), SO₂, SO oder S;
**R_{y}** ausgewählt ist aus: Wasserstoff, Alkyl, Alkenyl, Cycloalkyl, Phenyl, Aralkyl, Heteroaralkyl oder Heteroaryl;
**R₅** ein, zwei oder drei Substituenten ist, die unabhängig ausgewählt sind aus: Halogen, Cyano, Trifluormethyl, Amino, Hydroxyl, Alkoxy, -C(O)Alkyl, -SO₂Alkyl, -C(O)N(Alkyl)₂, Alkyl, C(₁₋₄)Alkyl-OH oder Alkylamino
ist; und
**R₃** ein oder mehrere Substituenten ist, die unabhängig ausgewählt sind aus: Wasserstoff, Alkyl, Alkoxy, Halogen, Alkoxyether, Hydroxyl, Thio, Nitro, Cycloalkyl, gegebenenfalls substituiert mit R₄, Heteroaryl, gegebenenfalls substituiert mit R₄, Alkylamino, Heterocyclyl, gegebenenfalls substituiert mit R₄, -O(Cycloalkyl), Pyrrolidinonyl, gegebenenfalls substituiert mit R₄, Phenoxy, gegebenenfalls substituiert mit R₄, -CN, -OCHF₂, -OCF₃, -CF₃, halogeniertem Alkyl, Heteroaryloxy, gegebenenfalls substituiert mit R₄, Dialkylamino, -NHSO₂Alkyl, Thioalkyl oder -SO₂Alkyl; wobei **R₄** unabhängig ausgewählt ist aus: Halogen, Cyano, Trifluormethyl, Amino, Hydroxyl, Alkoxy, -C(O)Alkyl, -CO₂Alkyl, -SO₂Alkyl, -C(O)N(Alkyl)₂, Alkyl oder Alkylamino.

2. Verbindung nach Anspruch 1, wobei: **R_{w}** und **Rₓ** unabhängig ausgewählt sind aus:
Wasserstoff, Alkyl, Alkenyl, Aralkyl oder Heteroaralkyl oder **R_{w}** und **Rₓ** gegebenenfalls zusammengenommen sein können, um einen 5- bis 7-gliedrigen Ring zu bilden, der ausgewählt ist aus der Gruppe, bestehend aus:

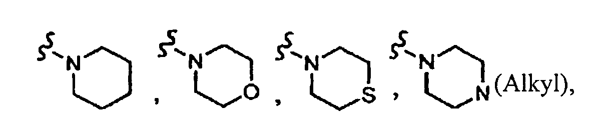

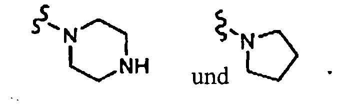

3. Verbindung nach Anspruch 2, wobei **B** Phenyl oder Heteroaryl ist.

4. Verbindung nach Anspruch 1, wobei: **B** Phenyl oder Heteroaryl ist.

5. Verbindung nach Anspruch 4, wobei:
**Z** NH oder CH₂ ist; und
**R₃** ein oder mehrere Substituenten ist, die unabhängig ausgewählt sind aus: Wasserstoff, Alkyl, Alkoxy, Halogen, Alkoxyether, Hydroxyl, Cycloalkyl, gegebenenfalls substituiert mit R₄, Heteroaryl, gegebenenfalls substituiert mit R₄, Heterocyclyl, gegebenenfalls substituiert mit R₄, -O(Cycloalkyl), Phenoxy, gegebenenfalls substituiert mit R₄, Heteroaryloxy, gegebenenfalls substituiert mit R₄, Dialkylamino oder -SO₂Alkyl.

6. Verbindung nach Anspruch 5, wobei: **Rₐ** Wasserstoff, Alkoxy, Heteroaryl, gegebenenfalls substituiert mit R₅, Hydroxyl, Amino, Alkylamino, Dialkylamino, Oxazolidinonyl, gegebenenfalls substituiert mit R₅, Pyrrolidinonyl, gegebenenfalls substituiert mit R₅, Heterocyclyl, gegebenenfalls substituiert mit R₅, -CONR_{w}Rₓ, -N(R_{w})CON(R_{y})(Rₓ), -N(R_{y})CON(R_{w})(Rₓ), -N(R_{w})C(O)ORₓ, -N(R_{w})COR_{y}, -SO₂R_{y}, -NR_{w}SO₂R_{y} oder -SO₂NR_{w}Rₓ ist.

7. Verbindung nach Anspruch 6, wobei:
**r** 1 ist;
**Rₐ** Wasserstoff, Hydroxyl, Amino, Alkylamino, Dialkylamino, Heteroaryl, Heterocyclyl, gegebenenfalls substituiert mit R₅, -CONR_{w}Rₓ, -SO₂R_{y}, -NR_{w}SO₂R_{y}, -N(R_{y})CON(R_{w})(Rₓ) oder -N(R_{w})C(O)ORₓ ist;
**R₅** ein Substituent ist, der unabhängig ausgewählt ist aus: -C(O)Alkyl, -SO₂Alkyl, -C(O)N(Alkyl)₂, Alkyl oder -C(₁₋₄)Alkyl-OH; und
**R₃** ein Substituent ist, der unabhängig ausgewählt ist aus: Alkyl, Alkoxy, Halogen, Cycloalkyl, Heterocyclyl, -O(Cycloalkyl), Phenoxy oder Dialkylamino.

8. Verbindung nach Anspruch 7, wobei:
**B** Phenyl oder Pyridinyl ist;
**Rₐ** Wasserstoff, Dialkylamino, Heterocyclyl, gegebenenfalls substituiert mit R₅, -CONR_{w}Rₓ, -N(R_{y})CON(R_{w})(Rₓ) oder -NR_{w}SO₂R_{y} ist; und
**R₃** ein Substituent ist, der unabhängig ausgewählt ist aus: Alkyl, Alkoxy, Heterocyclyl, Cycloalkyl oder -O(Cycloalkyl).

9. Verbindung nach Anspruch 1, ausgewählt aus der Gruppe, bestehend aus: 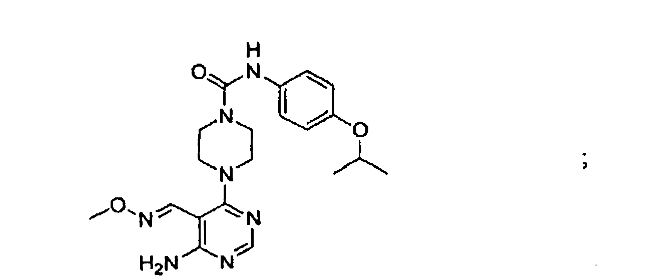 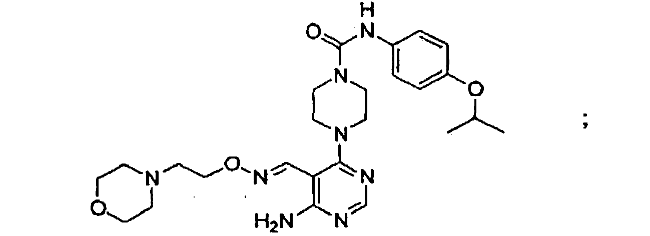 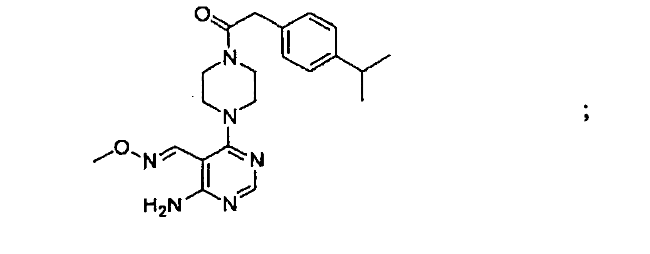 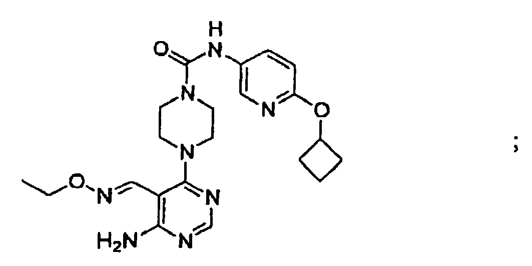 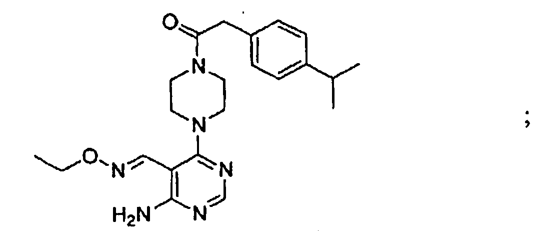 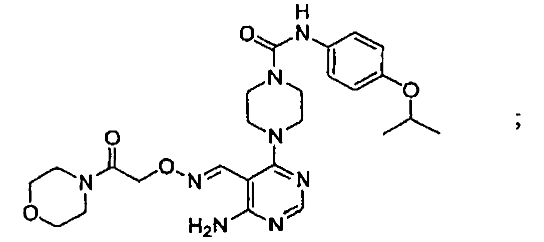 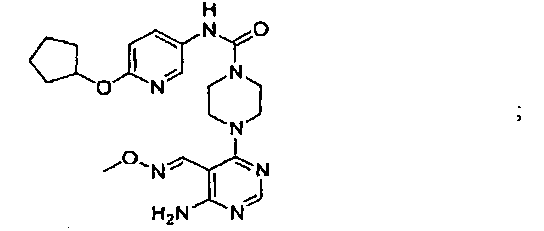 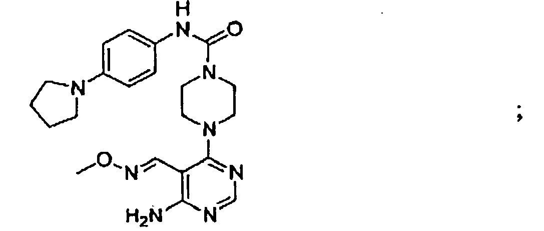 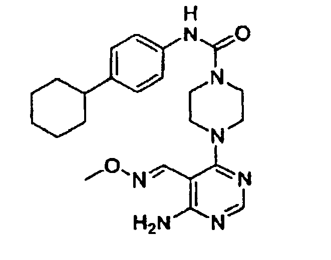 und

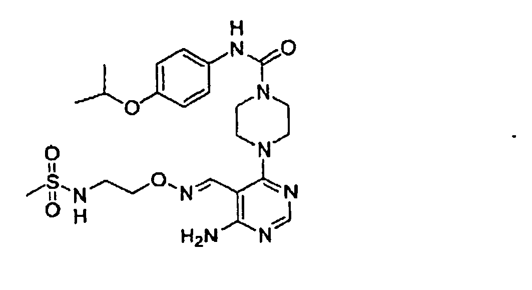

10. Verbindung nach Anspruch 1. ausgewählt aus der Gruppe, bestehend aus: 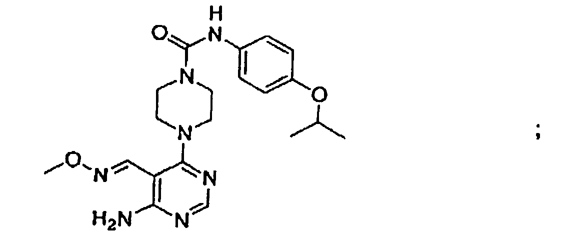 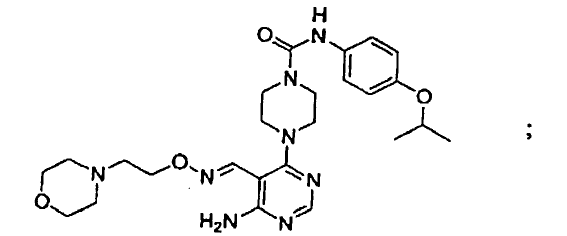 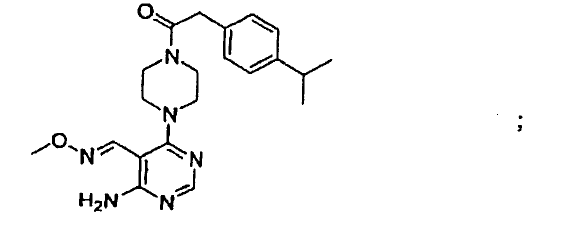 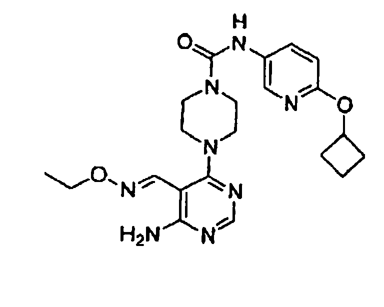 und

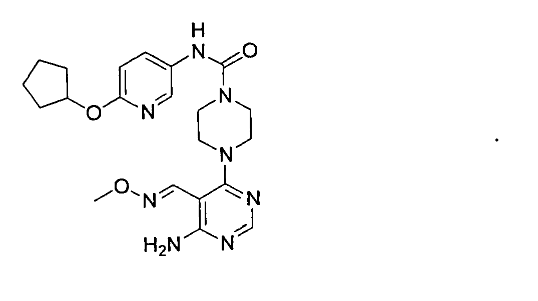

11. Pharmazeutische Zusammensetzung, die eine Verbindung nach den Ansprüchen 1 - 10 und einen pharmazeutisch verträglichen Trägerstoff umfasst.

12. Verbindung, wie beansprucht in einem der Ansprüche 1 bis 10, zur Verwendung als ein Arzneimittel.

13. Verwendung einer Verbindung, wie beansprucht in einem der Ansprüche 1 bis 10, zur Herstellung eines Arzneimittels zur Behandlung einer zellproliferativen Störung.

14. Verbindung, wie beansprucht in einem der Ansprüche 1 bis 10, zur Behandlung einer zellproliferativen Störung.

15. Verbindung nach den Ansprüchen 1 - 10 zur Verringerung oder Hemmung der Kinase-Aktivität von einem von TrkB, c-Kit und FLT3 in einer Zelle.

16. Verbindung nach den Ansprüchen 1 - 10 zur Verringerung oder Hemmung der Kinase-Aktivität von einem von TrkB, c-Kit und FLT3 in einem Patienten.

17. Pharmazeutische Zusammensetzung, die eine Verbindung nach den Ansprüchen 1 - 10 und einen pharmazeutisch verträglichen Trägerstoff umfasst, zur Verhinderung oder Behandlung einer Störung, die zusammenhängt mit einem von TrkB, c-Kit und FLT3, in einem Patienten.

18. Pharmazeutische Zusammensetzung nach Anspruch 17, die weiter die Verabreichung von einem von Gentherapie, Immuntherapie, Strahlungstherapie und einem chemotherapeutischen Mittel umfasst.

19. Verbindung nach den Ansprüchen 1 - 10 zur Behandlung einer zellproliferativen Störung oder einer Störung, die zusammenhängt mit einem von TrkB, c-Kit und FLT3, in einem Patienten, wobei die Verbindung dem Patienten durch kontrollierte Zuführung durch Freisetzung aus einer intraluminalen medizinischen Vorrichtung verabreicht werden soll.

20. Verbindung nach Anspruch 19, wobei die intraluminale medizinische Vorrichtung einen Stent umfasst.

21. Pharmazeutische Zusammensetzung, die eine Verbindung nach den Ansprüchen 1 - 10, konjugiert an ein Targeting-Mittel, und einen pharmazeutisch verträglichen Trägerstoff umfasst.

22. Verbindung nach den Ansprüchen 1 - 10, konjugiert an ein Targeting-Mittel, zur Behandlung einer zellproliferativen Störung oder einer Störung, die zusammenhängt mit einem von TrkB, c-Kit und FLT3.

23. Kombination aus einem chemotherapeutischen Mittel und einer Verbindung, wie beansprucht in einem der Ansprüche 1 bis 10.

24. Verfahren zur Herstellung einer Verbindung nach Anspruch 1, wobei das Verfahren umfasst, dass eine Verbindung von Formel IV: 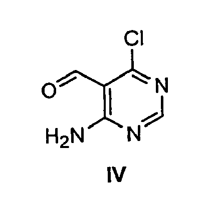 mit einer Verbindung von Formel V: 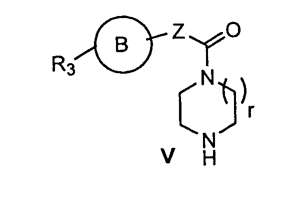 in Gegenwart einer Base umgesetzt wird.

25. Verfahren zur Herstellung einer Verbindung nach Anspruch 1, wobei das Verfahren umfasst, dass eine Verbindung von Formel XII: 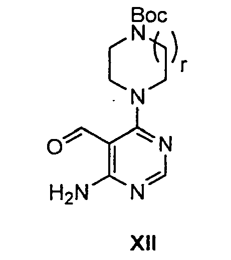 mit einer Verbindung, die R₁ONH₂ umfasst, umgesetzt wird.

26. Pharmazeutische Zusammensetzung, die das Produkt umfasst, das hergestellt ist mit dem Verfahren der Ansprüche 24 - 25.

## Revendications

1. Composé de la Formule I: 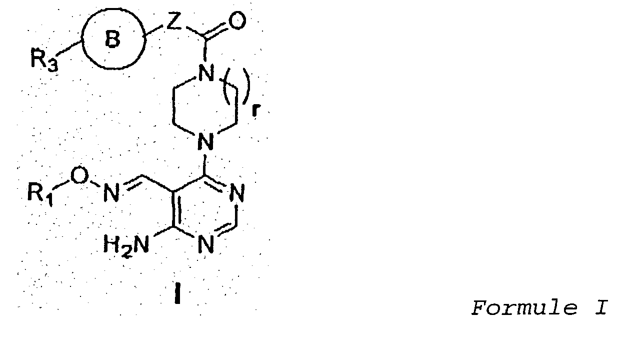 et N-oxydes, sels pharmaceutiquement acceptables, solvates, isomères géométriques et isomères stéréochimiques de ceux-ci, où:
r est 1 ou 2;
Z est NH, N(alkyl), ou CH₂;
B est phényle, hétéroaryle, ou un hétéroaryl benzo-fondu à neuf jusqu'à dix chaînons;
R₁ est 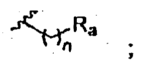
où n est 1,2,3 ou 4;
Rₐ est l'hydrogène, alcoxy, phénoxy, phényle, hétéroaryle optionnellement substitué avec R₅, hydroxyle, amino, alkylamino, dialkylamino, oxazolidinonyle, optionnellement substitué avec R₅, pyrrolidinonyle optionnellement substitué avec R₅, pipéridinonyle optionnellement substitué avec R₅, hétérodionyle cyclique optionnellement substitué avec R₅, hétérocyclyle optionnellement substitué avec R₅, -COOR_{y}, -CONR_{w}R_{y}, -N(R_{w})CON(R_{y})(Rₓ), -N (Ry) CON(R_{w})(Rₓ), -N(R_{w})C(O)ORₓ, -N(R_{w})COR_{y}, -SR_{y}, -SOR_{y}, -SO₂R_{y}, -NR_{w}SO₂R_{y}, -NR_{w}SO₂Rₓ, -SO₂R_{y}, -OSO₂NR_{w}Rₓ ou -SO₂NR_{w}Rₓ;
R_{w} et Rₓ sont indépendamment sélectionnés parmi: hydrogène, alkyle, alkényle, aralkyle ou hétéroaralkyle, ou R_{w} et Rₓ peuvent être optionnellement pris ensemble pour former un cycle à 5 jusqu'à 7 chaînons, optionnellement contenant un hétérofragment sélectionné parmi O, NH, N(alkyle), SO₂, SO, ou S;
R_{y} est sélectionné parmi: hydrogène, alkyle, alkényle, cycloalkyle, phényle, aralkyle, hétéroaralkyle, ou hétéroaryle;
R₅ est un, deux ou trois substituants indépendamment sélectionnés parmi: halogène, cyano, trifluorométhyle, amino, hydroxyle, alcoxy, -C(O)alkyle, -SO₂alkyle, -C(O)N(alkyle)₂, alkyle, C(₁₋₄)alkyl-OH, ou alkylamino; et
R₃ est un ou plusieurs substituants indépendamment sélectionnés parmi: hydrogène, alkyle, alcoxy, halogène, alcoxyéther, hydroxyle, thio, nitro, cycloalkyle optionnellement substitué avec R₄, hétéroaryle optionnellement substitué avec R₄, alkylamino, hétérocyclyle optionnellement substitué avec R₄, -O(cycloalkyle), pyrrolidinonyle optionnellement substitué avec R₄, phénoxy optionnellement substitué avec R₄, -CN, -OCHF₂, -OCF₃, -CF₃, alkyle halogéné, hétéroaryloxy optionnellement substitué avec R₄, dialkylamino, -NHSO₂alkyle, thioalkyle, ou -SO₂alkyle; où R₄ est indépendamment sélectionné parmi: halogène, cyano, trifluorométhyle, amino, hydroxyle, alcoxy, -C(O)alkyle, -CO₂alkyle, -SO₂alkyle, -C(O)N (alkyle)₂, alkyle, ou alkylamino.

2. Composé selon la revendication 1, où: R_{w} et Rₓ sont indépendamment sélectionnés parmi: hydrogène, alkyle, alkényle, aralkyle, ou hétéroalkyle, ou R_{w} et Rₓ peuvent optionnellement être pris ensemble pour former un cycle à 5 jusqu'à 7 chaînons sélectionnés dans le groupe constitué de:

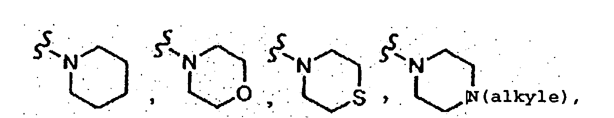

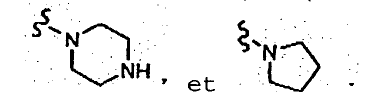

3. Composé selon la revendication 2, où B est phényle ou hétéroaryle.

4. Composé selon la revendication 1, où:
B est phényle ou hétéroaryle.

5. Composé selon la revendication 4, où:
Z est NH ou CH₂; et
R₃ est un ou plusieurs substituants indépendamment sélectionnés parmi: hydrogène, alkyle, alcoxy, halogène, alcoxyéther, hydroxyle, cycloalkyle optionnellement substitué avec R₄, hétéroaryle optionnellement substitué avec R₄, hétérocyclyle optionnellement substitué avec R₄, -O(cycloalkyle), phénoxy optionnellement substitué avec R₄, hétéroaryloxy optionnellement substitué avec R₄, dialkylamino, ou -SO₂alkyle.

6. Composé selon la revendication 5, où: Rₐ est hydrogène, alcoxy, hétéroaryle optionnellement substitué avec R₅, hydroxyle, amino, alkylamino, dialkylamino, oxazolidinonyle optionnellement substitué avec R₅, pyrrolidinonyle optionnellement substitué avec R₅, hétérocyclyle optionnellement substitué avec R₅, -CONR_{w}Rₓ, -N(R_{w})CON(R_{y})(Rₓ), -N(R_{y})CON(R_{w})(Rₓ), -N(R_{w})C(O)ORₓ, -N(R_{w}) COR_{y}, -SO₂R_{y}, -NR_{w}SO₂R_{y}, ou -SO₂NR_{w}Rₓ.

7. Composé selon la revendication 6, où:
r est 1;
Rₐ est hydrogène, hydroxyle, amino, alkylamino, dialkylamino, hétéroaryle, hétérocyclyle optionnellement substitué avec R₅, -CONR_{w}Rₓ, -SO₂R_{y}, -NR_{w}SO₂R_{y}, -N(R_{y}) CON(R_{w})(Rₓ), ou -N(R_{w})C(O)ORₓ;
R₅ est un substituant indépendamment sélectionné parmi: -C(O)alkyle, -SO₂alkyle, -C(O)N(alkyle)₂, alkyle, ou -C(₁₋₄)alkyl-OH; et
R₃ est un substituant indépendamment sélectionné parmi: alkyle, alcoxy, halogène, cycloalkyle, hétérocyclyle, -O(cycloalkyle), phénoxy, ou dialkylamino.

8. Composé selon la revendication 7, où:
B est phényle ou pyridinyle;
Rₐ est hydrogène, dialkylamino, hétérocyclyle optionnellement substitué avec R₅, -CONR_{w}Rₓ, -N(R_{y}) CON(R_{w})(Rₓ), ou -NR_{w}SO₂R_{y}; et
R₃ est un substituant indépendamment sélectionné parmi: alkyle, alcoxy, hétérocyclyle, cycloalkyle, ou -O(cycloalkyle).

9. Composé selon la revendication 1, sélectionné dans le groupe constitué de: 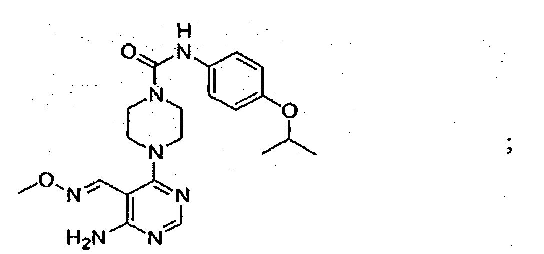 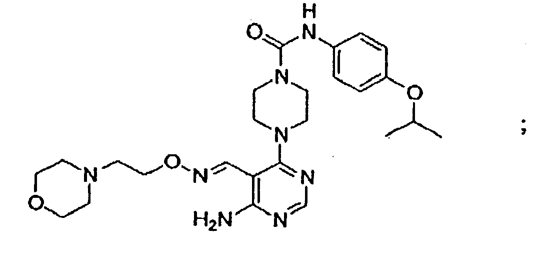 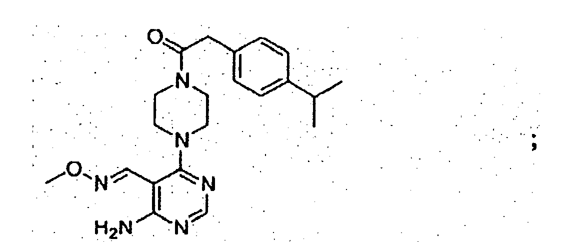 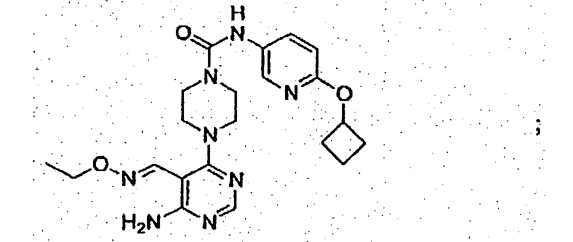 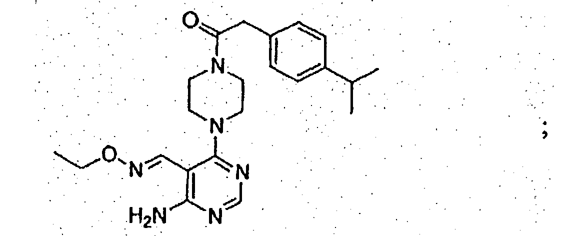 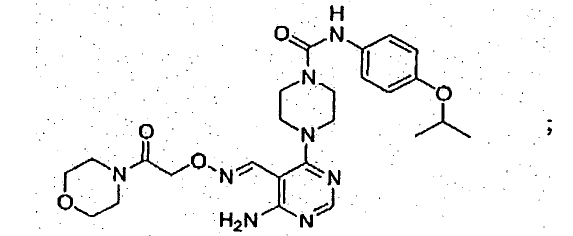 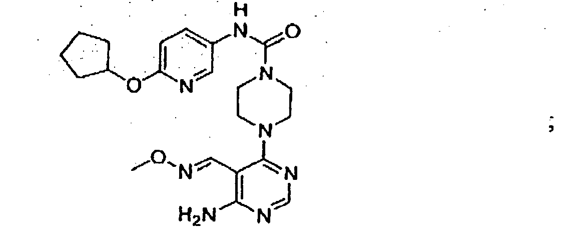 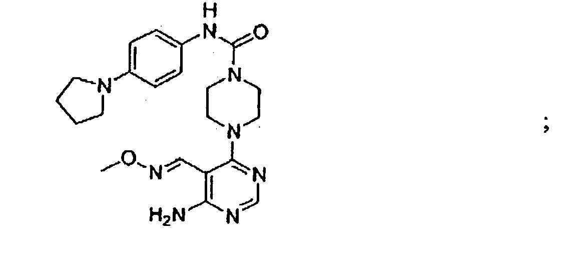 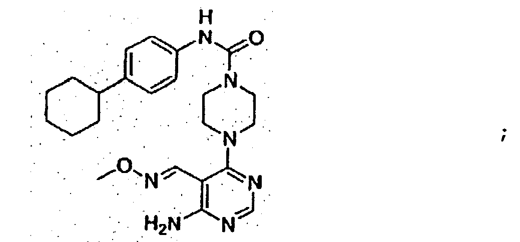 et

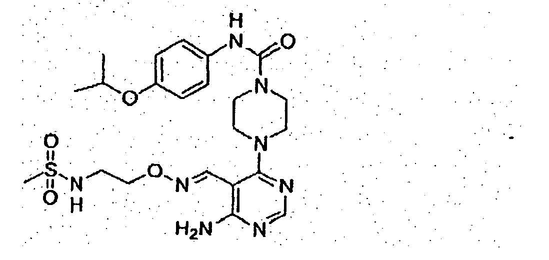

10. Composé selon la revendication 1, sélectionné dans le groupe constitué de: 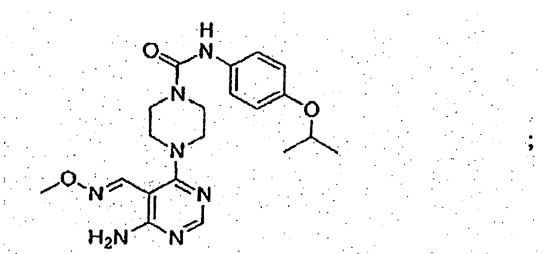 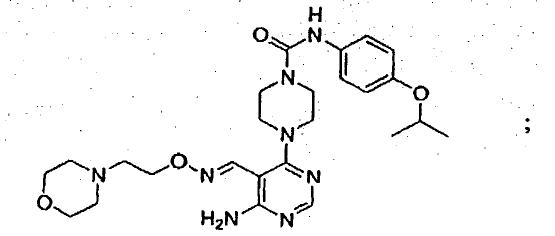 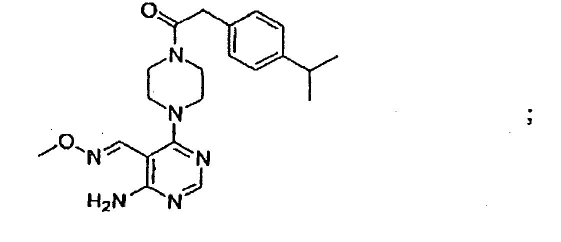 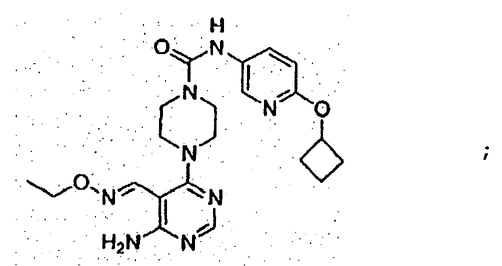 et

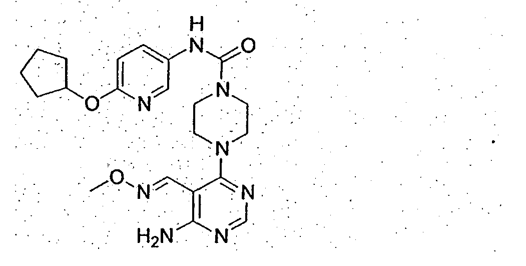

11. Composition pharmaceutique comprenant un composé selon les revendications 1-10 et un support pharmaceutiquement acceptable.

12. Composé tel que revendiqué dans l'une quelconque des revendications 1 à 10 pour utilisation comme médecine.

13. Utilisation d'un composé tel que revendiqué dans l'une quelconque des revendications 1 à 10 pour la fabrication d'un médicament pour le traitement de troubles cellulaires prolifératifs.

14. Composé tel que revendiqué dans l'une quelconque des revendications 1 à 10 pour le traitement de troubles cellulaires prolifératifs.

15. Composé selon les revendications 1 à 10 pour réduire ou empêcher une activité de kinase selon l'une quelconque de TrkB, c-Kit et FLT3 dans une cellule.

16. Composé selon les revendications 1 à 10 pour réduire ou empêcher l'activité de kinase de l'une quelconque de TrkB, c-Kit et FLT3 dans un sujet.

17. Composition pharmaceutique comprenant un composé selon les revendications 1 à 10 et un support pharmaceutiquement acceptable pour empêcher ou traiter dans un sujet un trouble lié à l'une quelconque de TrkB, c-Kit et FLT3.

18. Composition pharmaceutique selon la revendication 17, comprenant en outre l'administration d'un quelconque parmi la thérapie génétique, l'immunothérapie, la radiothérapie et un agent chimiothérapeutique.

19. Composé selon les revendications 1 à 10 pour le traitement de troubles cellulaires prolifératifs ou un trouble lié à l'une quelconque de TrkB, c-Kit et FLT3 dans un sujet, où ledit composé doit être administré au sujet par une distribution contrôlée par libération d'un dispositif médical intraluminal.

20. Composé selon la revendication 19, où ledit dispositif médical intraluminal comprend un écarteur.

21. Composition pharmaceutique comprenant un composé selon les revendications 1 à 10, conjugué à un agent de ciblage et un support pharmaceutiquement acceptable.

22. Composé selon les revendications 1 à 10, conjugué à un agent de ciblage pour traiter des troubles cellulaires prolifératifs ou un trouble lié à l'une quelconque parmi TrkB, c-Kit et FLT3.

23. Combinaison d'un agent chimiothérapeutique et d'un composé tel que revendiqué dans l'une quelconque des revendications 1 à 10.

24. Procédé de préparation d'un composé d'un composé de la revendication 1, ledit procédé comprenant la réaction d'un composé de la Formule IV: 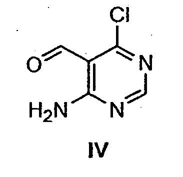 avec un composé de la Formule V: 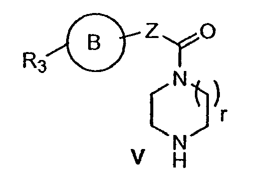 en présence d'une base.

25. Procédé de préparation d'un composé selon la revendication 1, ledit procédé comprenant la réaction d'un composé de la Formule XII: 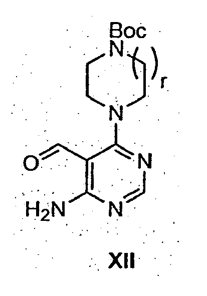 avec un composé comprenant R₁ONH₂.

26. Composition pharmaceutique comprenant le produit réalisé par le procédé selon les revendications 24-25.
